(19) Europäisches Patentamt
European Patent Office
Office européen des brevets

(11) EP 1 676 910 A1

(12) **EUROPEAN PATENT APPLICATION**
published in accordance with Art. 158(3) EPC

(43) Date of publication:
**05.07.2006 Bulletin 2006/27**

(21) Application number: **04773769.7**

(22) Date of filing: **08.10.2004**

(51) Int Cl.:
*C12N 5/10* (1990.01) *C12N 15/13* (1990.01)
*C12P 21/08* (1990.01) *A01K 67/027* (1990.01)
*A01H 5/00* (1968.09) *A61K 39/395* (1985.01)

(86) International application number:
**PCT/JP2004/015318**

(87) International publication number:
**WO 2005/035741 (21.04.2005 Gazette 2005/16)**

(84) Designated Contracting States:
**AT BE BG CH CY CZ DE DK EE ES FI FR GB GR HU IE IT LI LU MC NL PL PT RO SE SI SK TR**

(30) Priority: **09.10.2003 JP 2003350165**

(71) Applicant: **KYOWA HAKKO KOGYO CO., LTD.
Chiyoda-ku,
Tokyo 100-8185 (JP)**

(72) Inventors:
- **SATOH, Mitsuo,
c/o Kyowa Hakko Kogyo Co., Ltd.
Machida-shi,
Tokyo 194-8533 (JP)**
- **IIDA, Shigeru,,
c/o Kyowa Hakko Kogyo Co., Ltd.
Machida-shi,
Tokyo 194-8533 (JP)**
- **MORI, Katsuhiro,
c/o Kyowa Hakko Kogyo Co., Ltd.
Machida-shi,
Tokyo 194-8533 (JP)**
- **INOUE, Miho,
c/o Kyowa Hakko Kogyo Co., Ltd.
Machida-shi,
Tokyo 194-8533 (JP)**
- **KAMOCHI, Reiko,
c/o Kyowa Hakko Kogyo Co., Ltd.
Machida-shi,
Tokyo 194-8533 (JP)**
- **YAMANO, Kazuya,,
c/o Kyowa Hakko Kogyo Co., Ltd.
Machida-shi,
Tokyo 194-8533 (JP)**

(74) Representative: **Vossius & Partner
Siebertstrasse 4
81675 München (DE)**

(54) **GENOMICALLY MODIFIED CELL**

(57) The present invention provides a cell capable of controlling the sugar chain structure of a glycoprotein which is a producing protein, that is, a cell capable of producing a glycoprotein having high physiological activity; a process for producing a glycoprotein using the cell; a glycoprotein produced by the process; and use thereof.



Printed by Jouve, 75001 PARIS (FR)

SEQUENCE LISTING

<110> KYOWA HAKKO KOGYO CO., LTD.

<120> CELL IN WHICH GENOME IS MODIFIED

<130> 11619WO1

<150> JP2003-350165

<151> 2003-10-09

<160> 22

<170> PatentIn Ver. 2.0

<210> 1
<211> 1504
<212> DNA
<213> Cricetulus griseus

<220>
<221> CDS
<222> (1)..(1119)

<400> 1
```
atg gct cac gct ccc gct agc tgc ccg agc tcc agg aac tct ggg gac    48
Met Ala His Ala Pro Ala Ser Cys Pro Ser Ser Arg Asn Ser Gly Asp
  1               5                  10                  15

ggc gat aag ggc aag ccc agg aag gtg gcg ctc atc acg ggc atc acc    96
Gly Asp Lys Gly Lys Pro Arg Lys Val Ala Leu Ile Thr Gly Ile Thr
             20                  25                  30

ggc cag gat ggc tca tac ttg gca gaa ttc ctg ctg gag aaa gga tac   144
```

Gly Gln Asp Gly Ser Tyr Leu Ala Glu Phe Leu Leu Glu Lys Gly Tyr
35 40 45

gag gtt cat gga att gta cgg cga tcc agt tca ttt aat aca ggt cga 192
Glu Val His Gly Ile Val Arg Arg Ser Ser Ser Phe Asn Thr Gly Arg
50 55 60

att gaa cat tta tat aag aat cca cag gct cat att gaa gga aac atg 240
Ile Glu His Leu Tyr Lys Asn Pro Gln Ala His Ile Glu Gly Asn Met
65 70 75 80

aag ttg cac tat ggt gac ctc acc gac agc acc tgc cta gta aaa atc 288
Lys Leu His Tyr Gly Asp Leu Thr Asp Ser Thr Cys Leu Val Lys Ile
85 90 95

atc aat gaa gtc aaa cct aca gag atc tac aat ctt ggt gcc cag agc 336
Ile Asn Glu Val Lys Pro Thr Glu Ile Tyr Asn Leu Gly Ala Gln Ser
100 105 110

cat gtc aag att tcc ttt gac tta gca gag tac act gca gat gtt gat 384
His Val Lys Ile Ser Phe Asp Leu Ala Glu Tyr Thr Ala Asp Val Asp
115 120 125

gga gtt ggc acc ttg cgg ctt ctg gat gca att aag act tgt ggc ctt 432
Gly Val Gly Thr Leu Arg Leu Leu Asp Ala Ile Lys Thr Cys Gly Leu
130 135 140

ata aat tct gtg aag ttc tac cag gcc tca act agt gaa ctg tat gga 480
Ile Asn Ser Val Lys Phe Tyr Gln Ala Ser Thr Ser Glu Leu Tyr Gly
145 150 155 160

aaa gtg caa gaa ata ccc cag aaa gag acc acc cct ttc tat cca agg 528
Lys Val Gln Glu Ile Pro Gln Lys Glu Thr Thr Pro Phe Tyr Pro Arg
165 170 175

tcg ccc tat gga gca gcc aaa ctt tat gcc tat tgg att gta gtg aac 576

Ser Pro Tyr Gly Ala Ala Lys Leu Tyr Ala Tyr Trp Ile Val Val Asn
180 185 190

ttt cga gag gct tat aat ctc ttt gcg gtg aac ggc att ctc ttc aat 624
Phe Arg Glu Ala Tyr Asn Leu Phe Ala Val Asn Gly Ile Leu Phe Asn
195 200 205

cat gag agt cct aga aga gga gct aat ttt gtt act cga aaa att agc 672
His Glu Ser Pro Arg Arg Gly Ala Asn Phe Val Thr Arg Lys Ile Ser
210 215 220

cgg tca gta gct aag att tac ctt gga caa ctg gaa tgt ttc agt ttg 720
Arg Ser Val Ala Lys Ile Tyr Leu Gly Gln Leu Glu Cys Phe Ser Leu
225 230 235 240

gga aat ctg gac gcc aaa cga gac tgg ggc cat gcc aag gac tat gtc 768
Gly Asn Leu Asp Ala Lys Arg Asp Trp Gly His Ala Lys Asp Tyr Val
245 250 255

gag gct atg tgg ctg atg tta caa aat gat gaa cca gag gac ttt gtc 816
Glu Ala Met Trp Leu Met Leu Gln Asn Asp Glu Pro Glu Asp Phe Val
260 265 270

ata gct act ggg gaa gtt cat agt gtc cgt gaa ttt gtt gag aaa tca 864
Ile Ala Thr Gly Glu Val His Ser Val Arg Glu Phe Val Glu Lys Ser
275 280 285

ttc atg cac att gga aag acc att gtg tgg gaa gga aag aat gaa aat 912
Phe Met His Ile Gly Lys Thr Ile Val Trp Glu Gly Lys Asn Glu Asn
290 295 300

gaa gtg ggc aga tgt aaa gag acc ggc aaa att cat gtg act gtg gat 960
Glu Val Gly Arg Cys Lys Glu Thr Gly Lys Ile His Val Thr Val Asp
305 310 315 320

ctg aaa tac tac cga cca act gaa gtg gac ttc ctg cag gga gac tgc 1008

Leu Lys Tyr Tyr Arg Pro Thr Glu Val Asp Phe Leu Gln Gly Asp Cys
325 330 335

tcc aag gcg cag cag aaa ctg aac tgg aag ccc cgc gtt gcc ttt gac 1056
Ser Lys Ala Gln Gln Lys Leu Asn Trp Lys Pro Arg Val Ala Phe Asp
340 345 350

gag ctg gtg agg gag atg gtg caa gcc gat gtg gag ctc atg aga acc 1104
Glu Leu Val Arg Glu Met Val Gln Ala Asp Val Glu Leu Met Arg Thr
355 360 365

aac ccc aac gcc tga gcacctctac aaaaaaattc gcgagacatg gactatggtg 1159
Asn Pro Asn Ala
370

cagagccagc caaccagagt ccagccactc ctgagaccat cgaccataaa ccctcgactg 1219
cctgtgtcgt ccccacagct aagagctggg ccacaggttt gtgggcacca ggacggggac 1279
actccagagc taaggccact tcgcttttgt caaaggctcc tctcaatgat tttgggaaat 1339
caagaagttt aaaatcacat actcatttta cttgaaatta tgtcactaga caacttaaat 1399
ttttgagtct tgagattgtt tttctctttt cttattaaat gatctttcta tgacccagca 1459
aaaaaaaaaa aaaaaaggga tataaaaaaa aaaaaaaaaa aaaaa 1504

<210> 2
<211> 1119
<212> DNA
<213> Homo sapiens

<220>
<221> CDS
<222> (1)..(1119)

<400> 2
atg gca cac gca ccg gca cgc tgc ccc agc gcc cgg ggc tcc ggg gac 48
Met Ala His Ala Pro Ala Arg Cys Pro Ser Ala Arg Gly Ser Gly Asp
1 5 10 15

```
ggc gag atg ggc aag ccc agg aac gtg gcg ctc atc acc ggt atc aca     96
Gly Glu Met Gly Lys Pro Arg Asn Val Ala Leu Ile Thr Gly Ile Thr
            20                  25                  30

ggc cag gat ggt tcc tac ctg gct gag ttc ctg ctg gag aaa ggc tat    144
Gly Gln Asp Gly Ser Tyr Leu Ala Glu Phe Leu Leu Glu Lys Gly Tyr
        35                  40                  45

gag gtc cat gga att gta cgg cgg tcc agt tca ttt aat acg ggt cga    192
Glu Val His Gly Ile Val Arg Arg Ser Ser Ser Phe Asn Thr Gly Arg
    50                  55                  60

att gag cat ctg tat aag aat ccc cag gct cac att gaa gga aac atg    240
Ile Glu His Leu Tyr Lys Asn Pro Gln Ala His Ile Glu Gly Asn Met
65                  70                  75                  80

aag ttg cac tat ggc gat ctc act gac agt acc tgc ctt gtg aag atc    288
Lys Leu His Tyr Gly Asp Leu Thr Asp Ser Thr Cys Leu Val Lys Ile
                85                  90                  95

att aat gaa gta aag ccc aca gag atc tac aac ctt gga gcc cag agc    336
Ile Asn Glu Val Lys Pro Thr Glu Ile Tyr Asn Leu Gly Ala Gln Ser
            100                 105                 110

cac gtc aaa att tcc ttt gac ctc gct gag tac act gcg gac gtt gac    384
His Val Lys Ile Ser Phe Asp Leu Ala Glu Tyr Thr Ala Asp Val Asp
        115                 120                 125

gga gtt ggc act cta cga ctt cta gat gca gtt aag act tgt ggc ctt    432
Gly Val Gly Thr Leu Arg Leu Leu Asp Ala Val Lys Thr Cys Gly Leu
    130                 135                 140

atc aac tct gtg aag ttc tac caa gcc tca aca agt gaa ctt tat ggg    480
Ile Asn Ser Val Lys Phe Tyr Gln Ala Ser Thr Ser Glu Leu Tyr Gly
145                 150                 155                 160
```

aaa gtg cag gaa ata ccc cag aag gag acc acc cct ttc tat ccc cgg 528
Lys Val Gln Glu Ile Pro Gln Lys Glu Thr Thr Pro Phe Tyr Pro Arg
165 170 175

tca ccc tat ggg gca gca aaa ctc tat gcc tat tgg att gtg gtg aac 576
Ser Pro Tyr Gly Ala Ala Lys Leu Tyr Ala Tyr Trp Ile Val Val Asn
180 185 190

ttc cgt gag gcg tat aat ctc ttt gca gtg aac ggc att ctc ttc aat 624
Phe Arg Glu Ala Tyr Asn Leu Phe Ala Val Asn Gly Ile Leu Phe Asn
195 200 205

cat gag agt ccc aga aga gga gct aat ttc gtt act cga aaa att agc 672
His Glu Ser Pro Arg Arg Gly Ala Asn Phe Val Thr Arg Lys Ile Ser
210 215 220

cgg tca gta gct aag att tac ctt gga caa ctg gaa tgt ttc agt ttg 720
Arg Ser Val Ala Lys Ile Tyr Leu Gly Gln Leu Glu Cys Phe Ser Leu
225 230 235 240

gga aat ctg gat gcc aaa cga gat tgg ggc cat gcc aag gac tat gtg 768
Gly Asn Leu Asp Ala Lys Arg Asp Trp Gly His Ala Lys Asp Tyr Val
245 250 255

gag gct atg tgg ttg atg ttg cag aat gat gag ccg gag gac ttc gtt 816
Glu Ala Met Trp Leu Met Leu Gln Asn Asp Glu Pro Glu Asp Phe Val
260 265 270

ata gct act ggg gag gtc cat agt gtc cgg gaa ttt gtc gag aaa tca 864
Ile Ala Thr Gly Glu Val His Ser Val Arg Glu Phe Val Glu Lys Ser
275 280 285

ttc ttg cac att gga aaa acc att gtg tgg gaa gga aag aat gaa aat 912
Phe Leu His Ile Gly Lys Thr Ile Val Trp Glu Gly Lys Asn Glu Asn
290 295 300

gaa gtg ggc aga tgt aaa gag acc ggc aaa gtt cac gtg act gtg gat 960
Glu Val Gly Arg Cys Lys Glu Thr Gly Lys Val His Val Thr Val Asp
305 310 315 320

ctc aag tac tac cgg cca act gaa gtg gac ttt ctg cag ggc gac tgc 1008
Leu Lys Tyr Tyr Arg Pro Thr Glu Val Asp Phe Leu Gln Gly Asp Cys
325 330 335

acc aaa gcg aaa cag aag ctg aac tgg aag ccc cgg gtc gct ttc gat 1056
Thr Lys Ala Lys Gln Lys Leu Asn Trp Lys Pro Arg Val Ala Phe Asp
340 345 350

gag ctg gtg agg gag atg gtg cac gcc gac gtg gag ctc atg agg aca 1104
Glu Leu Val Arg Glu Met Val His Ala Asp Val Glu Leu Met Arg Thr
355 360 365

aac ccc aat gcc tga 1119
Asn Pro Asn Ala
370

<210> 3
<211> 1119
<212> DNA
<213> Mus musculus

<220>
<221> CDS
<222> (1)..(1119)

<400> 3
atg gct caa gct ccc gct aag tgc ccg agc tac ccg ggc tcc ggg gat 48
Met Ala Gln Ala Pro Ala Lys Cys Pro Ser Tyr Pro Gly Ser Gly Asp
1 5 10 15

ggc gag atg ggc aag ctc agg aag gtg gct ctc atc act ggc atc acc 96
Gly Glu Met Gly Lys Leu Arg Lys Val Ala Leu Ile Thr Gly Ile Thr
20 25 30

gga cag gat ggt tcg tac ttg gca gaa ttc ctg ttg gag aaa ggg tac 144
Gly Gln Asp Gly Ser Tyr Leu Ala Glu Phe Leu Leu Glu Lys Gly Tyr
35 40 45

gag gtc cat gga ata gta cgg cga tct agt tca ttt aat aca ggt cga 192
Glu Val His Gly Ile Val Arg Arg Ser Ser Ser Phe Asn Thr Gly Arg
50 55 60

att gaa cat tta tat aag aat cct cag gct cat att gaa gga aac atg 240
Ile Glu His Leu Tyr Lys Asn Pro Gln Ala His Ile Glu Gly Asn Met
65 70 75 80

aag ttg cac tat ggt gac ctc act gac agc acc tgc cta gtg aaa atc 288
Lys Leu His Tyr Gly Asp Leu Thr Asp Ser Thr Cys Leu Val Lys Ile
85 90 95

atc aat gaa gtc aag cct aca gag atc tat aat ctt gga gcc cag agc 336
Ile Asn Glu Val Lys Pro Thr Glu Ile Tyr Asn Leu Gly Ala Gln Ser
100 105 110

cat gtc aag atc tcc ttt gac tta gct gag tac acc gca gat gtt gat 384
His Val Lys Ile Ser Phe Asp Leu Ala Glu Tyr Thr Ala Asp Val Asp
115 120 125

ggc gtt ggc acc ttg cgg ctt ctg gat gca att aaa act tgt ggc ctt 432
Gly Val Gly Thr Leu Arg Leu Leu Asp Ala Ile Lys Thr Cys Gly Leu
130 135 140

ata aat tct gtg aag ttc tac cag gcc tca aca agt gaa ctt tat gga 480
Ile Asn Ser Val Lys Phe Tyr Gln Ala Ser Thr Ser Glu Leu Tyr Gly
145 150 155 160

```
aaa gtg cag gaa ata ccc cag aag gag acc aca cct ttc tat ccg agg     528
Lys Val Gln Glu Ile Pro Gln Lys Glu Thr Thr Pro Phe Tyr Pro Arg
                165                 170                 175

tca ccc tat gga gca gcc aaa ctc tat gcc tat tgg att gtg gtg aat     576
Ser Pro Tyr Gly Ala Ala Lys Leu Tyr Ala Tyr Trp Ile Val Val Asn
            180                 185                 190

ttc cgt gaa gct tat aat ctc ttt gca gtg aat gga att ctc ttc aat     624
Phe Arg Glu Ala Tyr Asn Leu Phe Ala Val Asn Gly Ile Leu Phe Asn
        195                 200                 205

cat gag agt ccc aga aga gga gct aat ttt gtt act cga aaa att agc     672
His Glu Ser Pro Arg Arg Gly Ala Asn Phe Val Thr Arg Lys Ile Ser
    210                 215                 220

cgg tca gta gct aag att tac ctt gga caa ctg gaa tgt ttc agc ttg     720
Arg Ser Val Ala Lys Ile Tyr Leu Gly Gln Leu Glu Cys Phe Ser Leu
225                 230                 235                 240

gga aat ctg gat gcc aaa cga gac tgg ggc cat gcc aag gac tat gta     768
Gly Asn Leu Asp Ala Lys Arg Asp Trp Gly His Ala Lys Asp Tyr Val
                245                 250                 255

gag gct atg tgg ctc atg ttg cag aat gat gag cca gag gac ttt gtc     816
Glu Ala Met Trp Leu Met Leu Gln Asn Asp Glu Pro Glu Asp Phe Val
            260                 265                 270

ata gct act ggg gaa gtt cac agt gtc cgt gaa ttt gtt gaa aag tca     864
Ile Ala Thr Gly Glu Val His Ser Val Arg Glu Phe Val Glu Lys Ser
        275                 280                 285

ttc atg cac atc gga aaa acc att gtg tgg gaa gga aag aat gaa aat     912
Phe Met His Ile Gly Lys Thr Ile Val Trp Glu Gly Lys Asn Glu Asn
    290                 295                 300
```

gaa gtg ggc aga tgt aaa gag acc ggc aaa gtt cac gtg act gtg gat 960
Glu Val Gly Arg Cys Lys Glu Thr Gly Lys Val His Val Thr Val Asp
305 310 315 320

ctg aaa tac tac cga ccg act gaa gtg gac ttt ctg cag gga gac tgc 1008
Leu Lys Tyr Tyr Arg Pro Thr Glu Val Asp Phe Leu Gln Gly Asp Cys
325 330 335

tcc aag gct cag cag aag cta aac tgg aag ccc cgc gtt gcc ttt gac 1056
Ser Lys Ala Gln Gln Lys Leu Asn Trp Lys Pro Arg Val Ala Phe Asp
340 345 350

gag ctg gtg agg gag atg gtg cag gcc gac gtg gag ctc atg agg acc 1104
Glu Leu Val Arg Glu Met Val Gln Ala Asp Val Glu Leu Met Arg Thr
355 360 365

aac ccc aac gct tga 1119
Asn Pro Asn Ala
370

<210> 4
<211> 372
<212> PRT
<213> Cricetulus griseus

<400> 4
Met Ala His Ala Pro Ala Ser Cys Pro Ser Ser Arg Asn Ser Gly Asp
1 5 10 15

Gly Asp Lys Gly Lys Pro Arg Lys Val Ala Leu Ile Thr Gly Ile Thr
20 25 30

Gly Gln Asp Gly Ser Tyr Leu Ala Glu Phe Leu Leu Glu Lys Gly Tyr
35 40 45

Glu Val His Gly Ile Val Arg Arg Ser Ser Ser Phe Asn Thr Gly Arg
50 55 60

Ile Glu His Leu Tyr Lys Asn Pro Gln Ala His Ile Glu Gly Asn Met
65 70 75 80

Lys Leu His Tyr Gly Asp Leu Thr Asp Ser Thr Cys Leu Val Lys Ile
85 90 95

Ile Asn Glu Val Lys Pro Thr Glu Ile Tyr Asn Leu Gly Ala Gln Ser
100 105 110

His Val Lys Ile Ser Phe Asp Leu Ala Glu Tyr Thr Ala Asp Val Asp
115 120 125

Gly Val Gly Thr Leu Arg Leu Leu Asp Ala Ile Lys Thr Cys Gly Leu
130 135 140

Ile Asn Ser Val Lys Phe Tyr Gln Ala Ser Thr Ser Glu Leu Tyr Gly
145 150 155 160

Lys Val Gln Glu Ile Pro Gln Lys Glu Thr Thr Pro Phe Tyr Pro Arg
165 170 175

Ser Pro Tyr Gly Ala Ala Lys Leu Tyr Ala Tyr Trp Ile Val Val Asn
180 185 190

Phe Arg Glu Ala Tyr Asn Leu Phe Ala Val Asn Gly Ile Leu Phe Asn
195 200 205

His Glu Ser Pro Arg Arg Gly Ala Asn Phe Val Thr Arg Lys Ile Ser
210 215 220

Arg Ser Val Ala Lys Ile Tyr Leu Gly Gln Leu Glu Cys Phe Ser Leu
225 230 235 240

Gly Asn Leu Asp Ala Lys Arg Asp Trp Gly His Ala Lys Asp Tyr Val
245 250 255

Glu Ala Met Trp Leu Met Leu Gln Asn Asp Glu Pro Glu Asp Phe Val
260 265 270

Ile Ala Thr Gly Glu Val His Ser Val Arg Glu Phe Val Glu Lys Ser
275 280 285

Phe Met His Ile Gly Lys Thr Ile Val Trp Glu Gly Lys Asn Glu Asn
290 295 300

Glu Val Gly Arg Cys Lys Glu Thr Gly Lys Ile His Val Thr Val Asp
305 310 315 320

Leu Lys Tyr Tyr Arg Pro Thr Glu Val Asp Phe Leu Gln Gly Asp Cys
325 330 335

Ser Lys Ala Gln Gln Lys Leu Asn Trp Lys Pro Arg Val Ala Phe Asp
340 345 350

Glu Leu Val Arg Glu Met Val Gln Ala Asp Val Glu Leu Met Arg Thr
355 360 365

Asn Pro Asn Ala
370

<210> 5
<211> 372
<212> PRT
<213> Homo sapiens

<400> 5
Met Ala His Ala Pro Ala Arg Cys Pro Ser Ala Arg Gly Ser Gly Asp
1 5 10 15

Gly Glu Met Gly Lys Pro Arg Asn Val Ala Leu Ile Thr Gly Ile Thr
20 25 30

Gly Gln Asp Gly Ser Tyr Leu Ala Glu Phe Leu Leu Glu Lys Gly Tyr
35 40 45

Glu Val His Gly Ile Val Arg Arg Ser Ser Ser Phe Asn Thr Gly Arg
50 55 60

Ile Glu His Leu Tyr Lys Asn Pro Gln Ala His Ile Glu Gly Asn Met
65 70 75 80

Lys Leu His Tyr Gly Asp Leu Thr Asp Ser Thr Cys Leu Val Lys Ile
85 90 95

Ile Asn Glu Val Lys Pro Thr Glu Ile Tyr Asn Leu Gly Ala Gln Ser
100 105 110

His Val Lys Ile Ser Phe Asp Leu Ala Glu Tyr Thr Ala Asp Val Asp
115 120 125

Gly Val Gly Thr Leu Arg Leu Leu Asp Ala Val Lys Thr Cys Gly Leu
130 135 140

Ile Asn Ser Val Lys Phe Tyr Gln Ala Ser Thr Ser Glu Leu Tyr Gly
145 150 155 160

Lys Val Gln Glu Ile Pro Gln Lys Glu Thr Thr Pro Phe Tyr Pro Arg
165 170 175

Ser Pro Tyr Gly Ala Ala Lys Leu Tyr Ala Tyr Trp Ile Val Val Asn
180 185 190

Phe Arg Glu Ala Tyr Asn Leu Phe Ala Val Asn Gly Ile Leu Phe Asn
195 200 205

His Glu Ser Pro Arg Arg Gly Ala Asn Phe Val Thr Arg Lys Ile Ser
210 215 220

Arg Ser Val Ala Lys Ile Tyr Leu Gly Gln Leu Glu Cys Phe Ser Leu
225 230 235 240

Gly Asn Leu Asp Ala Lys Arg Asp Trp Gly His Ala Lys Asp Tyr Val
245 250 255

Glu Ala Met Trp Leu Met Leu Gln Asn Asp Glu Pro Glu Asp Phe Val
260 265 270

Ile Ala Thr Gly Glu Val His Ser Val Arg Glu Phe Val Glu Lys Ser
275 280 285

Phe Leu His Ile Gly Lys Thr Ile Val Trp Glu Gly Lys Asn Glu Asn
290 295 300

Glu Val Gly Arg Cys Lys Glu Thr Gly Lys Val His Val Thr Val Asp
305 310 315 320

Leu Lys Tyr Tyr Arg Pro Thr Glu Val Asp Phe Leu Gln Gly Asp Cys
325 330 335

Thr Lys Ala Lys Gln Lys Leu Asn Trp Lys Pro Arg Val Ala Phe Asp
340 345 350

Glu Leu Val Arg Glu Met Val His Ala Asp Val Glu Leu Met Arg Thr
355 360 365

Asn Pro Asn Ala
370

<210> 6

<211> 372
<212> PRT
<213> Mus musculus

<400> 6
Met Ala Gln Ala Pro Ala Lys Cys Pro Ser Tyr Pro Gly Ser Gly Asp
1               5                   10                  15

Gly Glu Met Gly Lys Leu Arg Lys Val Ala Leu Ile Thr Gly Ile Thr
            20                  25                  30

Gly Gln Asp Gly Ser Tyr Leu Ala Glu Phe Leu Leu Glu Lys Gly Tyr
        35                  40                  45

Glu Val His Gly Ile Val Arg Arg Ser Ser Ser Phe Asn Thr Gly Arg
    50                  55                  60

Ile Glu His Leu Tyr Lys Asn Pro Gln Ala His Ile Glu Gly Asn Met
65                  70                  75                  80

Lys Leu His Tyr Gly Asp Leu Thr Asp Ser Thr Cys Leu Val Lys Ile
                85                  90                  95

Ile Asn Glu Val Lys Pro Thr Glu Ile Tyr Asn Leu Gly Ala Gln Ser
            100                 105                 110

His Val Lys Ile Ser Phe Asp Leu Ala Glu Tyr Thr Ala Asp Val Asp
        115                 120                 125

Gly Val Gly Thr Leu Arg Leu Leu Asp Ala Ile Lys Thr Cys Gly Leu
    130                 135                 140

Ile Asn Ser Val Lys Phe Tyr Gln Ala Ser Thr Ser Glu Leu Tyr Gly
145                 150                 155                 160

Lys Val Gln Glu Ile Pro Gln Lys Glu Thr Thr Pro Phe Tyr Pro Arg

165 170 175

Ser Pro Tyr Gly Ala Ala Lys Leu Tyr Ala Tyr Trp Ile Val Val Asn
180 185 190

Phe Arg Glu Ala Tyr Asn Leu Phe Ala Val Asn Gly Ile Leu Phe Asn
195 200 205

His Glu Ser Pro Arg Arg Gly Ala Asn Phe Val Thr Arg Lys Ile Ser
210 215 220

Arg Ser Val Ala Lys Ile Tyr Leu Gly Gln Leu Glu Cys Phe Ser Leu
225 230 235 240

Gly Asn Leu Asp Ala Lys Arg Asp Trp Gly His Ala Lys Asp Tyr Val
245 250 255

Glu Ala Met Trp Leu Met Leu Gln Asn Asp Glu Pro Glu Asp Phe Val
260 265 270

Ile Ala Thr Gly Glu Val His Ser Val Arg Glu Phe Val Glu Lys Ser
275 280 285

Phe Met His Ile Gly Lys Thr Ile Val Trp Glu Gly Lys Asn Glu Asn
290 295 300

Glu Val Gly Arg Cys Lys Glu Thr Gly Lys Val His Val Thr Val Asp
305 310 315 320

Leu Lys Tyr Tyr Arg Pro Thr Glu Val Asp Phe Leu Gln Gly Asp Cys
325 330 335

Ser Lys Ala Gln Gln Lys Leu Asn Trp Lys Pro Arg Val Ala Phe Asp
340 345 350

Glu Leu Val Arg Glu Met Val Gln Ala Asp Val Glu Leu Met Arg Thr

355 360 365

Asn Pro Asn Ala
370

<210> 7
<211> 383
<212> DNA
<213> Cricetulus griseus

<400> 7
gttaactggg gctcttttaa accctgaatt tttctaaatc cccacctcca agagtttggt 60

ttaaactgat ttttttaatg aataccttttt gaagaataga gcattgtctc atcatgcaaa 120

gcttctcagg gattcagcta gcatgttgaa gaaacataag ggtgttaaat tgtttgtcac 180

aagtgctgaa taaatattga cgtagtcttc agctattcta tactggaagt agatgatatt 240

ctcattggaa attctgttag gaagtaaccc ttcttgtctt cttacctgca tagaatccca 300

ggatataaaa cttgtgcttg tcgcccttgc cattgtctct cactggtggc ctttattgca 360

tctcatatct gccttctctt tcc 383

<210> 8
<211> 504
<212> DNA
<213> Cricetulus griseus

<400> 8
taagaattcc tgtgcccagc tgtatgtgag gctctctgca ggtgtaggga tgtttctgct 60

ttctttctgc acatgcttca cagctgaagt cctttgggtg tgagattgac attcagatag 120

actaaagtga ctggacttgt tgggaaacat actgtatgca ttattgccgt tgcctccagg 180

tgaaattaac acctcattca ccaatccctg ttcatccaaa ctttctaccc acatcacttt 240

aaatagaaat tagacccaat atgactcctt ttttcctaag ctgtttatag agattgtgct 320

ggagcagtga gcttttgtgt ttgtttgttt gttttgtaat tttccccatg aaaatttctc 300

taaactcaaa cctaagaggg aaaaaaaaaa aacagactta tatgtgccac acttgtaaaa 360

aaaaatcatg aaagatgtat atgatatttt taaacagttt gaatattaag atcacaattt 420

ctattttaaa aacaatcttg ttttacatat caatcaccca attcccttgc cttcccatcc 480

tcccattccc cccactgatc cccc 504

<210> 9
<211> 120
<212> DNA
<213> Cricetulus griseus

<400> 9
atgaatgttc attctttggg tatatgccca agagtagaat tgctaaatat tgaggtagac 60

tgattcccat tttcttgagg agtcgccata ttgatttcca aagtgactgt acaagttaac 120

<210> 10
<211> 274
<212> DNA
<213> Cricetulus griseus

<400> 10
aggcactagg taaatatttt tgaagaaaga atgagtatct cctatttcag aaaaactttt 60

attgacttaa atttaggata tcagaattag aaaacagtaa aaatttatag gagagttttt 120

aatgaatgtt attttaaggt tccatacaaa tagtaattaa aacttacaca aactatttgt 180

agtaatgatt cagtctggta taccctgatg agcattatac acttttaaat tctttttgta 240

aatttttta ttagttcaaa ttaggaacaa gctt 274

<210> 11
<211> 24
<212> DNA
<213> Artificial Sequence

<220>
<223> Description of Artificial Sequence: Synthetic DNA

<400> 11
gatatcgctg cgctcgttgt cgac 24

<210> 12
<211> 24
<212> DNA
<213> Artificial Sequence

<220>
<223> Description of Artificial Sequence: Synthetic DNA

<400> 12
caggaaggaa ggctggaaaa gagc 24

<210> 13
<211> 26

<212> DNA
<213> Artificial Sequence

<220>
<223> Description of Artificial Sequence: Synthetic DNA

<400> 13
aagcccagga aggtggcgct catcac 26

<210> 14
<211> 28
<212> DNA
<213> Artificial Sequence

<220>
<223> Description of Artificial Sequence: Synthetic DNA

<400> 14
cactagttga ggcctggtag aacttcac 28

<210> 15
<211> 25
<212> DNA
<213> Artificial Sequence

<220>
<223> Description of Artificial Sequence: Synthetic DNA

<400> 15
tcctttgact tagctgagta caccg 25

<210> 16
<211> 25

<212> DNA
<213> Artificial Sequence

<220>
<223> Description of Artificial Sequence: Synthetic DNA

<400> 16
catagggtga cctcggatag aaagg 25

<210> 17
<211> 18
<212> PRT
<213> Homo Sapiens

<400> 17
Asp Glu Ser Ile Tyr Ser Asn Tyr Tyr Leu Tyr Glu Ser Ile Pro Lys
1 5 10 15

Pro Cys
1

<210> 18
<211> 1395
<212> DNA
<213> Homo sapiens

<220>
<221> CDS
<222> (1)..(1395)

<400> 18
atg tat tcc aat gtg ata gga act gta acc tct gga aaa agg aag gtt 48
Met Tyr Ser Asn Val Ile Gly Thr Val Thr Ser Gly Lys Arg Lys Val
1 5 10 15

```
tat ctt ttg tcc ttg ctg ctc att ggc ttc tgg gac tgc gtg acc tgt      96
Tyr Leu Leu Ser Leu Leu Leu Ile Gly Phe Trp Asp Cys Val Thr Cys
                20                  25                  30

cac ggg agc cct gtg gac atc tgc aca gcc aag ccg cgg gac att ccc     144
His Gly Ser Pro Val Asp Ile Cys Thr Ala Lys Pro Arg Asp Ile Pro
            35                  40                  45

atg aat ccc atg tgc att tac cgc tcc ccg gag aag aag gca act gag     192
Met Asn Pro Met Cys Ile Tyr Arg Ser Pro Glu Lys Lys Ala Thr Glu
        50                  55                  60

gat gag ggc tca gaa cag aag atc ccg gag gcc acc aac cgg cgt gtc     240
Asp Glu Gly Ser Glu Gln Lys Ile Pro Glu Ala Thr Asn Arg Arg Val
65                  70                  75                  80

tgg gaa ctg tcc aag gcc aat tcc cgc ttt gct acc act ttc tat cag     288
Trp Glu Leu Ser Lys Ala Asn Ser Arg Phe Ala Thr Thr Phe Tyr Gln
                85                  90                  95

cac ctg gca gat tcc aag aat gac aat gat aac att ttc ctg tca ccc     336
His Leu Ala Asp Ser Lys Asn Asp Asn Asp Asn Ile Phe Leu Ser Pro
            100                 105                 110

ctg agt atc tcc acg gct ttt gct atg acc aag ctg ggt gcc tgt aat     384
Leu Ser Ile Ser Thr Ala Phe Ala Met Thr Lys Leu Gly Ala Cys Asn
        115                 120                 125

gac acc ctc cag caa ctg atg gag gta ttt aag ttt gac acc ata tct     432
Asp Thr Leu Gln Gln Leu Met Glu Val Phe Lys Phe Asp Thr Ile Ser
    130                 135                 140

gag aaa aca tct gat cag atc cac ttc ttc ttt gcc aaa ctg aac tgc     480
Glu Lys Thr Ser Asp Gln Ile His Phe Phe Phe Ala Lys Leu Asn Cys
145                 150                 155                 160
```

```
cga ctc tat cga aaa gcc aac aaa tcc tcc aag tta gta tca gcc aat     528
Arg Leu Tyr Arg Lys Ala Asn Lys Ser Ser Lys Leu Val Ser Ala Asn
                165                 170                 175

cgc ctt ttt gga gac aaa tcc ctt acc ttc aat gag acc tac cag gac     576
Arg Leu Phe Gly Asp Lys Ser Leu Thr Phe Asn Glu Thr Tyr Gln Asp
            180                 185                 190

atc agt gag ttg gta tat gga gcc aag ctc cag ccc ctg gac ttc aag     624
Ile Ser Glu Leu Val Tyr Gly Ala Lys Leu Gln Pro Leu Asp Phe Lys
        195                 200                 205

gaa aat gca gag caa tcc aga gcg gcc atc aac aaa tgg gtg tcc aat     672
Glu Asn Ala Glu Gln Ser Arg Ala Ala Ile Asn Lys Trp Val Ser Asn
    210                 215                 220

aag acc gaa ggc cga atc acc gat gtc att ccc tcg gaa gcc atc aat     720
Lys Thr Glu Gly Arg Ile Thr Asp Val Ile Pro Ser Glu Ala Ile Asn
225                 230                 235                 240

gag ctc act gtt ctg gtg ctg gtt aac acc att tac ttc aag ggc ctg     768
Glu Leu Thr Val Leu Val Leu Val Asn Thr Ile Tyr Phe Lys Gly Leu
                245                 250                 255

tgg aag tca aag ttc agc cct gag aac aca agg aag gaa ctg ttc tac     816
Trp Lys Ser Lys Phe Ser Pro Glu Asn Thr Arg Lys Glu Leu Phe Tyr
            260                 265                 270

aag gct gat gga gag tcg tgt tca gca tct atg atg tac cag gaa ggc     864
Lys Ala Asp Gly Glu Ser Cys Ser Ala Ser Met Met Tyr Gln Glu Gly
        275                 280                 285

aag ttc cgt tat cgg cgc gtg gct gaa ggc acc cag gtg ctt gag ttg     912
Lys Phe Arg Tyr Arg Arg Val Ala Glu Gly Thr Gln Val Leu Glu Leu
    290                 295                 300
```

ccc ttc aaa ggt gat gac atc acc atg gtc ctc atc ttg ccc aag cct 960
Pro Phe Lys Gly Asp Asp Ile Thr Met Val Leu Ile Leu Pro Lys Pro
305 310 315 320

gag aag agc ctg gcc aag gtg gag aag gaa ctc acc cca gag gtg ctg 1008
Glu Lys Ser Leu Ala Lys Val Glu Lys Glu Leu Thr Pro Glu Val Leu
325 330 335

cag gag tgg ctg gat gaa ttg gag gag atg atg ctg gtg gtt cac atg 1056
Gln Glu Trp Leu Asp Glu Leu Glu Glu Met Met Leu Val Val His Met
340 345 350

ccc cgc ttc cgc att gag gac ggc ttc agt ttg aag gag cag ctg caa 1104
Pro Arg Phe Arg Ile Glu Asp Gly Phe Ser Leu Lys Glu Gln Leu Gln
355 360 365

gac atg ggc ctt gtc gat ctg ttc agc cct gaa aag tcc aaa ctc cca 1152
Asp Met Gly Leu Val Asp Leu Phe Ser Pro Glu Lys Ser Lys Leu Pro
370 375 380

ggt att gtt gca gaa ggc cga gat gac ctc tat gtc tca gat gca ttc 1200
Gly Ile Val Ala Glu Gly Arg Asp Asp Leu Tyr Val Ser Asp Ala Phe
385 390 395 400

cat aag gca ttt ctt gag gta aat gaa gaa ggc agt gaa gca gct gca 1248
His Lys Ala Phe Leu Glu Val Asn Glu Glu Gly Ser Glu Ala Ala Ala
405 410 415

agt acc gct gtt gtg att gct ggc cgt tcg cta aac ccc aac agg gtg 1296
Ser Thr Ala Val Val Ile Ala Gly Arg Ser Leu Asn Pro Asn Arg Val
420 425 430

act ttc aag gcc aac agg ccc ttc ctg gtt ttt ata aga gaa gtt cct 1344
Thr Phe Lys Ala Asn Arg Pro Phe Leu Val Phe Ile Arg Glu Val Pro
435 440 445

ctg aac act att atc ttc atg ggc aga gta gcc aac cct tgt gtt aag 1392
Leu Asn Thr Ile Ile Phe Met Gly Arg Val Ala Asn Pro Cys Val Lys
450 455 460

taa 1395

465

<210> 19
<211> 43
<212> DNA
<213> Artificial Sequence

<220>
<223> Description of Artificial Sequence : Synthetic DNA

<400> 19
cggaattcgc caccatgtat tccaatgtga taggaactgt aac 43

<210> 20
<211> 36
<212> DNA
<213> Artificial Sequence

<220>
<223> Description of Artificial Sequence : Synthetic DNA

<400> 20
cgggatcctt acttaacaca agggttggct actctg 36

<210> 21
<211> 34
<212> DNA

<213> Artificial Sequence

<220>
<223> Description of Artificial Sequence : Synthetic DNA

<400> 21
ctctatcgaa aagcccagaa atcctccaag ttag 34

<210> 22
<211> 34
<212> DNA
<213> Artificial Sequence

<220>
<223> Description of Artificial Sequence : Synthetic DNA

<400> 22
ctaacttgga ggatttctgg gcttttcgat agag 34

## Description

TECHNICAL FIELD

The present invention relates to a cell in which a genomic gene encoding an enzyme capable of catalyzing a dehydration reaction to convert GDP-mannose into GDP-4-keto,6-deoxy-GDP-mannose is knocked out, and a process for producing a glycoprotein, such as an antibody, using the cell.

BACKGROUND ART

In general, glycoproteins such as antibodies applicable to medicaments are prepared by using genetic recombination techniques. That is, glycoproteins such as antibodies are produced by using, as a host cell, an animal cell such as Chinese hamster ovary tissue-derived CHO cell. Sugar chain structures of glycoproteins are different depending on the host cell which produces the glycoprotein. Accordingly, under the present situation, a sugar chain for having the most suitable pharmacological activity is not always added to the glycoprotein.

Regarding antibodies, Hanai *et al.* have reported that addition of fucose to N-acetylglucosamine in the reducing end in N-glycoside-linked sugar chains of an antibody decreases ADCC activity of the antibody to fiftieth [WO00/61739, *J. Biol. Chem.,* 278, 3466 (2003)]. These reports show that the sugar chain structure plays a considerably important role in the effector function of human IgG1 subclass antibodies, and that a change in the sugar chain structure results in a change in the pharmacological activity relating to the effector function.

The sugar chain structure of a glycoprotein is determined by the expression of sugar chain genes, that is, genes respectively encoding a glycosyltransferase which synthesizes the sugar chain and a glycolytic enzyme which degrades the sugar chain, and genes encoding proteins capable of carrying out respective functions such as biosynthesis of an intracellular sugar nucleotide which becomes the donor of a saccharide to the sugar chain and transfer thereof to the Golgi body. A possibility has been shown that the sugar chain structure of a glycoprotein produced by the host cell can be controlled by introducing a gene encoding an enzyme or protein relating to the modification of these sugar chains into a host cell, or mutating them.

Attempts have been made to modify the sugar chain structure of a produced glycoprotein by introducing a gene encoding an enzyme relating to the modification of a sugar chain (hereinafter referred to as gene encoding an enzyme relating to the modification of a sugar chain) into a producing cell. Specifically, it has been reported that 1) it is possible to produce a protein in which sialic acid is added in large numbers to the non-reducing end of its sugar chain, by introducing a gene encoding rat β-galactoside α2,6-sialyltransferase into CHO cell [*J. Biol. Chem.,* 261, 13848 (1989)], 2) it is possible to express an H antigen (Fuc α1-2Gal β1-) in which fucose (hereinafter referred also to as Fuc) is added to the non-reducing end of its sugar chain, by introducing a gene encoding human β-galactoside 2-α-fucosyltransferase into mouse L cell [*Science,* 252, 1668 (1991)], and 3) it is possible to produce an antibody having a high addition ratio of N-acetylglucosamine at the bisecting of N-glycoside-linked sugar chain, by producing the antibody using CHO cell into which a gene encoding β1,4-N-acetylglucosamine transferase III (GnTIII) is introduced [WO99/54342, *Glycobiology,* 5, 813 (1995)]. It has been reported that, when an antibody was expressed using CHO cell into which a gene encoding GnTIII was introduced, it showed 16 times higher ADCC activity than the antibody expressed by the parent cell line, but over-expression of GnTIII or β1,4-N-acetylglucosamine transferase V (GnTV) showed toxicity upon CHO cell.

The mutants in which the activity of a gene encoding a protein or enzyme relating to the modification of a sugar chain is changed have been obtained, for example, as clones showing resistance to a lectin such as WGA (wheat-germ agglutinin derived from *T. vulgaris),* ConA (concanavalin A derived from *C. ensiformis),* RIC (a toxin derived from *R. communis),* L-PHA (leukoagglutinin derived from *P. vulgaris),* LCA (lentil agglutinin derived from *L. culinaris),* PSA (pea lectin derived from *P. sativum)* or the like [*Somatic Cell Mol. Genet.,* 12, 51 (1986)]. A case has been reported in which a glycoprotein having a changed sugar chain structure is produced by using such a mutant, as the host cell, in which the activity of a gene encoding a protein or enzyme relating to the modification of a sugar chain was changed. Specific examples include a report on the production of an antibody having a high mannose type sugar chain structure using a CHO cell mutant clone in which the activity of N-acetylglucosamine transferase I (GnTI) was deleted [*J. Immunol.,* 160, 3393 (1998)]. In addition, a case has been reported on the production of an antibody having a sugar chain structure in which sialic acid is not added to the non-reducing end in the sugar chains or an antibody without addition of galactose thereto, using a CMP-sialic acid transporter- or UDP-galactose transporter-deficient clone, but each of them does not express an antibody having effector activity improved to a degree suitable as a medicament [*J Immunol.,* 160, 3393 (1998)]. Under such a situation, it has been reported that an antibody having high ADCC activity can be produced by using, as the host cell, a clone having decreased activity of GDP-mannose 4,6-dehydratase, an enzyme capable of catalyzing a dehydration reaction to convert GDP-mannose into GDP-4-keto,6-deoxy-GDP-mannose [WO00/61739, *J. Biol. Chem.,* 278, 3466 (2003), *J. Biol. Chem.,* 277, 26733 (2002)]. In these reports, as the host cell, a lectin-resistant clone which can recognize a sugar chain structure in which 1-position of fucose are bound to 6-position of N-acetylglucosamine in the reducing end in the complex type N-glycoside-linked sugar chains through α-bond, such as clone CHO-AAL which is resistant to AAL (a lectin derived from *Aleuria aurantia*), clone CHO-LCA which is resistant to LCA (lentil agglutinin derived from *L. culinaris)* or clone Lec 13 is used as the host cell. In addition to these, $PL^R1.3$ established as a PSA (pea lectin derived from *P. sativum*) resistant mutant of a mouse leukemia-derived clone BW 5147 is also known as a clone having decreased activity of GDP-mannose 4,6-dehydratase [*J. Biol. Chem.,* 255, 9900 (1980)]. However, since each of these clones is not a complete gene deficient clone, it is difficult to completely inhibit addition of fucose to a sugar chain structure, wherein inhibitory addition of fucose to a sugar chain, namely to the N-acetylglucosamine in the reducing end in the N-glycoside-linked sugar chains is a cause of showing high ADCC activity by the antibody,. Particularly, since mutant clones such as $PL^R1.3$ and Lec13 are obtained by randomly introducing mutation through a mutagen treatment, it cannot always be said that they have properties suited as clones to be used in the production of pharmaceutical preparations.

There are no reports on the preparation of a knockout animal or plant in which only a target gene was disrupted by gene engineering techniques targeting at a gene encoding GDP-mannose 4,6-dehydratase which is an enzyme capable of catalyzing a dehydration reaction to convert GDP-mannose into GDP-4-keto,6-deoxy-GDP-mannose, and there are no reports also on the production of a glycoprotein using such a knockout individual.

In order to modify the sugar chain structure added to a glycoprotein produced by a cell, attempts have been made to control the activity of an enzyme or protein relating to the modification of a sugar chain of the host cell. However, since the modification mechanism of sugar chain is varied and complex in reality, and elucidation of the physiological role played by the sugar chain has not been sufficiently clarified, it is the present situation that trial and error are repeated.

## DISCLOSURE OF THE INVENTION

Concern has been directed toward the development of a host cell capable of producing a glycoprotein such as an antibody useful in developing a medicament. Accordingly, the present invention provides a cell which can control a modified sugar chain structure of a glycoprotein as the produced protein, namely a cell capable of producing a glycoprotein which keeps high pharmacological activity, a process for producing a glycoprotein using the cell, and the glycoprotein produced by the production process.

The present invention relating to the following (1) to (24):

(1) A cell in which a genomic gene encoding an enzyme capable of catalyzing a dehydration reaction to convert GDP-mannose into GDP-4-keto,6-deoxy-GDP-mannose is knocked out.

(2) The cell according to the above (1), wherein all of alleles on a genome encoding an enzyme capable of catalyzing a dehydration reaction to convert GDP-mannose into GDP-4-keto,6-deoxy-GDP-mannose are knocked out.

(3) The cell according to the above (1) or (2), wherein at least exon 5 of the genomic gene encoding an enzyme capable of catalyzing a dehydration reaction to convert GDP-mannose into GDP-4-keto,6-deoxy-GDP-mannose is deleted.

(4) The knockout cell according to any one of the above (1) to (3), wherein the enzyme capable of catalyzing a dehydration reaction to convert GDP-mannose into GDP-4-keto,6-deoxy-GDP-mannose is GDP-mannose 4,6-dehydratase.

(5) The cell according to any one of the above (1) to (4), which is resistant to a lectin which recognizes a sugar chain structure in which 1-position of fucose is bound to 6-position of N-acetylglucosamine in the reducing end through α-bond in a complex type N-glycoside-linked sugar chain.

(6) The cell according to the above (5), wherein the cell which is resistant to a lectin is a cell selected based on the fact that the cell shows a higher survival ratio than a cell before the genomic gene is not knocked out, when the cells are cultured in a medium comprising lectin which recognizes a sugar chain structure in which 1-position of fucose is bound to 6-position of N-acetylglucosamine in the reducing end through α-bond in a complex type N-glycoside-linked sugar chain.

(7) The cell according to any one of the above (1) to (6), which comprises a gene encoding a glycoprotein.

(8) The cell according to the above (7), wherein the glycoprotein is a glycoprotein which does not have a sugar chain structure in which 1-position of fucose is bound to 6-position of N-acetylglucosamine in the reducing end through α-bond in a complex type N-glycoside-linked sugar chain.

(9) The cell according to the above (7) or (8), wherein the glycoprotein is an antibody.

(10) The cell according to the above (9), wherein the antibody belongs to an IgG class.

(11) A process for producing a glycoprotein composition, which comprises using the cell according to any one of the above (1) to (10).

(12) A process for producing a glycoprotein composition, which comprises culturing the cell according to any one of the above (1) to (10) in a medium to form and accumulate the glycoprotein composition in the culture, and recovering and purifying the glycoprotein composition from the culture.

(13) The process according to the above (11) or (12), wherein the glycoprotein is an antibody.

(14) A transgenic non-human animal or plant or the progenies thereof, which is produced by using the cell according to any one of the above (1) to (6).

(15) The transgenic non-human animal or plant or the progenies thereof according to the above (14), wherein the transgenic non-human animal is an animal selected from the group consisting of cattle, sheep, goat, pig, horse, mouse, rat, fowl, monkey and rabbit.

(16) The transgenic non-human animal or plant or the progenies thereof according to the above (14) or (15), which is introduced with a gene encoding a glycoprotein.

(17) The transgenic non-human animal or plant or the progenies thereof according to the above (16), wherein the glycoprotein is an antibody.

(18) The transgenic non-human animal or plant or the progenies thereof according to the above (17), wherein the antibody belongs to an IgG class.

(19) A process for producing a glycoprotein, which comprises rearing the transgenic non-human animal or plant according to any one of the above (14) to (18); isolating a tissue or body fluid comprising a glycoprotein composition introduced from the reared animal or plant; and recovering and purifying the glycoprotein composition of interest from the isolated tissue or body fluid.

(20) A process for producing a glycoprotein composition, which comprises isolating a glycoprotein-producing cell from the transgenic non-human animal or plant or the progenies thereof according to any one of the above (14) to (18); culturing the isolated glycoprotein-producing cell in a medium to form and accumulate the glycoprotein composition in the culture; and recovering and purifying the glycoprotein composition from the culture.

(21) The process according to the above (19) or (20), wherein the glycoprotein is an antibody.

(22) A glycoprotein composition produced by the process according to any one of the above (11) to (13) and (19) to (21).

(23) An antibody composition produced by the process according to (13) or (21).

(24) A medicament comprising the composition according to the above (22) or
(23) as an active ingredient.

The cell of the present invention in which a genomic gene encoding an enzyme capable of catalyzing a dehydration reaction to convert GDP-mannose into GDP-4-keto,6-deoxy-GDP-mannose is knocked out (hereinafter referred to as the cell of the present invention) includes a cell in which a genomic gene is modified so as to have deleted activity of an enzyme capable of catalyzing a dehydration reaction to convert GDP-mannose into GDP-4-keto,6-deoxy-GDP-mannose.

In the present invention, modification of genome so as to have deleted activity of an enzyme capable of catalyzing a dehydration reaction to convert GDP-mannose into GDP-4-keto,6-deoxy-GDP-mannose means that mutation is introduced into an expression-controlling region of the gene encoding enzyme so as to delete the expression of the enzyme, or that mutation is introduced into an amino acid sequence of the gene encoding the enzyme so as to delete the function of the enzyme. Introduction of the mutation means that modification such as deletion, substitution, insertion and/or addition is carried out in the nucleotide sequence on the genome. Complete inhibition of the expression or function of the modified genomic gene is called "knocked out". Examples in which a genomic gene is knocked out include a case in which the gene as the target is completely or partially deleted from the genome. Specific examples include partial or complete deletion of the genome corresponding to the ATG region, catalytic activity site, promoter region and the like from the chromosome or deletion of all of their alleles in the gene encoding an enzyme capable of catalyzing a dehydration reaction to convert GDP-mannose into GDP-4-keto,6-deoxy-GDP-mannose. Specific examples include deletion of at least genomic region of exon 5 from the chromosome or deletion of all of alleles.

Accordingly, the cell of the present invention includes a cell in which all of alleles on a genome encoding an enzyme capable of catalyzing a dehydration reaction to convert GDP-mannose into GDP-4-keto,6-deoxy-GDP-mannose are knocked out, a cell in which the genome corresponding to the ATG region, catalytic activity site, promoter region and the like is partly or completely deleted from the chromosome or all of alleles were deleted, specifically, a cell in which at least genomic region of exon 5 is deleted from the chromosome, and the like.

The enzyme capable of catalyzing a dehydration reaction to convert GDP-mannose into GDP-4-keto,6-deoxy-GDP-mannose means an enzyme having an enzymatic activity capable of converting GDP-mannose into GDP-4-keto, 6-deoxy-GDP-mannose.

Examples of the enzyme capable of catalyzing a dehydration reaction which converts GDP-mannose into GDP-4-keto,6-deoxy-GDP-mannose include GDP-mannose 4,6-dehydratase, and the like.

In the present invention, the GDP-mannose 4,6-dehydratase includes a protein encoded by a DNA of the following (a) to (f), a protein of the following (g) to (o), and the like:

(a) a DNA comprising the nucleotide sequence represented by SEQ ID NO:1;

(b) a DNA comprising the nucleotide sequence represented by SEQ ID NO:2;
(c) a DNA comprising the nucleotide sequence represented by SEQ ID NO:3;
(d) a DNA which hybridizes with the DNA consisting of the nucleotide sequence represented by SEQ ID NO:1 under stringent conditions and encodes a protein having GDP-mannose 4,6-dehydratase activity;
(e) a DNA which hybridizes with the DNA consisting of the nucleotide sequence represented by SEQ ID NO:2 under stringent conditions and encodes a protein having GDP-mannose 4,6-dehydratase activity;
(f) : a DNA which hybridizes with the DNA consisting of the nucleotide sequence represented by SEQ ID NO:3 under stringent conditions and encodes a protein having GDP-mannose 4,6-dehydratase activity;
(g) a protein comprising the amino acid sequence represented by SEQ ID NO:4;
(h) a protein comprising the amino acid sequence represented by SEQ ID NO: 5;
(i) a protein comprising the amino acid sequence represented by SEQ ID NO:6;
(j) a protein consisting of an amino acid sequence in which one or more amino acid(s) is/are deleted, substituted, inserted and/or added in the amino acid sequence represented by SEQ ID NO:4 and having GDP-mannose 4,6-dehydratase activity;
(k) a protein consisting of an amino acid sequence in which one or more amino acid(s) is/are deleted, substituted, inserted and/or added in the amino acid sequence represented by SEQ ID NO:5 and having GDP-mannose 4,6-dehydratase activity;
(1) a protein consisting of an amino acid sequence in which one or more amino acid(s) is/are deleted, substituted, inserted and/or added in the amino acid sequence represented by SEQ ID NO:6 and having GDP-mannose 4,6-dehydratase activity;
(m) a protein consisting of an amino acid sequence which has 80% or more homology to the amino acid sequence represented by SEQ ID NO:4 and having GDP-mannose 4,6-dehydratase activity;
(n) a protein consisting of an amino acid sequence which has 80% or more homology to the amino acid sequence represented by SEQ ID NO:5 and having GDP-mannose 4,6-dehydratase activity;
(o) a protein consisting of an amino acid sequence which has 80% or more homology to the amino acid sequence represented by SEQ ID NO:6 and having GDP-mannose 4,6-dehydratase activity.

Also, the DNA encoding the amino acid sequence of GDP-mannose 4,6-dehydratase includes a DNA comprising the nucleotide sequence represented by SEQ ID NO:1, 2 or 3 and a DNA which hybridizes with the DNA consisting of the nucleotide sequence represented by SEQ ID NO:1, 2 or 3 under stringent conditions and encodes an amino acid sequence having GDP-mannose 4,6-dehydratase activity.

In the present invention, a DNA which hybridizes under stringent conditions is a DNA obtained, e.g., by a method such as colony hybridization, plaque hybridization or Southern blot hybridization using a DNA consisting of the nucleotide sequence represented by SEQ ID NO:1, 2 or 3 or a partial fragment thereof as the probe, and specifically includes a DNA which can be identified by carrying out hybridization at 65°C in the presence of 0.7 to 1.0 M sodium chloride using a filter to which colony- or plaque-derived DNA fragments are immobilized, and then washing the filter at 65°C using 0.1 to 2 × SSC solution (composition of the 1 × SSC solution comprising 150 mM sodium chloride and 15 mM sodium citrate). The hybridization can be carried out in accordance with the methods described, e.g., in *Molecular Cloning, A Laboratory Manual,* Second Edition, Cold Spring Harbor Laboratory Press (1989) (hereinafter referred to as *"Molecular Cloning, Second Edition"*), *Current Protocols in Molecular Biology,* John Wiley & Sons, 1987-1997 (hereinafter referred to as *"Current Protocols in Molecular Biology"); DNA Cloning 1: Core Techniques, A Practical Approach,* Second Edition, Oxford University (1995); and the like. The hybridizable DNA includes a DNA having at least 60% or more, preferably 70% or more, more preferably 80% or more, still more preferably 90% or more, far more preferably 95% or more, and most preferably 98% or more, of homology with the nucleotide sequence represented by SEQ ID NO: 1, 2 or 3.

In the present invention, the protein consisting of an amino acid sequence in which one or more amino acid(s) is/are deleted, substituted, inserted and/or added in the amino acid sequence represented by SEQ ID NO:4, 5 or 6 and having GDP-mannose 4,6-dehydratase activity can be obtained, e.g., by introducing a site-directed mutation into a DNA encoding a protein consisting of the amino acid sequence represented by SEQ ID NO:4, 5 or 6, respectively, using the site-directed mutagenesis described, e.g., in *Molecular Cloning, Second Edition; Current Protocols in Molecular Biology; Nucleic Acids Research,* 10, 6487 (1982); *Proc. Natl. Acad. Sci. USA,* 79, 6409 (1982); *Gene,* 34, 315 (1985); *Nucleic Acids Research,* 13, 4431 (1985); *Proc. Natl. Acad. Sci. USA,* 82, 488 (1985); and the like. The number of amino acids to be deleted, substituted, inserted and/or added is one or more, and the number is not particularly limited, but is a number which can be deleted, substituted or added by a known technique such as the above-mentioned site-directed mutagenesis, e.g., it is 1 to several tens, preferably 1 to 20, more preferably 1 to 10, and most preferably 1 to 5.

Also, in the present invention, the protein consisting of an amino acid sequence which has 80% or more homology to the amino acid sequence represented by SEQ ID NO:4, 5 or 6 and having GDP-mannose 4,6-dehydratase activity is a protein having at least 80% or more homology, preferably 85% or more homology, more preferably 90% or

more homology, still more preferably 95% or more homology, far more preferably 97% or more homology, and most preferably 99% or more homology, to the protein consisting of the amino acid sequence represented by SEQ ID NO:4, 5 or 6, when calculated by using an analyzing soft such as BLAST [*J. Mol. Biol.,* 215, 403 (1990)], FASTA [*Methods in Enzymology,* 183, 63 (1990)] or the like.

As a method for obtaining the cell of the present invention, any technique can be used, so long as the genome of interest can be modified. However, genetic engineering techniques are preferred. Examples include:

(a) a gene disruption technique which comprises targeting at a gene encoding an enzyme capable of catalyzing a dehydration reaction which converts GDP-mannose into GDP-4-keto,6-deoxy-GDP-mannose,

(b) a technique for introducing mutation into a gene encoding an enzyme capable of catalyzing a dehydration reaction which converts GDP-mannose into GDP-4-keto,6-deoxy-GDP-mannose, and the like.

Furthermore, the cell of the present invention can be selected by using a method for selecting a cell line resistant to a lectin which recognizes a sugar chain structure in which 1-position of fucose is bound to 6-position of *N*-acetylglucosamine in the reducing end through α-bond in the complex type N-glycoside-linked sugar chain.

The cell which is resistant to lectin means a cell in which growth is not inhibited in the presence of a lectin at an effective concentration. The effective concentration is a concentration in which the cell before the genomic gene is knocked out (hereinafter also referred to as the parent cell) cannot normally grow or higher than the concentration, and is a concentration which is preferably similar to, more preferably 2 to 5 times, still more preferably 10 times, and most preferably 20 times or more, higher than the concentration in which the cell before the genomic gene is knocked out cannot grow.

In the present invention, the effective concentration of a lectin which does not inhibit the growth can be decided depending on the cell line, and is generally 10 μg/ml to 10 mg/ml, preferably 0.5 to 2.0 mg/ml.

As the lectin which recognizes a sugar chain structure in which 1-position of fucose is bound to 6-position of *N*-acetylglucosamine in the reducing end through α-bond in the N-glycoside-linked sugar chain, any lectin can be used, so long as it can recognize the sugar chain structure. Examples include a *Lens culinaris* lectin LCA (lentil agglutinin derived from *Lens culinaris),* a pea lectin PSA (pea lectin derived from *Pisum sativum),* a broad bean lectin VFA (agglutinin derived from *Vicia faba),* an *Aleuria aurantia* lectin AAL (lectin derived from *Aleuria aurantia)* and the like.

The cell of the present invention may be any cell, so long as it can express a glycoprotein. Examples include a yeast, an animal cell, an insect cell, a plant cell and the like, and specific examples include cells described in the item 3 below. The animal cell includes a CHO cell derived from a Chinese hamster ovary tissue, a rat myeloma cell line YB2/3HL.P2.G11.16Ag.20 cell, a mouse myeloma cell line NSO cell, a mouse myeloma SP2/0-Ag14 cell, a BHK cell derived from a Syrian hamster kidney tissue, an antibody producing-hybridoma cell, a human leukemia cell line Namalwa cell, an embryonic stem cell, a fertilized egg cell, a plant cell and the like. Preferable examples include the above myeloma cell and hybridoma cell used for producing an antibody composition, a host cell for producing a humanized antibody and a human antibody, an embryonic stem cell and fertilized egg cell for preparing a non-human transgenic animal which produces a human antibody, a plant cell for preparing a transgenic plant which produces a humanized antibody and a human antibody, and the like.

Also, as the cell of the present invention, the cell having an ability to a glycoprotein such as an antibody composition having higher antigen-dependent cell-mediated cytotoxic activity than that of an antibody composition produced by a cell before the genomic gene is knocked out.

The cell before the genomic gene is knocked out is a cell before a method for knocking out a genomic gene encoding an enzyme capable of catalyzing a dehydration reaction to convert GDP-mannose into GDP-4-keto,6-deoxy-GDP-mannose is applied. The cell before the genomic gene is knocked out is not particularly limited, and includes, as NSO cell before the genomic gene is knocked out, NSO cells described in literatures such as *BIO/TECHNOLOGY,* 10, 169 (1992) and *Biotechnol. Bioeng.,* 73, 261 (2001). Further examples include NS0 cell line (RCB 0213) registered at RIKEN Cell Bank, The Institute of Physical and Chemical Research, sub-cell lines obtained by naturalizing these cell lines to serum-free media, and the like.

SP2/0-Ag14 cell before the genomic gene is knocked out includes SP2/0-Ag14 cells described in literatures such as *J. Immunol.,* 126, 317 (1981), *Nature,* 276, 269 (1978) and *Human Antibodies and Hybridomas,* 3, 129 (1992). Further examples include SP2/0-Ag14 cell (ATCC CRL-1581) registered at ATCC, sub-cell lines obtained by naturalizing these cell lines to serum-free media (ATCC CRL-1581.1), and the like.

CHO cell derived from Chinese hamster ovary tissue before the genomic gene is knocked out includes CHO cells described in literatures such as *Journal of Experimental Medicine,* 108, 945 (1958), *Proc. Natl. Acad. Sci. USA,* 60, 1275 (1968), *Genetics,* 55, 513 (1968), *Chromosoma,* 41, 129 (1973), *Methods in Cell Science,* 18, 115 (1996), *Radiation Research,* 148, 260 (1997), *Proc. Natl. Acad. Sci. USA,* 77, 4216 (1980), *Proc. Natl. Acad. Sci. USA,* 60, 1275 (1968), *Cell,* 6, 121 (1975) and *Molecular Cell Genetics,* Appendix I, II (p. 883-900). Further examples include cell line CHO-K1 (ATCC CCL-61), cell line DUXB11 (ATCC CRL-9096) and cell line Pro-5 (ATCC CRL-1781) registered at

ATCC, commercially available cell line CHO-S (Cat # 11619 of Life Technologies), sub-cell lines obtained by naturalizing these cell lines to serum-free media, and the like.

A rat myeloma cell line YB2/3HL.P2.G11.16Ag.20 cell before the genomic gene is knocked out includes cell lines established from Y3/Agl.2.3 cell (ATCC CRL-1631). Specific examples include YB2/3HL.P2.G11.16Ag.20 cell described in literatures such as *J. Cell. Biol.,* 93, 576 (1982) and *Methods Enzymol.,* 73B, 1 (1981). Further examples include YB2/3HL.P2.G11.16Ag.20 cell (ATCC CRL-1662) registered at ATCC, sub-lines obtained by naturalizing these cell lines to serum-free media, and the like.

In the cell of the present invention, among the enzymes relating to the sugar chain structure of a glycoprotein, an enzyme relating to the modification of fucose is inactivated. Accordingly, fucose is not added to a glycoprotein produced by a cell of the present invention prepared by introducing a gene encoding a glycoprotein, so that a glycoprotein composition having high physiological activity can be produced.

Examples of the glycoprotein composition having high physiological activity include a glycoprotein composition having improved affinity with a receptor, a glycoprotein composition having improved half-life in blood, a glycoprotein composition in which its tissue distribution after administration into blood is changed, and a glycoprotein composition in which its interaction with a protein necessary for expressing pharmacological activity is improved.

Accordingly, any glycoprotein composition is included in the glycoprotein composition produced by the present invention, so long as it is a glycoprotein composition in which the produced protein has a sugar chain structure in which fucose is bound, when it is produced by a cell before the genomic gene is knocked out. Examples include an antibody, erythropoietin, thrombopoietin, tissue type plasminogen activator, prourokinase, thrombomodulin, antithrombin III, protein C, blood coagulation factor VII, blood coagulation factor VIII, blood coagulation factor IX, blood coagulation factor X, blood coagulation factor XII, gonadotropic hormone, thyroid-stimulating hormone, epidermal growth factor (EGF), hepatocyte growth factor (HGF), keratinocyte growth factor, activin, bone formation factor, stem cell factor (SCF), interferon α, interferon β, interferon γ, interleukin 2, interleukin 6, interleukin 10, interleukin 11, soluble interleukin 4 receptor, tumor necrosis factor α, DNase I, galactosidase, α-glucosidase, glucocerebrosidase and the like.

Sugar chains binding to a glycoprotein are roughly classified into two types, namely a sugar chain which binds to asparagine (N-glycoside-linked sugar chain) and a sugar chain which binds to other amino acid such as serine or threonine (O-glycoside-linked sugar chain), based on the binding form to the protein moiety. They are generically called glycoside-linked sugar chain.

The N-glycoside-linked sugar chains have various structures, but have a common core structure shown by the following formula (I) [*Biochemical Experimentation Method 23 - Method for Studying Glycoprotein Sugar Chain* (Gakujutsu Shuppan Center), edited by Reiko Takahashi (1989)]:

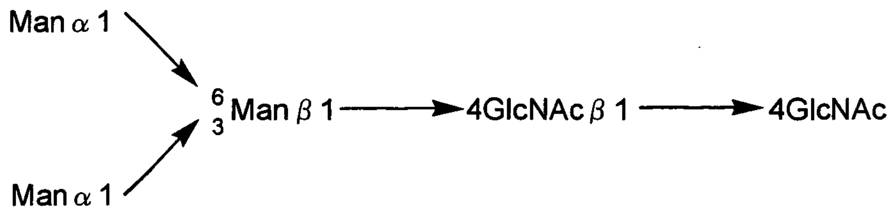

Formula (I)

In formula (I), the sugar chain terminus which binds to asparagine is called a reducing end, and the opposite side is called a non-reducing end.

The N-glycoside-linked sugar chain includes a high mannose type sugar chain in which mannose alone binds to the non-reducing end of the core structure; a complex type sugar chain (hereinafter referred to as the complex type) which the non-reducing end side of the core structure has one or plurality of parallel branches of galactose-*N*-acetylglucosamine (hereinafter referred to as "Gal-GlcNAc") and the non-reducing end side of Gal-GlcNAc further has a structure of sialic acid, bisecting *N*-acetylglucosamine or the like; a hybrid type sugar chain in which the non-reducing end side of the core structure comprises branches of both of the high mannose type and complex type; and the like.

Examples of the O-glycoside-linked sugar chain include a sugar chain in which the reducing end of N-acetylgalactosamine is bound to a hydroxyl group of serine or threonine through α-bond and further bound to galactose, N-acetylglucosamine, N-acetylgalactosamine, fucose or sialic acid, a sugar chain in which xylose is bound to a hydroxyl group of serine through β-bond, a sugar chain in which galactose is bound to a hydroxyl group of hydroxylysine through β-bond and the like.

In a sugar chain in which xylose is bound to a hydroxyl group of serine through β-bond, plurality of saccharides are generally bound to the 4-position of the xylose, and a straight chain polysaccharide consisting of disaccharides is bound to the top of the bound saccharide. Cartilage proteoglycan and the like can be exemplified as the substance

having such a sugar chain structure. Collagen and the like can be exemplified as the substance having a sugar chain structure in which galactose is bound to a hydroxyl group of hydroxylysine through β-bond.

The glycoprotein composition means a composition comprising a glycoprotein molecule having a complex type N-glycoside-linked sugar chain or an O-glycoside-linked sugar chain. The sugar chains which bind to glycoproteins are present in a large numbers and their sugar chain structures are variable, so that a combination of a large number of sugar chains is present in the sugar chain of a glycoprotein.

Accordingly, the glycoprotein composition which is prepared by using the cell of the present invention may comprise a glycoprotein molecule bound to the same sugar chain structure or a glycoprotein molecule bound to different sugar chain structures, so long as the effect of the present invention can be obtained.

The glycoprotein composition prepared by the cell of the present invention is preferably a glycoprotein composition having sugar chains in which fucose is not bound to N-acetylgalactosamine in the reducing end in complex type N-glycoside-linked sugar chains, and sugar chains in which fucose is not bound to N-acetylgalactosamine in the non-reducing end in O-glycoside-linked sugar chains.

The glycoprotein composition having sugar chains in which fucose is not bound to N-acetylgalactosamine in the reducing end in complex type N-glycoside-linked sugar chains, and sugar chains in which fucose is not bound to N-acetylgalactosamine in the non-reducing end in O-glycoside-linked sugar chains includes a glycoprotein composition wherein the ratio of the sugar chains in which fucose is not bound to *N*-acetylglucosamine in the reducing end in complex type N-glycoside-linked sugar chains and the sugar chains in which fucose is not bound to N-acetylgalactosamine in the non-reducing end in O-glycoside-linked sugar chains, among the total glycoside-linked sugar chains bound to the amino acid region in the glycoprotein composition, is 100%

The glycoprotein composition having sugar chains in which fucose is not bound means a glycoprotein composition in which fucose is not substantially detected by the sugar chain analysis described in the following item 6. Herein, the fucose is not substantially detected means that the content is lower than the detection limit for the measurement.

Furthermore, in the present invention, the glycoprotein composition which is prepared by using a transgenic non-human animal or plant or the progenies thereof in which a genomic gene encoding an enzyme capable of catalyzing a hydration reaction which converts GDP-mannose into GDP-4-keto,6-deoxy-GDP-mannose is knocked out may comprise a glycoprotein molecule having the same sugar chain structure or glycoprotein molecules having different sugar chain structures, so long as the effect of the present invention can be obtained. The glycoprotein composition is preferably a glycoprotein composition having sugar chains in which fucose is not bound to N-acetylglucosamine in the reducing end in complex type N-glycoside-linked sugar chains and sugar chains in which fucose is not bound to N-acetylgalactosamine in the non-reducing end in O-glycoside-linked sugar chains, among the total complex glycoside-linked sugar chains bound to the amino acid region in the glycoprotein composition.

In the non-human animal or plant or the progenies thereof before the genomic gene is knocked out (hereinafter referred to as the parent individual), a glycoprotein composition which is modified with fucose is prepared.

The transgenic non-human animal or plant or the progenies thereof in which a genomic gene capable of catalyzing a dehydration reaction which converts GDP-mannose into GDP-4-keto,6-deoxy-GDP-mannose is knocked out can be prepared by using an embryonic stem cell, a fertilized egg or a plant cell according to the present invention.

In the glycoprotein composition produced by the present invention, when the ratio of sugar chains in which fucose is not bound to *N*-acetylglucosamine in the reducing end in complex type N-glycoside-linked sugar chains and sugar chains in which fucose is not bound to N-acetylgalactosamine in the non-reducing end in O-glycoside-linked sugar chains, among the total N-glycoside-linked sugar chains bound to the sugar chain-binding amino acid region, is higher than those in a glycoprotein composition produced by the cell or parent individual before the genomic gene is knocked out, the glycoprotein composition produced in the present invention has higher physiological activity than the glycoprotein composition produced by the cell or parent individual.

The ratio of sugar chains in which fucose is not bound to N-acetylglucosamine in the reducing end in complex type N-glycoside-linked sugar chains and sugar chains in which fucose is not bound to N-acetylgalactosamine in the non-reducing end in O-glycoside-linked sugar chains in a composition comprising the glycoprotein molecules bound to the glycoside-linked sugar chains can be determined by releasing the sugar chains from the glycoprotein molecule by known methods such as hydrazinolysis and enzyme digestion [*Seibutsukagaku Jikkenho (Biochemical Experimentation Methods) 23 - Totanpakushitsu Tosa Kenkyuho (Methods of Studies on Glycoprotein Sugar Chains),* Gakkai Shuppan Center, edited by Reiko Takahashi (1989)], labeling the released sugar chains with a fluorescent substance or radioisotope, and separating the labeled sugar chains by chromatography. Alternatively, the released sugar chains may be analyzed by the HPAED-PAD method [*J. Liq. Chromatogr.,* 6, 1577 (1983)].

Specific examples of the glycoprotein having remarkably improved physiological activity by having a sugar chain structure to which fucose is not bound include an antibody composition.

The antibody composition is a composition which comprises an antibody molecule having a complex type N-glycoside-linked sugar chain in the Fc region.

The antibody is a tetramer in which two molecules of each of two polypeptide chains, a heavy chain and a light

chain, are respectively associated. Each of about a quarter of the *N*-terminal side of the heavy chain and about a quarter of the *N*-terminal side of the light chain (more than 100 amino acids for each) is called variable region which is rich in diversity and directly relates to the binding to an antigen. The greater part of the moiety other than the variable region is called constant region. Based on homology with the constant region, antibody molecules are classified into classes IgG, IgM, IgA, IgD and IgE.

Also, the IgG class is further classified into subclasses IgG1 to IgG4 based on homology with the constant region.

The heavy chain is classified into four immunoglobulin domains VH, CH1, CH2 and CH3 from its *N*-terminal side, and a highly flexible peptide region called hinge region is present between CH1 and CH2 to divide CH1 and CH2. A structural unit comprising CH2 and CH3 after the hinge region is called Fc region to which N-glycoside-linked sugar chain is bound and is also a region to which an Fc receptor, a complement and the like are bound *(Immunology Illustrated,* the Original, 5th edition, published on February 10, 2000, by Nankodo; *Handbook of Antibody Technology (Kotai Kogaku Nyumon),* 1st edition on January 25, 1994, by Chijin Shokan).

Since the Fc region in the antibody molecule has positions to which N-glycoside-linked sugar chains are separately bound, two sugar chains are bound per one antibody molecule. The N-glycoside-linked sugar chains which bind to antibody molecules have various sugar chain structures, but any sugar chain structure comprises the core structure represented by the above formula (I). A number of combinations of sugar chains are present for the two N-glycoside-linked sugar chains which bind to the antibody.

Accordingly, the antibody composition which is prepared by using the cell of the present invention may comprise an antibody molecule having the same sugar chain structure or an antibody molecule having different sugar chain structures, so long as the effect of the present invention is obtained.

The antibody composition prepared by the cell of the present invention is preferably an antibody composition having, among the total complex type glycoside-linked sugar chains bound to the Fc region in the antibody composition, sugar chains in which fucose is not bound to N-acetylglucosamine in the reducing end in the sugar chains.

The cell of the present invention can produce an antibody composition having higher antibody-dependent cell-mediated cytotoxic activity than an antibody composition produced by a cell before the genomic gene is knocked out.

The sugar chains in which fucose is not bound to N-acetylglucosamine includes complex type N-glycoside-linked sugar chains in which 1-position of fucose is not bound to 6-position of N-acetylglucosamine in the reducing end through $\alpha$-bond in the complex type N-glycoside sugar chains.

Furthermore, in the present invention, the antibody composition which is prepared by using a transgenic non-human animal or plant or the progenies thereof in which a genomic gene encoding an enzyme capable of catalyzing a hydration reaction which converts GDP-mannose into GDP-4-keto,6-deoxy-GDP-mannose is knocked out may comprise an antibody molecule having the same sugar chain structure or antibody molecules having different sugar chain structures, so long as the effect of the present invention can be obtained. The antibody composition is preferably an antibody composition having, among the total complex glycoside-linked sugar chains bound to the Fc region in the antibody composition, sugar chains in which fucose is not bound to N-acetylglucosamine in the reducing end in the sugar chains.

The antibody composition having sugar chains in which fucose is not bound to N-acetylglucosamine in the reducing end in sugar chains, among the total complex type glycoside-linked sugar chains bound to the Fc region in the antibody composition, includes an antibody composition in which the ratio of sugar chains in which fucose is not bound to N-acetylglucosamine in the reducing end in the sugar chains, among the total complex type glycoside-linked sugar chain bound to the Fc region in the antibody composition, is 100%.

The antibody composition having sugar chains in which fucose is not bound means an antibody composition in which fucose in such a degree that fucose is not substantially detected by the sugar chain analysis described in the following item 6. Herein, the fucose is not substantially detected means that the content is lower than the detection limit for the measurement.

The transgenic non-human animal or plant or the progenies thereof in which a genomic gene capable of catalyzing a dehydration reaction which converts GDP-mannose into GDP-4-keto,6-deoxy-GDP-mannose is knocked out can be prepared by using an embryonic stem cell, a fertilized egg or a plant cell according to the present invention.

In the antibody composition produced by the present invention, when the ratio of sugar chains in which fucose is not bound to *N*-acetylglucosamine in the reducing end, among the total N-glycoside-linked sugar chains bound to the Fc region, is higher than that of an antibody composition produced by the cell or parent individual before the genomic gene is knocked out, the antibody composition produced in the present invention has higher ADCC activity than the antibody composition comprising the antibody molecule produced by the cell or parent individual.

The ADCC activity is a cytotoxic activity in which an antibody bound to a cell surface antigen existed on a tumor cell and the like in the living body activates an effector cell through an Fc receptor existing on the antibody Fc region and effector cell surface and thereby injure the tumor cell and the like [*Monoclonal Antibodies: Principles and Applications,* Wiley-Liss, Inc., Chapter 2.1 (1955)]. The effector cell includes a killer cell, a natural killer cell, an activated macrophage and the like.

The ratio of a sugar chain in which fucose is not bound to *N*-acetylglucosamine in the reducing end in the sugar

chain contained in the composition which comprises an antibody molecule having complex type N-glycoside-linked sugar chains in the Fc region can be determined by releasing the sugar chain from the antibody molecule by using a known method such as hydrazinolysis, enzyme digestion or the like [*Biochemical Experimentation Methods 23 - Method for Studying Glycoprotein Sugar Chain* (Japan Scientific Societies Press), edited by Reiko Takahashi (1989)], carrying out fluorescence labeling or radioisotope labeling of the released sugar chain and then separating the labeled sugar chain by chromatography. Also, the released sugar chain can also be analyzed and determined by the HPAED-PAD method [*J Liq. Chromatogr.,* 6, 1577 (1983)].

Also, the antibody of the present invention is preferably an antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen are exemplified below, and preferably belongs to IgG class.

The antibody which recognizes a tumor-related antigen includes anti-GD2 antibody [*Anticancer Res.,* 13, 331 (1993)], anti-GD3 antibody [*Cancer Immunol. Immunother.,* 36, 260 (1993)], anti-GM2 antibody [*Cancer Res.,* 54, 1511 (1994)], anti-HER2 antibody [*Proc. Natl. Acad. Sci. USA,* 89, 4285-(1992)], anti-CD52 antibody *[Nature,* 332, 323 (1998)], anti-MAGE antibody [*British J. Cancer,* 83, 493 (2000)], anti-HM1.24 antibody [*Molecular Immunol.,* 36, 387 (1999)], anti-parathyroid hormone-related protein (PTHrP) antibody [*Cancer,* 88, 2909 (2000)], anti-FGF8 antibody [*Proc. Natl. Acad Sci. USA,* 86, 9911 (1989)], anti-basic fibroblast growth factor antibody, anti-FGF8 receptor antibody [*J. Biol. Chem.,* 265, 16455 (1990)], anti-basic fibroblast growth factor receptor antibody, anti-insulin-like growth factor antibody [*J. Neurosci. Res.,* 40, 647 (1995)], anti-insulin-like growth factor receptor antibody [*J. Neurosci. Res.,* 40, 647 (1995)], anti-PMSA antibody [*J. Urology,* 160, 2396 (1998)], anti-vascular endothelial cell growth factor antibody [*Cancer Res.,* 57, 4593 (1997)], anti-vascular endothelial cell growth factor receptor antibody [*Oncogene,* 19, 2138 (2000)], anti-CA125 antibody, anti-17-1A antibody, anti-integrin α5β3 antibody, anti-CD33 antibody, anti-CD22 antibody, anti-HLA antibody, anti-HLA-DR antibody, anti-CD20 antibody, anti-CD19 antibody, anti-EGF receptor antibody [*Immunology Today,* 21, 403 (2000)], anti-CD10 antibody [*American Journal of Clinical Pathology,* 113, 374 (2000)] and the like.

The antibody which recognizes an allergy- or inflammation-related antigen includes anti-interleukin 6 antibody [*Immunol. Rev.,* 127, 5 (1992)], anti-interleukin 6 receptor antibody [*Molecular Immunol.,* 31, 371 (1994)], anti-interleukin 5 antibody [*Immunol. Rev.,* 127, 5 (1992)], anti-interleukin 5 receptor antibody and anti-interleukin 4 antibody [*Cytokine,* 3, 562 (1991)], anti-interleukin 4 receptor antibody [*J. Immunol. Meth.,* 217, 41 (1998)], anti-tumor necrosis factor antibody [*Hybridoma,* 13, 183 (1994)], anti-tumor necrosis factor receptor antibody [*Molecular Pharmacol.,* 58, 237 (2000)], anti-CCR4 antibody [*Nature,* 400, 776 (1999)], anti-chemokine antibody *[J. Immuno. Meth.,* 174, 249 (1994)], anti-chemokine receptor antibody [*J. Exp. Med.,* 186, 1373 (1997)], anti-IgE antibody, anti-CD23 antibody, anti-CD11a antibody [*Immunology Today,* 21, 403 (2000)], anti-CRTH2 antibody [*J Immunol.,* 162, 1278 (1999)], anti-CCR8 antibody (WO99/25734), anti-CCR3 antibody (US6207155) and the like.

The antibody which recognizes a cardiovascular disease-related antigen includes anti-GpIIb/IIIa antibody [*J. Immunol.,* 152, 2968 (1994)], anti-platelet-derived growth factor antibody [*Science,* 253, 1129 (1991)], anti-platelet-derived growth factor receptor antibody [*J. Biol. Chem.,* 272, 17400 (1997)], anti-blood coagulation factor antibody [*Circulation,* 101, 1158 (2000)] and the like.

The antibody which recognizes an antigen relating to autoimmune diseases (psoriasis, rheumarthritis, Crohn's diseases, colitis ulcerosa, systemic erythematodes, multiple sclerosis, *etc.)* includes an anti-auto-DNA antibody [*Immunol. Letters,* 72, 61 (2000)], anti-CD11a antibody, anti-ICAM3 antibody, anti-CD80 antibody, anti-CD2 antibody, anti-CD3 antibody, anti-CD4 antibody, anti-integrin α4β7 antibody, anti-GD40L antibody, anti-IL-2 receptor antibody [*Immunology Today,* 21, 403 (2000)], and the like.

The antibody which recognizes a viral or bacterial infection-related antigen includes anti-gp120 antibody *[Structure,* 8, 385 (2000)], anti-CD4 antibody [*J. Rheumatology,* 25, 2065 (1998)], anti-CCR4 antibody, anti-Vero toxin antibody [*J. Clin. Microbiol.,* 37, 396 (1999)], , and the like.

The antibody molecule may be any antibody molecule, so long as it comprises the Fc region of an antibody. Examples include an antibody, an antibody fragment, a fusion protein comprising an Fc region, and the like.

The antibody is a protein which is produced in the living body by immune reaction as a result of exogenous antigen stimulation and has an activity to specifically bind to the antigen. Examples include an antibody secreted by a hybridoma cell prepared from a spleen cell of an animal immunized with an antigen; an antibody prepared by a genetic recombination technique, namely an antibody obtained by introducing an antibody gene-inserted antibody expression vector into a host cell; and the like. Specific examples include an antibody produced by a hybridoma, a humanized antibody, a human antibody and the like.

A hybridoma is a cell which is obtained by cell fusion between a B cell obtained by immunizing a non-human mammal with an antigen and a myeloma cell derived from mouse, rat or the like and can produce a monoclonal antibody having the desired antigen specificity.

The humanized antibody includes a human chimeric antibody, a human CDR-grafted antibody and the like.

A human chimeric antibody is an antibody which comprises a heavy chain variable region (hereinafter referred

to as "HV" or "VH", as the variable region being referred to as V region) and a light chain variable region (hereinafter referred to as "LV" or "VL", as the light region being referred to as L region), both of a non-human animal antibody, a human antibody heavy chain constant region (hereinafter also referred to as "CH") and a human antibody light chain constant region (hereinafter also referred to as "CL"). The non-human animal may be any animal such as mouse, rat, hamster, rabbit or the like, so long as a hybridoma can be prepared therefrom.

The human chimeric antibody can be produced by obtaining cDNAs encoding VH and VL from a monoclonal antibody-producing hybridoma, inserting them into an expression vector for host cell having genes encoding human antibody CH and human antibody CL to thereby construct a vector for expression of human chimeric antibody, and then introducing the vector into a host cell to express the antibody.

The CH of human chimeric antibody may be any CH, so long as it belongs to human immunoglobulin (hereinafter referred to as "hIg"). Those belonging to the hIgG class are preferred and any one of the subclasses belonging to the hIgG class, such as hIgG1, hIgG2, hIgG3 and hIgG4, can be used. Also, as the CL of human chimeric antibody, any CL can be used, so long as it belongs to the hIg class, and those belonging to the κ class or λ class can also be used.

A human CDR-grafted antibody is an antibody in which amino acid sequences of CDRs of VH and VL of a non-human animal antibody are grafted into appropriate positions of VH and VL of a human antibody.

The human CDR-grafted antibody can be produced by constructing cDNAs encoding V regions in which CDRs of VH and VL of a non-human animal antibody are grafted into CDRs of VH and VL of a human antibody, respectively inserting them into an expression vector for host cell having genes encoding human antibody CH and human antibody CL to thereby construct a human CDR-grafted antibody expression vector, and then introducing the expression vector into a host cell to express the human CDR-grafted antibody.

The CH of human CDR-grafted antibody may be any CH, so long as it belongs to the hIg. Those belonging to the hIgG class are preferred and any one of the subclasses belonging to the hIgG class, such as hIgG1, hIgG2, hIgG3 and hIgG4, can be used. Also, as the CL of human CDR-grafted antibody, any CL can be used, so long as it belongs to the hIg class, and those belonging to the κ class or λ class can also be used.

A human antibody is originally an antibody naturally existing in the human body, but it also includes antibodies obtained from a human antibody phage library, a human antibody-producing transgenic non-human animal and a human antibody-producing transgenic plant, which are prepared based on the recent advance in genetic engineering, cell engineering and developmental engineering techniques.

Regarding the antibody existing in the human body, a lymphocyte capable of producing the antibody can be cultured by isolating a human peripheral blood lymphocyte, immortalizing it by infecting with EB virus or the like and then cloning it, and the antibody can be purified from the culture.

The human antibody phage library is a library in which antibody fragments such as Fab, single chain antibody and the like are expressed on the phage surface by inserting a gene encoding an antibody prepared from a human B cell into a phage gene. A phage expressing an antibody fragment having the desired antigen binding activity can be recovered from the library based on its binding activity to an antigen-immobilized substrate. The antibody fragment can be converted further into a human antibody molecule comprising two full H chains and two full L chains by genetic engineering techniques.

A human antibody-producing transgenic non-human animal is a non-human animal in which a human antibody gene is introduced into cells. Specifically, a human antibody-producing transgenic animal can be prepared by introducing a human antibody gene into embryonic stem cell of a mouse, transplanting the embryonic stem cell into an early stage embryo of other mouse and then developing it. The human antibody-producing transgenic non-human animal can also be prepared by introducing a human antibody gene into a fertilized egg of an animal and developing it. Regarding the preparation method of a human antibody from the human antibody-producing transgenic non-human animal, the human antibody can be formed and accumulated in a culture by obtaining a human antibody-producing hybridoma by a hybridoma preparation method usually carried out in non-human mammals and then culturing it.

The transgenic non-human animal includes cattle, sheep, goat, pig, horse, mouse, rat, fowl, monkey, rabbit and the like.

Also, in the present invention, it is preferred that the antibody is an antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen, and a human antibody which belongs to the IgG class is preferred.

An antibody fragment is a fragment which comprises at least a part of Fc region of the above antibody. The Fc region is CH2 region and CH3 region, which is a region at the C-terminal of H chain of an antibody, and includes a natural type and a mutant type. The part of Fc region is preferably a fragment which comprises CH2 region, and more preferably region which comprises aspartic acid at position 1 existing in the CH2 region.. The Fc region of the IgG class is from Cys at position 226 to the C-terminal or from Pro at position 230 to the C-terminal according to the numbering of EU Index of *Kabat et al.* [*Sequences of Proteins of Immunological Interest,* 5th Ed., Public Health Service, National

Institutes of Health, Bethesda, MD. (1991)]. The antibody fragment includes an H chain monomer, an H chain dimer and the like.

A fusion protein comprising a part of Fc region may be any fusion protein so long as it is a protein in which an antibody comprising the Fc region of an antibody or the antibody fragment is fused with a protein such as an enzyme or a cytokine (hereinafter referred to as "Fc fusion protein").

The present invention is explained below in detail.

1. Preparation of cell of the present invention

The cell of the present invention can be prepared by the following techniques.

(1) Gene disruption technique which comprises targeting gene encoding enzyme

The cell of the present invention can be prepared by using a gene disruption technique by targeting a genomic gene encoding an enzyme capable of catalyzing a dehydration reaction to convert GDP-mannose into GDP-4-keto,6-deoxy-GDP-mannose. The enzyme capable of catalyzing a dehydration reaction to convert GDP-mannose into GDP-4-keto,6-deoxy-GDP-mannose includes GDP-mannose 4,6-dehydratase and the like.

The gene disruption method may be any method, so long as it can disrupt the gene of the target enzyme. Examples include a homologous recombination method, an RNA-DNA oligonucleotide (RDO) method, a method using retrovirus, a method using transposon, and the like. The methods are specifically described below.

(a) Preparation of the cell of the present invention by homologous recombination

The cell of the present invention can be produced by modifying a target gene on chromosome through a homologous recombination technique for targeting at a gene encoding the enzyme capable of catalyzing a dehydration reaction to convert GDP-mannose into GDP-4-keto,6-deoxy-GDP-mannose.

The target gene on the chromosome can be modified by using a method described in *Manipulating the Mouse Embryo, A Laboratory Manual,* Second Edition, Cold Spring Harbor Laboratory Press (1994) (hereinafter referred to as *"Manipulating the Mouse Embryo, A Laboratory Manual"*); *Gene Targeting, A Practical Approach,* IRL Press at Oxford University Press (1993); *Biomanual Series 8, Gene Targeting, Preparation of Mutant Mice using ES Cells,* Yodo-sha (1995) (hereinafter referred to as *"Preparation of Mutant Mice using ES Cells"*); or the like, for example, as follows.

A cDNA encoding the enzyme capable of catalyzing a dehydration reaction to convert GDP-mannose into GDP-4-keto,6-deoxy-GDP-mannose is prepared.

Based on the obtained cDNA, a genomic DNA encoding the enzyme capable of catalyzing a dehydration reaction to convert GDP-mannose into GDP-4-keto,6-deoxy-GDP-mannose is prepared.

Based on the nucleotide sequence of the genomic DNA, a target vector is prepared for homologous recombination of a target gene to be modified (e.g., structural gene of the enzyme capable of catalyzing a dehydration reaction to convert GDP-mannose into GDP-4-keto,6-deoxy-GDP-mannose, or a promoter gene).

The cell of the present invention can be produced by introducing the prepared target vector into a host cell and selecting a cell in which homologous recombination generated between the target gene and target vector.

As the host cell, any cell such as yeast, an animal cell, an insect cell, a plant cell or the like can be used, so long as it has the target gene encoding the enzyme capable of catalyzing a dehydration reaction to convert GDP-mannose into GDP-4-keto,6-deoxy-GDP-mannose. Examples include cells described in the following item 3.

The method for obtaining a cDNA or a genomic DNA encoding the enzyme capable of catalyzing a dehydration reaction to convert GDP-mannose into GDP-4-keto,6-deoxy-GDP-mannose includes the method described below.

<u>Preparation method of cDNA:</u>

A total RNA or mRNA is prepared from various host cells.

A cDNA library is prepared from the prepared total RNA or mRNA.

Degenerative primers are produced based on the known amino acid sequence of the enzyme capable of catalyzing a dehydration reaction to convert GDP-mannose into GDP-4-keto,6-deoxy-GDP-mannose, e.g., human amino acid sequence, and a gene fragment encoding the enzyme capable of catalyzing a dehydration reaction to convert GDP-mannose into GDP-4-keto,6-deoxy-GDP-mannose is obtained by PCR using the prepared cDNA library as the template.

A cDNA encoding the enzyme capable of catalyzing a dehydration reaction to convert GDP-mannose into GDP-4-keto,6-deoxy-GDP-mannose can be obtained by screening the cDNA library by using the obtained gene fragment as a probe.

As the mRNA of various host cells, a commercially available product (e.g., manufactured by Clontech) may be

used or may be prepared from various host cells as follows. The method for preparing a total RNA from various host cells includes the guanidine thiocyanate-cesium trifluoroacetate method [*Methods in Enzymology,* 154, 3 (1987)], the acidic guanidine thiocyanate phenol chloroform (AGPC) method [*Analytical Biochemistry,* 162, 156 (1987); *Experimental Medicine. (Jikken Igaku),* 9, 1937 (1991)] and the like.

Furthermore, the method for preparing mRNA as poly(A)$^+$ RNA from a total RNA includes the oligo(dT)-immobilized cellulose column method *(Molecular Cloning, Second Edition)* and the like.

In addition, mRNA can be prepared by using a kit such as Fast Track mRNA Isolation Kit (manufactured by Invitrogen), Quick Prep mRNA Purification Kit (manufactured by Pharmacia) or the like.

A cDNA library is prepared from the prepared mRNA of various host cells. The method for preparing cDNA libraries includes the methods described in *Molecular Cloning, Second Edition; Current Protocols in Molecular Biology; A Laboratory Manual,* Second Edition (1989); and the like, or methods using commercially available kits such as SuperScript Plasmid System for cDNA Synthesis and Plasmid Cloning (manufactured by Life Technologies), ZAP-cDNA Synthesis Kit (manufactured by STRATAGENE) and the like.

As the cloning vector for the preparation of the cDNA library, any vector such as a phage vector, a plasmid vector or the like can be used, so long as it is autonomously replicable in *Escherichia coli* K12. Examples include ZAP Express [manufactured by STRATAGENE, *Strategies,* 5, 58 (1992)], pBluescript II SK(+) [*Nucleic Acids Research,* 17, 9494 (1989)], Lambda ZAP II (manufactured by STRATAGENE), λgt10 and λgt11 [DNA Cloning, A Practical Approach, 1, 49 (1985)], λTriplEx (manufactured by Clontech), λExCell (manufactured by Pharmacia), pT7T318U (manufactured by Pharmacia), pcD2 [*Mol. Cell. Biol.,* 3, 280 (1983)], pUC18 [*Gene,* 33, 103 (1985)] and the like.

Any microorganism can be used as the host microorganism for preparing the cDNA library, and *Escherichia coli* is preferably used. Examples include *Escherichia coli* XL1-Blue MRF' [manufactured by STRATAGENE, *Strategies,* 5, 81 (1992)], *Escherichia coli* C600 [*Genetics,* 39, 440 (1954)], *Escherichia coli* Y1088 [*Science,* 222, 778 (1983)], *Escherichia coli* Y1090 [*Science,* 222, 778 (1983)], *Escherichia coli* NM522 [*J. Mol. Biol.,* 166, 1 (1983)], *Escherichia coli* K802 [*J. Mol. Biol.,* 16, 118 (1966)], *Escherichia coli* JM105 [*Gene,* 38, 275 (1985)] and the like.

The cDNA library can be used as such in the subsequent analysis, and in order to obtain a full length cDNA as efficient as possible by decreasing the ratio of a cDNA fragment containing a partial coding sequence, a cDNA library prepared by using the oligo cap method developed by *Sugano et al.* [*Gene,* 138, 171 (1994); *Gene,* 200, 149 (1997); *Protein, Nucleic Acid, Enzyme,* 41, 603 (1996); *Experimental Medicine (Jikken Igaku),* 11, 2491 (1993); *cDNA Cloning* (Yodo-sha) (1996); *Methods for Preparing Gene Libraries* (Yodo-sha) (1994)] can be used in the following analysis.

Based on the amino acid sequence of the enzyme capable of catalyzing a dehydration reaction to convert GDP-mannose into GDP-4-keto,6-deoxy-GDP-mannose, degenerative primers specific for the 5'-terminal and 3'-terminal nucleotide sequences of a nucleotide sequence presumed to encode the amino acid sequence are prepared, and DNA is amplified by PCR [*PCR Protocols,* Academic Press (1990)] using the prepared cDNA library as the template to obtain a gene fragment encoding the enzyme capable of catalyzing a dehydration reaction to convert GDP-mannose into GDP-4-keto,6-deoxy-GDP-mannose.

It can be confirmed that the obtained gene fragment is a DNA encoding the enzyme capable of catalyzing a dehydration reaction to convert GDP-mannose into GDP-4-keto,6-deoxy-GDP-mannose by a method generally used for analyzing a nucleotide, such as the dideoxy method of Sanger *et al.* [*Proc. Natl. Acad. Sci. USA,* 74, 5463 (1977)], a nucleotide sequence analyzer such as ABI PRISM 377 DNA Sequencer (manufactured by PE Biosystems) or the like.

A DNA encoding the enzyme capable of catalyzing a dehydration reaction to convert GDP-mannose into GDP-4-keto,6-deoxy-GDP-mannose can be obtained by carrying out colony hybridization or plaque hybridization *(Molecular Cloning, Second Edition)* for the cDNA or cDNA library synthesized from the mRNA contained in the various host cells, using the gene fragment as a DNA probe.

Also, a DNA encoding the enzyme capable of catalyzing a dehydration reaction to convert GDP-mannose into GDP-4-keto,6-deoxy-GDP-mannose can also be obtained by carrying out screening by PCR using the cDNA or cDNA library synthesized from the mRNA contained in the various host cells as the template and using the primers used for obtaining the gene fragment encoding the enzyme capable of catalyzing a dehydration reaction to convert GDP-mannose into GDP-4-keto,6-deoxy-GDP-mannose.

The nucleotide sequence of the obtained DNA encoding the enzyme capable of catalyzing a dehydration reaction to convert GDP-mannose into GDP-4-keto, 6-deoxy-GDP-mannose is analyzed from its terminus and determined by a method generally used for analyzing a nucleotide, such as the dideoxy method of Sanger *et al.* [*Proc. Natl. Acad. Sci. USA,* 74, 5463 (1977)], a nucleotide sequence analyzer such as ABI PRISM 377 DNA Sequencer (manufactured by PE Biosystems) or the like.

A gene encoding the enzyme capable of catalyzing a dehydration reaction to convert GDP-mannose into GDP-4-keto,6-deoxy-GDP-mannose can also be determined from genes in data bases by searching nucleotide sequence data bases such as GenBank, EMBL, DDBJ and the like by using a homology retrieving program such as BLAST based on the determined cDNA nucleotide sequence.

The nucleotide sequence of the gene encoding the enzyme capable of catalyzing a dehydration reaction to

convert GDP-mannose into GDP-4-keto,6-deoxy-GDP-mannose includes the nucleotide sequence represented by SEQ ID NO: 1, 2 or 3.

The cDNA encoding the enzyme capable of catalyzing a dehydration reaction to convert GDP-mannose into GDP-4-keto,6-deoxy-GDP-mannose can also be obtained by chemically synthesizing it with a DNA synthesizer such as DNA Synthesizer model 392 manufactured by Perkin Elmer or the like by using the phosphoamidite method, based on the determined DNA nucleotide sequence.

As an example of the method for preparing a genomic DNA encoding the enzyme capable of catalyzing a dehydration reaction to convert GDP-mannose into GDP-4-keto,6-deoxy-GDP-mannose, the method described below is exemplified.

Preparation method of genomic DNA:

The method for preparing genomic DNA includes known methods described in *Molecular Cloning, Second Edition; Current Protocols in Molecular Biology;* and the like. In addition, a genomic DNA encoding the enzyme capable of catalyzing a dehydration reaction to convert GDP-mannose into GDP-4-keto,6-deoxy-GDP-mannose can also be isolated by using a kit such as Genome DNA Library Screening System (manufactured by Genome Systems), Universal Genome Walker™ Kits (manufactured by CLONTECH) or the like.

The nucleotide sequence of the genomic DNA encoding the enzyme capable of catalyzing a dehydration reaction to convert GDP-mannose into GDP-4-keto,6-deoxy-GDP-mannose includes the nucleotide sequence represented by any one of SEQ ID NOs:7, 8, 9 and 10.

The target vector used in the homologous recombination of the target gene can be prepared in accordance with a method described in *Gene Targeting, A Practical Approach,* IRL Press at Oxford University Press (1993); *Biomanual Series 8, Gene Targeting, Preparation of Mutant Mice using ES Cells,* Yodo-sha (1995); or the like. The target vector can be used as both a replacement type and an insertion type.

For introducing the target vector into various host cells, the methods for introducing recombinant vectors suitable for various host cells described in the following item 3 can be used.

The method for efficiently selecting a homologous recombinant includes a method such as the positive selection, promoter selection, negative selection or polyA selection described in *Gene Targeting, A Practical Approach,* IRL Press at Oxford University Press (1993); *Biomanual Series 8, Gene Targeting, Preparation of Mutant Mice using ES Cells,* Yodo-sha (1995); or the like. The method for selecting the homologous recombinant of interest from the selected clones includes the Southern hybridization method for genomic DNA *(Molecular Cloning, Second Edition),* PCR [*PCR Protocols,* Academic Press (1990)], and the like.

A homologous recombinant can be obtained based on the change of the activity of an enzyme capable of catalyzing a dehydration reaction to convert GDP-mannose into GDP-4-keto,6-deoxy-GDP-mannose. The following method is exemplified as a method for selecting a transformant.

Method for selecting transformant:

The method for selecting a cell in which the genomic gene encoding the enzyme capable of catalyzing a dehydration reaction to convert GDP-mannose into GDP-4-keto,6-deoxy-GDP-mannose is knocked out includes biochemical methods or genetic engineering techniques described in literatures such as *New Biochemical Experimentation Series 3-Saccharides I, Glycoprotein* (Tokyo Kagaku Dojin), edited by Japanese Biochemical Society (1988); *Cell Engineering, Supplement, Experimental Protocol Series, Glycobiology Experimental Protocol, Glycoprotein, Glycolipid and Proteoglycan* (Shujun-sha), edited by Naoyuki Taniguchi, Akemi Suzuki, Kiyoshi Furukawa and Kazuyuki Sugawara (1996); *Molecular Cloning, Second Edition; Current Protocols in Molecular Biology;* and the like. The biochemical method includes a method in which the enzyme activity is evaluated by using an enzyme-specific substrate and the like. The genetic engineering technique includes the Northern analysis, RT-PCR and the like which measures the amount of mRNA of a gene encoding the enzyme.

Furthermore, the method for selecting a cell based on morphological change caused by knocking out the gneomic gene encoding the enzyme capable of catalyzing a dehydration reaction to convert GDP-mannose into GDP-4-keto,6-deoxy-GDP-mannose includes a method for selecting a transformant based on the sugar chain structure of a produced antibody molecule, a method for selecting a transformant based on the sugar chain structure of a glycoprotein on a cell membrane, and the like. The method for selecting a transformant using the sugar chain structure of an antibody-producing molecule includes method described in the following item 6. The method for selecting a transformant based on the sugar chain structure of a glycoprotein on a cell membrane includes a method selecting a clone resistant to a lectin which recognizes a sugar chain structure wherein 1-position of fucose is bound to 6-position of *N*-acetylglucosamine in the reducing end through $\alpha$-bond in the complex type N-glycoside-linked sugar chain. Examples include a method using a lectin described in *Somatic Cell Mol. Genet.,* 12, 51 (1986).

As the lectin, any lectin can be used, so long as it is a lectin which recognizes a sugar chain structure in which 1-position of fucose is bound to 6-position of *N*-acetylglucosamine in the reducing end through α-bond in the N-glycoside-linked sugar chain. Examples include a *Lens culinaris* lectin LCA (lentil agglutinin derived from *Lens culinaris),* a pea lectin PSA (pea lectin derived from *Pisum sativum),* a broad bean lectin VFA (agglutinin derived from *Vicia faba),* an *Aleuria aurantia* lectin AAL (lectin derived from *Aleuria aurantia)* and the like.

Specifically, the host cell of the present invention can be selected by culturing cells for 1 day to 2 weeks, preferably 3 days to 1 week, in a medium comprising the above-mentioned lectin at a concentration of several ten μg/ml to several mg/ml, preferably 0.5 to 2.0 mg/ml, subculturing surviving cells or picking up a colony and transferring it into another culture vessel, and subsequently continuing the culturing in the lectin-containing medium.

(b) Preparation of the cell of the present invention by RDO method

The cell of the present invention can be prepared by an RDO method by targeting a gene encoding the enzyme capable of catalyzing a dehydration reaction to convert GDP-mannose into GDP-4-keto,6-deoxy-GDP-mannose, for example, as follows.

A cDNA or a genomic DNA encoding the enzyme capable of catalyzing a dehydration reaction to convert GDP-mannose into GDP-4-keto,6-deoxy-GDP-mannose is prepared.

The nucleotide sequence of the prepared cDNA or genomic DNA is determined.

Based on the determined DNA sequence, an RDO construct of an appropriate length comprising a part encoding the enzyme capable of catalyzing a dehydration reaction to convert GDP-mannose into GDP-4-keto,6-deoxy-GDP-mannose, a part of an untranslated region or a part of intron is designed and synthesized.

The cell of the present invention can be obtained by introducing the synthesized RDO into a host cell and then selecting a transformant in which a mutation generated in the target enzyme capable of catalyzing a dehydration reaction to convert GDP-mannose into GDP-4-keto,6-deoxy-GDP-mannose.

As the host cell, any cell such as yeast, an animal cell, an insect cell, a plant cell or the like can be used, so long as it has a gene encoding the enzyme capable of catalyzing a dehydration reaction to convert GDP-mannose into GDP-4-keto,6-deoxy-GDP-mannose. Examples include the host cells described in the following item 3.

The method for introducing RDO into various host cells includes the methods for introducing recombinant vectors suitable for various host cells, described in the following item 3.

The method for preparing cDNA encoding the enzyme capable of catalyzing a dehydration reaction to convert GDP-mannose into GDP-4-keto,6-deoxy-GDP-mannose includes the methods for "Preparation method of cDNA" described in the item 1(1)(a) and the like.

The method for preparing a genomic DNA encoding the enzyme capable of catalyzing a dehydration reaction to convert GDP-mannose into GDP-4-keto,6-deoxy-GDP-mannose includes the methods for "Preparation method of genomic DNA" described in the item 1(1)(a) and the like.

The nucleotide sequence of the DNA can be determined by digesting it with appropriate restriction enzymes, cloning the DNA fragments into a plasmid such as pBluescript SK(-) (manufactured by Stratagene), subjecting the clones to the reaction generally used as a method for analyzing a nucleotide sequence such as the dideoxy method of Sanger *et al.* [*Proc. Natl. Acad Sci. USA,* 74, 5463 (1977)] or the like, and then analyzing the clones by using an automatic nucleotide sequence analyzer such as A.L.F. DNA Sequencer (manufactured by Pharmacia) or the like.

The RDO can be prepared by a usual method or using a DNA synthesizer.

The method for selecting a cell in which a mutation occurred, by introducing the RDO into the host cell, in the target enzyme, i.e., the gene encoding the enzyme capable of catalyzing a dehydration reaction to convert GDP-mannose into GDP-4-keto,6-deoxy-GDP-mannose, includes the methods for directly detecting mutations in chromosomal genes described in *Molecular Cloning, Second Edition, Current Protocols in Molecular Biology* and the like.

Furthermore, "Method for selecting transformant" described in the item 1(1)(a) based on the change of the activity of the enzyme capable of catalyzing a dehydration reaction to convert GDP-mannose into GDP-4-keto,6-deoxy-GDP-mannose can also be used.

The construct of the RDO can be designed in accordance with the methods described in *Science,* 273, 1386 (1996); *Nature Medicine,* 4, 285 (1998); *Hepatology,* 25, 1462 (1997); *Gene Therapy,* 5, 1960 (1999); *J. Mol. Med.,* 75, 829 (1997); *Proc. Natl. Acad Sci. USA,* 96, 8774 (1999); *Proc. Natl. Acad Sci. USA,* 96, 8768 (1999); *Nuc. Acids. Res.,* 27, 1323 (1999); *Invest. Dematol.,* 111, 1172 (1998); *Nature Biotech.,* 16, 1343 (1998); *Nature Biotech.,* 18, 43 (2000); *Nature Biotech.,* 18, 555 (2000); and the like.

(c) Preparation of the cell of the present invention by method using transposon

The cell of the present invention can be prepared by inducing mutation using a transposon system described in *Nature Genet.,* 25, 35 (2000) or the like, and by selecting a mutant based on the activity of the enzyme capable of

catalyzing a dehydration reaction to convert GDP-mannose into GDP-4-keto,6-deoxy-GDP-mannose, or the sugar chain structure of a produced antibody molecule or of a glycoprotein on the cell membrane.

The transposon system is a system in which a mutation is induced by randomly inserting an exogenous gene into chromosome, wherein an exogenous gene interposed between transposons is generally used as a vector for inducing a mutation, and a transposase expression vector for randomly inserting the gene into chromosome is introduced into the cell at the same time.

Any transposase can be used, so long as it is suitable for the sequence of the transposon to be used.

As the exogenous gene, any gene can be used, so long as it can induce a mutation in the DNA of a cell.

As the cell, any cell such as yeast, an animal cell, an insect cell, a plant cell or the like can be used, so long as it has a gene encoding the target enzyme capable of catalyzing a dehydration reaction to convert GDP-mannose into GDP-4-keto,6-deoxy-GDP-mannose. Examples include the host cells described in the following item 3.

For introducing a gene into the cell, the method for introducing recombinant vectors suitable for various host cells described in the following item 3 can be used.

The method for selecting a mutant based on the activity of the enzyme capable of catalyzing a dehydration reaction to convert GDP-mannose into GDP-4-keto,6-deoxy-GDP-mannose includes "Method for selecting transformant" described in the above item 1(1)(a) based on change of the activity of the enzyme capable of catalyzing a dehydration reaction to convert GDP-mannose into GDP-4-keto,6-deoxy-GDP-mannose.

(2) Method for introducing mutation into enzyme

The cell of the present invention can be prepared by introducing a mutation into a gene encoding the enzyme capable of catalyzing a dehydration reaction to convert GDP-mannose into GDP-4-keto,6-deoxy-GDP-mannose, and then by selecting a clone of interest in which the mutation generated in the enzyme.

The enzyme capable of catalyzing a dehydration reaction to convert GDP-mannose into GDP-4-keto,6-deoxy-GDP-mannose includes GDP-mannose 4,6-dehydratase and the like.

The method includes 1) a method in which a desired clone is selected from mutants obtained by a mutation-inducing treatment of a parent cell line or spontaneously generated mutants based on the change of the activity of the enzyme capable of catalyzing a dehydration reaction to convert GDP-mannose into GDP-4-keto,6-deoxy-GDP-mannose, 2) a method in which a desired clone is selected from mutants obtained by a mutation-inducing treatment of a parent cell line or spontaneously generated mutants based on the sugar chain structure of a produced antibody molecule and 3) a method in which a desired clone is selected from mutants obtained by a mutation-inducing treatment of a parent cell line or spontaneously generated mutants based on the sugar chain structure of a glycoprotein on the cell membrane.

As the mutation-inducing treatment, any treatment can be used, so long as it can induce a point mutation, a deletion or frame shift mutation in the DNA of the parent cell line. Examples include treatment with ethyl nitrosourea, nitrosoguanidine, benzopyrene or an acridine pigment and treatment with radiation. Also, various alkylating agents and carcinogens can be used as mutagens. The method for allowing a mutagen to act upon cells includes the methods described in *Tissue Culture Techniques,* 3rd edition (Asakura Shoten), edited by Japanese Tissue Culture Association (1996), *Nature Genet.,* 24, 314 (2000) and the like.

The spontaneously generated mutant includes mutants which are spontaneously formed by continuing subculture under general cell culture conditions without applying special mutation-inducing treatment.

The method for selecting a clone of interest based on the change of the activity of the enzyme capable of catalyzing a dehydration reaction to convert GDP-mannose into GDP-4-keto,6-deoxy-GDP-mannose, the method for selecting a clone of interest based on the sugar chain structure of a prepared antibody molecule and the method for selecting a clone of interest based on the sugar chain structure of a glycoprotein on the cell membrane include "Method for selecting transformant" described in the above item 1(1)(a) based on change of the activity of the enzyme capable of catalyzing a dehydration reaction to convert GDP-mannose into GDP-4-keto,6-deoxy-GDP-mannose.

2. Preparation of the transgenic non-human animal or plant or the progenies thereof of the present invention

The transgenic non-human animal or plant or the progenies thereof of the present invention is a transgenic non-human animal or plant or the progenies thereof in which a genomic gene is modified in such a manner that the activity of an enzyme capable of catalyzing a dehydration reaction to convert GDP-mannose into GDP-4-keto,6-deoxy-GDP-mannose can be deleted, and it can be prepared according to a known method from an embryonic stem cell, fertilized egg cell or plant callus cell according to the method described in the item 1, by targeting at a gene encoding the enzyme capable of catalyzing a dehydration reaction to convert GDP-mannose into GDP-4-keto,6-deoxy-GDP-mannose.

The enzyme capable of catalyzing a dehydration reaction to convert GDP-mannose into GDP-4-keto,6-deoxy-GDP-mannose includes GDP-mannose 4,6-dehydratase and the like.

A specific method is described below.

In a transgenic non-human animal, the embryonic stem cell of the present invention in which the activity of the enzyme capable of catalyzing a dehydration reaction to convert GDP-mannose into GDP-4-keto,6-deoxy-GDP-mannose is deleted can be prepared by applying the method described in the item 1 to an embryonic stem cell of the intended non-human animal such as cattle, sheep, goat, pig, horse, mouse, rat, fowl, monkey, rabbit or the like.

Specifically, a mutant clone is prepared in which a gene encoding the enzyme capable of catalyzing a dehydration reaction to convert GDP-mannose into GDP-4-keto,6-deoxy-GDP-mannose is inactivated or substituted with any sequence, by a known homologous recombination technique [e.g., *Nature,* 326, 6110, 295 (1987); *Cell,* 51, 3, 503 (1987); or the like]. Using the prepared embryonic stem cell such as the mutant clone, a chimeric individual comprising an embryonic stem cell clone and a normal cell can be prepared by an injection chimera method into blastocyst of fertilized egg of an animal or by an aggregation chimera method. The chimeric individual is crossed with a normal individual, so that a transgenic non-human animal in which the activity of the enzyme capable of catalyzing a dehydration reaction to convert GDP-mannose into GDP-4-keto,6-deoxy-GDP-mannose is deleted in the whole body cells can be obtained.

The target vector for the homologous recombination of the target gene can be prepared in accordance with a method described in *Gene Targeting, A Practical Approach,* IRL Press at Oxford University Press (1993); *Biomanual Series 8, Gene Targeting, Preparation of Mutant Mice using ES Cells,* Yodo-sha (1995) or the like. The target vector can be used as any of a replacement type, an insertion type and a gene trap type.

As the method for introducing the target vector into the embryonic stem cell, any method can be used, so long as it can introduce DNA into an animal cell. Examples include electroporation [*Cytotechnology,* 3, 133 (1990)], the calcium phosphate method (Japanese Published Unexamined Patent Application No. 227075/90), the lipofection method [*Proc. Natl. Acad. Sci. USA,* 84, 7413 (1987)], the injection method [*Manipulating the Mouse Embryo,* Second Edition], a method using particle gun (gene gun) (Japanese Patent No. 2606856, Japanese Patent No. 2517813), the DEAE-dextran method [*Biomanual Series 4-Gene Transfer and Expression Analysis* (Yodo-sha), edited by Takashi Yokota and Kenichi Arai (1994)], the virus vector method [*Manipulating Mouse Embryo,* Second Edition] and the like.

The method for efficiently selecting a homologous recombinant includes a method such as the positive selection, promoter selection, negative selection or polyA selection described in *Gene Targeting, A Practical Approach,* IRL Press at Oxford University Press (1993), or the like. Specifically, in the case of the target vector containing hprt gene, it is introduced into the hprt gene-defected embryonic stem cell, the embryonic stem cell is cultured in a medium comprising aminopterin, hypoxanthine and thymidine, and positive selection which selects the homologous recombinant comprising hprt gene can be carried out by selecting an aminopterin-resistant clone. In the case of the target vector comprising a neomycin-resistant gene, the vector-introduced embryonic stem cell is cultured in a medium comprising G418, and selection of homologous recombinant comprising a neomycin-resistant gene can be carried out by selecting G418-resistant clone. In the case of the target vector comprising DT gene, since the DT gene is expressed while integrated in the chromosome, the recombinants introduced into a chromosome at random other than the homogenous recombination cannot grow due to the toxicity of DT. The vector-introduced embryonic stem cell is cultured, and negative selection of a DT gene-free homogenous recombinant can be carried out by selecting the grown clone. The method for selecting the homogenous recombinant of interest among the selected clones include the Southern hybridization for genomic DNA *(Molecular Cloning,* Second Edition), PCR [*PCR Protocols,* Academic Press (1990)] and the like.

When the embryonic stem cell is introduced into a fertilized egg by using an aggregation chimera method, in general, a fertilized egg at the development stage before 8-cell stage is preferably used. When the embryonic stem cell is introduced into a fertilized egg by using an injection chimera method, in general, it is preferred that a fertilized egg at the development stage from 8-cell stage to blastocyst stage is preferably used.

When the fertilized egg is transplanted into a female mouse, it is preferred that a fertilized egg obtained from a pseudopregnant female mouse in which fertility is induced by mating with a male non-human mammal which is subjected to vasoligation is artificially transplanted or implanted. Although the psuedopregnant female mouse can be obtained by natural mating, the pseudopregnant female mouse in which fertility is induced can be obtained by mating with a male mouse after administration of a luteinizing hormone-releasing hormone (hereinafter referred to as "LHRH") or its analogue thereof The analogue of LHRH includes [3,5-Dil-Tyr5]-LHRH, [Gln8]-LHRH, [D-Ala6]-LHRH, des-Gly10-[D-His(Bzl) 6]-LHRH ethylamide and the like.

Also, a fertilized egg cell of the present invention in which the activity of the enzyme capable of catalyzing a dehydration reaction to convert GDP-mannose into GDP-4-keto,6-deoxy-GDP-mannose is deleted can be prepared by applying the method described in the item 1 to fertilized egg of a non-human animal of interest such as cattle, sheep, goat, pig, horse, mouse, rat, fowl, monkey, rabbit or the like.

A transgenic non-human animal in which the activity of the enzyme capable of catalyzing a dehydration reaction to convert GDP-mannose into GDP-4-keto,6-deoxy-GDP-mannose is deleted can be prepared by transplanting the prepared fertilized egg cell into the oviduct or uterus of a pseudopregnant female by using the embryo transplantation method described in *Manipulating Mouse Embryo,* Second Edition or the like, followed by childbirth by the animal.

In a transgenic plant, the callus of the present invention in which the activity of the enzyme capable of catalyzing a dehydration reaction to convert GDP-mannose into GDP-4-keto,6-deoxy-GDP-mannose is deleted can be prepared by applying the method described in the item 1 to a callus or cell of the plant of interest.

A transgenic plant in which the activity of the enzyme capable of catalyzing a dehydration reaction to convert GDP-mannose into GDP-4-keto,6-deoxy-GDP-mannose is deleted can be prepared by culturing the prepared callus using a medium comprising auxin and cytokinin to redifferentite it in accordance with a known method [*Tissue Culture,* 20 (1994); *Tissue Culture,* 21 (1995); *Trends in Biotechnology,* 15, 45 (1997)].

3. Process for producing glycoprotein composition

A process for producing a glycoprotein composition using the cell of the present invention is explained below based on production of an antibody composition as a specific example.

The antibody composition can be obtained by expressing it in a host cell to which a gene encoding an antibody molecular is introduced, by using the methods described in *Molecular Cloning, Second Edition; Current Protocols in Molecular Biology; Antibodies, A Laboratory Manual,* Cold Spring Harbor Laboratory, 1988 (hereinafter sometimes referred to as *"Antibodies"); Monoclonal Antibodies: Principles and Practice,* Third Edition, Acad. Press, 1993 (hereinafter sometimes referred to as *"Monoclonal Antibodies");* and *Antibody Engineering, A Practical Approach,* IRL Press at Oxford University Press, 1996 (hereinafter sometimes referred to as *"Antibody Engineering"),* for example, as follows.

A cDNA of an antibody molecule is prepared.

Based on the prepared full length cDNA of an antibody molecule, an appropriate length of a DNA fragment comprising a moiety encoding the protein is prepared, if necessary.

A recombinant vector is prepared by inserting the DNA fragment or the full length cDNA into downstream of the promoter of an appropriate expression vector.

A transformant which produces the antibody composition of the present invention can be obtained by introducing the recombinant vector into a host cell suitable for the expression vector.

The cDNA can be prepared from a human or non-human tissue or cell using, e.g., a probe primer specific for the antibody molecule of interest, in accordance with the methods described in "Preparation method of cDNA" in the item 1(1)(a).

When yeast is used as the host cell, the expression vector includes YEP 13 (ATCC 37115), YEp24 (ATCC 37051), YCp50 (ATCC 37419) and the like.

Any promoter can be used, so long as it can function in yeast. Examples include a promoter of a gene of the glycolytic pathway such as a hexose kinase gene, PHOS promoter, PGK promoter, GAP promoter, ADH promoter, gal 1 promoter, gal 10 promoter, heat shock protein promoter, MF $\alpha$1 promoter, CUP 1 promoter and the like.

The host cell includes microorganisms belonging to the genus *Saccharomyces,* the genus *Schizosaccharomyces,* the genus *Kluyveromyces,* the genus *Trichosporon,* the genus *Schwanniomyces* and the like, such as *Saccharomyces cerevisiae, Schizosaccharomyces pombe, Kluyveromyces lactis, Trichosporon pullulans* and *Schwanniomyces alluvius.*

As the method for introducing the recombinant vector, any method can be used, so long as it can introduce DNA into yeast. Examples include electroporation *[Methods in Enzymology,* 194, 182 (1990)], the spheroplast method [*Proc. Natl. Acad. Sci. USA,* 84, 1929 (1978)], the lithium acetate method *[J. Bacteriol.,* 153, 163 (1983)], the method described in *Proc. Natl. Acad. Sci. USA,* 75, 1929 (1978) and the like.

When an animal cell is used as the host cell, the expression vector includes pcDNAI, pcDM8 (available from Funakoshi), pAGE107 [Japanese Published Unexamined Patent Application No. 22979/91; *Cytotechnology,* 3, 133 (1990)], pAS3-3 (Japanese Published Unexamined Patent Application No. 227075/90), pCDM8 [*Nature,* 329, 840 (1987)], pcDNAI/Amp (manufactured by Invitrogen), pREP4 (manufactured by Invitrogen), pAGE103 [*J. Biochemistry,* 101, 1307 (1987)], pAGE210 and the like.

Any promoter can be used, so long as it can function in an animal cell. Examples include a promoter of IE (immediate early) gene of cytomegalovirus (CMV), an early promoter of SV40, a promoter of retrovirus, a promoter of metallothionein, a heat shock promoter, an SR$\alpha$ promoter and the like. Also, an enhancer of the IE gene of human CMV may be used together with the promoter.

The host cell includes a human cell such as Namalwa cell, a monkey cell such as COS cell, a Chinese hamster cell such as CHO cell or HBT5637 (Japanese Published Unexamined Patent Application No. 299/88), a rat myeloma cell, a mouse myeloma cell, a cell derived from Syrian hamster kidney, an embryonic stem cell, a fertilized egg cell and the like.

As the method for introducing the recombinant vector, any method can be used, so long as it can introduce DNA into an animal cell. Examples include electroporation [*Cytotechnology,* 3, 133 (1990)], the calcium phosphate method (Japanese Published Unexamined Patent Application No. 227075/90), the lipofection method [*Proc. Natl. Acad Sci. USA,* 84, 7413 (1987)], the injection method [*Manipulating the Mouse Embryo, A Laboratory Manual,* Second

Edition], a method using particle gun (gene gun) (Japanese Patent No. 2606856, Japanese Patent No. 2517813), the DEAE-dextran method [*Biomanual Series 4-Gene Transfer and Expression Analysis* (Yodo-sha), edited by Takashi Yokota and Kenichi Arai (1994)], the virus vector method *(Manipulating Mouse Embryo,* Second Edition) and the like.

When an insect cell is used as the host, the protein can be expressed by the method described in *Current Protocols in Molecular Biology, Baculovirus Expression Vectors, A Laboratory Manual,* W.H. Freeman and Company, New York (1992), *Bio/Technology,* 6, 47 (1988) or the like.

The protein can be expressed by co-infecting a recombinant gene introducing vector and a baculovirus into an insect cell to obtain a recombinant virus in an insect cell culture supernatant and then infecting the insect cell with the recombinant virus.

The gene introducing vector used in the method includes pVL1392, pVL1393, pBlueBacIII (all manufactured by Invitrogen) and the like.

The baculovirus includes *Autographa californica* nuclear polyhedrosis virus which is infected by an insect of the family *Barathra.*

The insect cell includes *Spodoptera frugiperda* oocytes Sf9 and Sf21 [*Current Protocols in Molecular Biology, Baculovirus Expression Vectors, A Laboratory Manual,* W.H. Freeman and Company, New York (1992)], a *Trichoplusia ni* oocyte High 5 (manufactured by Invitrogen) and the like.

The method for the co-introducing the above recombinant gene-introducing vector and the above baculovirus into an insect cell for preparing the recombinant virus includes the calcium phosphate method (Japanese Published Unexamined Patent Application No. 227075/90), the lipofection method [*Proc. Natl. Acad. Sci. USA,* 84, 7413 (1987)] and the like.

When a plant cell is used as the host cell, the expression vector includes Ti plasmid, tobacco mosaic virus vector and the like.

As the promoter, any promoter can be used, so long as it can function in a plant cell. Examples include cauliflower mosaic virus (CaMV) 35S promoter, rice actin 1 promoter and the like.

The host cell includes plant cells of tobacco, potato, tomato, carrot, soybean, rape, alfalfa, rice, wheat, barley, waterweed and the like.

As the method for introducing the recombinant vector, any method can be used, so long as it can introduce DNA into a plant cell. Examples include a method using *Agrobacterium* (Japanese Published Unexamined Patent Application No. 140885/84, Japanese Published Unexamined Patent Application No. 70080/85, WO94/00977 electroporation (Japanese Published Unexamined Patent Application No. 251887/85), a method using a particle gun (gene gun) (Japanese Patent No. 2606856, Japanese Patent No. 2517813) and the like.

As the method for expressing a gene, secretion production, expression of a fusion protein of the Fc region with other protein and the like can be carried out in accordance with the method described in *Molecular Cloning, Second Edition* or the like, in addition to the direct expression.

When a gene is expressed by yeast, an animal cell, an insect cell, a plant cell or the like into which a gene relating to the synthesis of a sugar chain is introduced, an antibody molecule to which a sugar or a sugar chain is added by the introduced gene can be obtained.

An antibody composition can be obtained by culturing the obtained transformant in a medium to form and accumulate the antibody molecule in the culture and then recovering it from the resulting culture. The method for culturing the transformant using a medium can be carried out in accordance with a general method which is used for the culturing of host cells.

As the medium for culturing the transformant obtained by using yeast as a host, either a natural medium or a synthetic medium can be used, so long as it comprises carbon sources, nitrogen sources, inorganic salts and the like which can be assimilated by the organisms and culturing of the transformant can be carried out efficiently.

As the carbon source, those which can be assimilated by the organism can be used. Examples include carbohydrates such as glucose, fructose, sucrose, molasses containing them, starch and starch hydrolysate; organic acids such as acetic acid and propionic acid; alcohols such as ethanol and propanol; and the like.

The nitrogen source includes ammonia; ammonium salts of inorganic acid or organic acid such as ammonium chloride, ammonium sulfate, ammonium acetate and ammonium phosphate; other nitrogen-containing compounds; peptone; meat extract; yeast extract; corn steep liquor; casein hydrolysate; soybean meal; soybean meal hydrolysate; various fermented cells and hydrolysates thereof; and the like.

The inorganic material includes potassium dihydrogen phosphate, dipotassium hydrogen phosphate, magnesium phosphate, magnesium sulfate, sodium chloride, ferrous sulfate, manganese sulfate, copper sulfate, calcium carbonate, and the like.

The culturing is carried out generally under aerobic conditions such as shaking culture or submerged-aeration stirring culture. The culturing temperature is preferably 15 to 40°C, and the culturing time is generally 16 hours to 7 days. During the culturing, the pH is maintained at 3.0 to 9.0. The pH is adjusted using an inorganic or organic acid, an alkali solution, urea, calcium carbonate, ammonia or the like.

If necessary, antibiotics such as ampicillin or tetracycline may be added to the medium during the culturing.

When a microorganism transformed with a recombinant vector obtained by using an inducible promoter as the promoter is cultured, an inducer may be added to the medium, if necessary. For example, when a microorganism transformed with a recombinant vector obtained by using *lac* promoter is cultured, isopropyl-β-D-thiogalactopyranoside or the like may be added to the medium, and when a microorganism transformed with a recombinant vector obtained by using *trp* promoter is cultured, indoleacrylic acid may be added to the medium.

When a transformant obtained by using an animal cell as the host is cultured, the medium includes generally used RPMI 1640 medium [*The Journal of the American Medical Association,* 199, 519 (1967)], Eagle's MEM medium [*Science,* 122, 501 (1952)], Dulbecco's modified MEM medium [*Virology,* 8, 396 (1959)], 199 medium [*Proceeding of the Society for the Biological Medicine,* 73, 1 (1950)] and Whitten's medium [*Developmental Engineering Experimentation Manual-Preparation of Transgenic Mice* (Kodan-sha), edited by M. Katsuki (1987)], the media to which fetal calf serum, *etc.* is added, and the like.

The culturing is carried out generally at a pH of 6 to 8 and 30 to 40°C for 1 to 7 days in the presence of 5% $CO_2$.

If necessary, antibiotics such as kanamycin or penicillin may be added to the medium during the culturing.

The medium for the culturing of a transformant obtained by using an insect cell as the host includes generally used TNM-FH medium (manufactured by Pharmingen), Sf-900 II SFM medium (manufactured by Life Technologies), ExCell 400 and ExCell 405 (both manufactured by JRH Biosciences), Grace's Insect Medium [*Nature,* 195, 788 (1962)] and the like.

The culturing is carried out generally at a medium pH of 6 to 7 and 25 to 30°C for 1 to 5 days.

In addition, antibiotics such as gentamicin may be added to the medium during the culturing, if necessary.

A transformant obtained by using a plant cell as the host can be cultured as a cell or after differentiating it into a plant cell or organ. The medium for culturing the transformant includes generally used Murashige and Skoog (MS) medium and White medium, the media to which a plant hormone such as auxin, cytokinin, *etc.* is added, and the like.

The culturing is carried out generally at a pH of 5 to 9 and 20 to 40°C for 3 to 60 days.

If necessary, antibiotics such as kanamycin or hygromycin may be added to the medium during the culturing.

Accordingly, an antibody composition can be produced by culturing a transformant derived from a microorganism, an animal cell or a plant cell, which comprises a recombinant vector into which a DNA encoding an antibody molecule is inserted, in accordance with a general culturing method, to thereby form and accumulate the antibody composition, and then recovering the antibody composition from the culture.

The method for producing an antibody composition includes a method of intracellular expression in a host cell, a method of extracellular secretion from a host cell, and a method of production on a host cell membrane outer envelope. The method can be selected by changing the host cell used or the structure of the antibody composition produced.

When the antibody composition is produced in a host cell or on a host cell membrane outer envelope, it can be positively secreted extracellularly in accordance with the method of Paulson *et al.* [*J. Biol. Chem.,* 264, 17619 (1989)], the method of *Lowe et al. [Proc. Natl. Acad. Sci. USA,* 86, 8227 (1989), *Genes Develop.,* 4, 1288 (1990)], the methods described in Japanese Published Unexamined Patent Application No. 336963/93 and WO94/23021 and the like.

That is, an antibody molecule of interest can be positively secreted extracellularly from a host cell by inserting a DNA encoding the antibody molecule and a DNA encoding a signal peptide suitable for the expression of the antibody molecule into an expression vector using a gene recombination technique, introducing the expression vector into the host cell and then expressing the antibody molecule.

Also, its production amount can be increased in accordance with the method described in Japanese Published Unexamined Patent Application No. 227075/90 by using a gene amplification system using a dihydrofolate reductase gene and the like.

In addition, the antibody composition can also be produced by using a gene-introduced animal individual (transgenic non-human animal) or a plant individual (transgenic plant) which is constructed by the redifferentiation of an animal or plant cell into which the gene is introduced.

When the transformant is an animal individual or a plant individual, an antibody composition can be produced in accordance with a general method by rearing or cultivating it to thereby form and accumulate the antibody composition and then recovering the antibody composition from the animal or plant individual.

The method for producing an antibody composition by using an animal individual includes a method in which the antibody composition of interest is produced in an animal constructed by introducing a gene in accordance with a known method [*American Journal of Clinical Nutrition,* 63, 6395 (1996); *American Journal of Clinical Nutrition,* 63, 627S (1996); *Bio/Technology,* 9, 830 (1991)].

In the case of an animal individual, an antibody composition can be produced by rearing a transgenic non-human animal into which a DNA encoding an antibody molecule is introduced to thereby form and accumulate the antibody composition in the animal, and then recovering the antibody composition from the animal. The place in the animal where the composition is formed and accumulated includes milk (Japanese Published Unexamined Patent Application No. 309192/88), eggs of the animal and the like. As the promoter used in this case, any promoter can be used,

so long as it can function in an animal. Preferred examples include mammary gland cell-specific promoters such as α casein promoter, β casein promoter, β lactoglobulin promoter, whey acidic protein promoter and the like.

The method for producing an antibody composition by using a plant individual includes a method in which an antibody composition is produced by cultivating a transgenic plant into which a DNA encoding an antibody molecule is introduced by a known method [*Tissue Culture,* 20 (1994); *Tissue Culture,* 21 (1995); *Trends in Biotechnology,* 15, 45 (1997)] to form and accumulate the antibody composition in the plant, and then recovering the antibody composition from the plant.

Regarding purification of an antibody composition produced by a transformant into which a gene encoding an antibody molecule is introduced, for example, when the antibody composition is intracellularly expressed in a dissolved state, the cells after culturing are recovered by centrifugation, suspended in an aqueous buffer and then disrupted with ultrasonic oscillator, French press, Manton Gaulin homogenizer, dynomill or the like to obtain a cell-free extract. A purified product of the antibody composition can be obtained from a supernatant obtained by centrifuging the cell-free extract, by using a general enzyme isolation purification techniques such as solvent extraction; salting out and desalting with ammonium sulfate, and the like; precipitation with an organic solvent; anion exchange chromatography using a resin such as diethylaminoethyl (DEAE)-Sepharose or DIAION HPA-75 (manufactured by Mitsubishi Chemical); cation exchange chromatography using a resin such as S-Sepharose FF (manufactured by Pharmacia); hydrophobic chromatography using a resin such as butyl-Sepharose, phenyl-Sepharose; gel filtration using a molecular sieve; affinity chromatography; chromatofocusing; electrophoresis such as isoelectric focusing; and the like which may be used alone or in combination.

Also, when the antibody composition is expressed intracellularly by forming an insoluble body, the cells are recovered, disrupted and centrifuged in the same manner, and the insoluble body of the antibody composition is recovered as a precipitation fraction. The recovered insoluble body of the antibody composition is solubilized using a protein denaturing agent. The antibody composition is made into a normal three-dimensional structure by diluting or dialyzing the solubilized solution, and then a purified preparation of the antibody composition is obtained by the same isolation purification method as above.

When the antibody composition is secreted extracellularly, the antibody composition or derivatives thereof can be recovered from the culture supernatant. That is, the culture is treated by a technique such as centrifugation to obtain a soluble fraction, and a purified preparation of the antibody composition can be obtained from the soluble fraction by the same isolation purification method as above.

The thus obtained antibody composition includes an antibody, the fragment of the antibody, a fusion protein comprising the Fc region of the antibody, and the like.

As an example for obtaining the antibody composition, methods for producing a humanized antibody composition and an Fc fusion protein composition is described below in detail, but other antibody compositions can also be obtained in a manner similar to the method.

A. Preparation of humanized antibody composition

(1) Construction of vector for expression of humanized antibody

A vector for expression of humanized antibody is an expression vector for animal cell into which genes encoding the heavy chain (H chain) and light (L chain) C regions of a human antibody are inserted, which can be constructed by cloning each of genes encoding the H chain and L chain C regions of a human antibody into an expression vector for animal cell.

The C regions of a human antibody may be the H chain and L chain C regions of any human antibody. Examples include the C region belonging to IgG1 subclass in the H chain of a human antibody (hereinafter referred to as "hCγ1"), the C region belonging to κ class in the L chain of a human antibody (hereinafter referred to as "hCκ"), and the like.

As the genes encoding the H chain and L chain C regions of a human antibody, a chromosomal DNA comprising an exon and an intron can be used, and a cDNA can also be used.

As the expression vector for animal cell, any vector can be used, so long as a gene encoding the C region of a human antibody can be inserted thereinto and expressed therein. Examples include pAGE107 [*Cytotechnology,* 3, 133 (1990)], pAGE103 [*J. Biochem.,* 101, 1307 (1987)], pHSG274 [*Gene,* 27, 223 (1984)], pKCR [*Proc. Natl. Acad Sci. USA,* 78, 1527 (1981), pSG1 β d2-4 [*Cytotechnology,* 4, 173 (1990)] and the like. The promoter and enhancer in the expression vector for animal cell includes SV40 early promoter and enhancer [*J. Biochem.,* 101, 1307 (1987)], Moloney mouse leukemia virus LTR promoter [*Biochem. Biophys. Res. Commun.,* 149, 960 (1987)], immunoglobulin H chain promoter [*Cell,* 41, 479 (1985)] and enhancer [*Cell,* 33, 717 (1983)] and the like.

The vector for expression of humanized antibody may be either of a type in which genes encoding the H chain and L chain of an antibody exist on separate vectors or of a type in which both genes exist on the same vector (hereinafter referred to as "tandem type"). In respect of easiness of construction of a vector for expression of humanized antibody,

easiness of introduction into animal cells, and balance between the expression amounts of the H and L chains of an antibody in animal cells, a tandem type of the vector for expression of humanized antibody is more preferred [*J. Immunol. Methods,* 167, 271 (1994)].

The constructed vector for expression of humanized antibody can be used for expression of a human chimeric antibody and a human CDR-grafted antibody in animal cells.

(2) Obtaining of cDNA encoding V region of non-human animal antibody

cDNAs encoding the H chain and L chain V regions of a non-human animal antibody, such as a mouse antibody, can be obtained in the following manner.

A cDNA is synthesized from mRNA extracted from a hybridoma cell which produces the mouse antibody of interest. The synthesized cDNA is cloned into a vector such as a phage or a plasmid to obtain a cDNA library. Each of a recombinant phage or recombinant plasmid comprising a cDNA encoding the H chain V region and a recombinant phage or recombinant plasmid comprising a cDNA encoding the L chain V region is isolated from the library by using a C region part or a V region part of an existing mouse antibody as the probe. Full nucleotide sequences of the H chain and L chain V regions of the mouse antibody of interest on the recombinant phage or recombinant plasmid are determined, and full length amino acid sequences of the H chain and L chain V regions are deduced from the nucleotide sequences.

As the non-human animal, any animal such as mouse, rat, hamster or rabbit can be used so long as a hybridoma cell can be produced therefrom.

The method for preparing total RNA from a hybridoma cell includes the guanidine thiocyanate-cesium trifluoroacetate method [*Methods in Enzymology,* 154, 3 (1987)] and the like, and the method for preparing mRNA from total RNA includes an oligo(dT)-immobilized cellulose column method [*Molecular Cloning, A Laboratory Manual,* Cold Spring Harbor Lab. Press New York (1989)] and the like. In addition, examples of a kit for preparing mRNA from a hybridoma cell include Fast Track mRNA Isolation Kit (manufactured by Invitrogen), Quick Prep mRNA Purification Kit (manufactured by Pharmacia) and the like.

The method for synthesizing cDNA and preparing a cDNA library includes the usual methods [*Molecular Cloning, A Laboratory Manual,* Cold Spring Harbor Lab. Press New York (1989), *Current Protocols in Molecular Biology,* Supplement 1-34], methods using a commercially available kit such as SuperScript™, Plasmid System for cDNA Synthesis and Plasmid Cloning (manufactured by GIBCO BRL) or ZAP-cDNA Synthesis Kit (manufactured by Stratagene), and the like.

In preparing the cDNA library, the vector into which a cDNA synthesized by using mRNA extracted from a hybridoma cell as the template is inserted may be any vector so long as the cDNA can be inserted. Examples include ZAP Express [*Strategies,* 5,58 (1992)], pBluescript II SK(+) [*Nucleic Acids Research,* 17, 9494 (1989)], λzapII (manufactured by Stratagene), λgt10 and λgt11 [*DNA Cloning, A Practical Approach,* I, 49 (1985)], Lambda BlueMid (manufactured by Clontech), λExCell, pT7T3 18U (manufactured by Pharmacia), pcD2 [*Mol. Cell. Biol.,* 3, 280 (1983)], pUC18 [*Gene,* 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF [*Strategies,* 5, 81 (1992)], C600 [*Genetics,* 39, 440 (1954)], Y1088 and Y1090 [*Science,* 222, 778 (1983)], NM522 [*J. Mol. Biol.,* 166, 1 (1983)], K802 [*J. Mol. Biol.,* 16, 118 (1966)], JM105 [*Gene,* 38, 275 (1985)] and the like.

As the method for selecting a cDNA clone encoding the H chain and L chain V regions of a non-human animal antibody from the cDNA library, a colony hybridization or a plaque hybridization using an isotope- or fluorescence-labeled probe can be used [*Molecular Cloning, A Laboratory Manual,* Cold Spring Harbor Lab. Press New York(1989)]. The cDNA encoding the H chain and L chain V regions can also be prepared by preparing primers and carrying out polymerase chain reaction [hereinafter referred to as "PCR"; *Molecular Cloning, A Laboratory Manual,* Cold Spring Harbor Lab. Press New York (1989); *Current Protocols in Molecular Biology,* Supplement 1-34] using a cDNA synthesized from mRNA or a cDNA library as the template.

The nucleotide sequences of the cDNAs can be determined by digesting the selected cDNAs according to the above methods with appropriate restriction enzymes, cloning the DNA fragments into a plasmid such as pBluescript SK (-) (manufactured by Stratagene), carrying out the reaction of a generally used nucleotide sequence analyzing method such as the dideoxy method of Sanger *et al.* [*Proc. Natl. Acad. Sci. USA,* 74, 5463 (1977)] or the like and then analyzing the clones using an automatic nucleotide sequence analyzer such as A.L.F. DNA Sequencer (manufactured by Pharmacia) or the like.

Whether or not the obtained cDNAs encode the full length amino acid sequences of the H chain and L chain V regions of the antibody comprising a secretory signal sequence can be confirmed by deducing the full length amino acid sequences of the H chain and L chain V regions from the determined nucleotide sequence and comparing them with the full length amino acid sequences of the H chain and L chain V regions of known antibodies [*Sequences of*

*Proteins of Immunological Interest,* US Dept. Health and Human Services (1991)].

(3) Analysis of amino acid sequence of V region of non-human animal antibody

Regarding the full length amino acid sequences of the H chain and L chain V regions of the antibody comprising a secretory signal sequence, the length of the secretory signal sequence and the *N*-terminal amino acid sequences can be deduced and subgroups to which they belong can also be found, by comparing them with the full length amino acid sequences of the H chain and L chain V regions of known antibodies [*Sequences of Proteins of Immunological Interest,* US Dept. Health and Human Services, (1991)]. In addition, the amino acid sequences of each CDR of the H chain and L chain V regions can also be found by comparing them with the amino acid sequences of the H chain and L chain V regions of known antibodies [*Sequences of Proteins of immunological Interest,* US Dept. Health and Human Services, (1991)].

(4) Construction of human chimeric antibody expression vector

A human chimeric antibody expression vector can be constructed by cloning cDNAs encoding the H chain and L chain V regions of a non-human animal antibody into upstream of genes encoding the H chain and L chain C regions of a human antibody in the vector for expression of humanized antibody described in the item 3(1). For example, a human chimeric antibody expression vector can be constructed by linking each of cDNAs encoding the H chain and L chain V regions of a non-human animal antibody to a synthetic DNA comprising nucleotide sequences at the 3'-terminals of the H chain and L chain V regions of a non-human animal antibody and nucleotide sequences at the 5'-terminals of the H chain and L chain C regions of a human antibody and also having a recognition sequence of an appropriate restriction enzyme at both terminals, and by cloning them into upstream of genes encoding the H chain and L chain C regions of a human antibody contained in the vector for expression of humanized antibody described in the item 3(1).

(5) Construction of cDNA encoding V region of human CDR-grafted antibody

cDNAs encoding the H chain and L chain V regions of a human CDR-grafted antibody can be obtained as follows. First, amino acid sequences of the frameworks (hereinafter referred to as "FR") of the H chain and L chain V regions of a human antibody for grafting CDR of the H chain and L chain V regions of a non-human animal antibody is selected. As the amino acid sequences of FRs of the H chain and L chain V regions of a human antibody, any amino acid sequences can be used so long as they are derived from a human antibody. Examples include amino acid sequences of FRs of the H chain and L chain V regions of human antibodies registered at databases such as Protein Data Bank, and the like, amino acid sequences common in each subgroup of FRs of the H chain and L chain V regions of human antibodies [*Sequences of Proteins of Immunological Interest,* US Dept. Health and Human Services (1991)] and the like. In order to prepare a human CDR-grafted antibody having sufficient activities, it is preferred to select an amino acid sequence having a homology as high as possible (at least 60% or more) with amino acid sequences of the H chain and L chain V regions of a non-human animal antibody of interest.

Next, the amino acid sequences of CDRs of the H chain and L chain V regions of the non-human animal antibody of interest are grafted to the selected amino acid sequences of FRs of the H chain and L chain V regions of a human antibody to design amino acid sequences of the H chain and L chain V regions of the human CDR-grafted antibody. The designed amino acid sequences are converted into DNA sequences by considering the frequency of codon usage found in nucleotide sequences of antibody genes [*Sequences of Proteins of Immunological Interest,* US Dept. Health and Human Services (1991)], and the DNA sequences encoding the amino acid sequences of the H chain and L chain V regions of the human CDR-grafted antibody are designed. Based on the designed DNA sequences, several synthetic DNAs having a length of about 100 bases are synthesized, and PCR is carried out by using them. In this case, it is preferred in each of the H chain and the L chain that 6 synthetic DNAs are designed in view of the reaction efficiency of PCR and the lengths of DNAs which can be synthesized.

Also, they can be easily cloned into the vector for expression of humanized antibody described in the item 3 (1) by introducing recognition sequences of an appropriate restriction enzyme into the 5'-terminals of the synthetic DNA on both terminals. After the PCR, the amplified product is cloned into a plasmid such as pBluescript SK(-) (manufactured by Stratagene) and the nucleotide sequences are determined by the method in the item 3(2) to thereby obtain a plasmid having DNA sequences encoding the amino acid sequences of the H chain and L chain V regions of the desired human CDR-grafted antibody.

(6) Modification of amino acid sequence of V region of human CDR-grafted antibody

It is known that a human CDR-grafted antibody in which CDRs in the H chain and L chain V regions of a non-human animal antibody of interest are simply grafted with FRs in the H chain and L chain V regions of a human antibody has lowered antigen-binding activity than the original non-human animal antibody [*BIO/TECHNOLOGY,* 9, 266 (1991)]. As the reason, it is considered that several amino acid residues of FRs other than CDRs directly or indirectly relate to antigen-binding activity in the H chain and L chain V regions of the original non-human animal antibody, and that they are changed to different amino acid residues of FRs in the H chain and L chain V regions of a human antibody. In order to solve the problem, in human CDR-grafted antibodies, among the amino acid sequences of FRs in H chain and L chain V regions of a human antibody, an amino acid residue which directly relates to binding to an antigen, or an amino acid residue which interacts with CDR, or an amino cid residue which indirectly relates to binding to an antigen by maintaining the three-dimensional structure of an antibody is identified and modified to an amino acid residue which is found in the original non-human animal antibody to thereby increase the antigen binding activity which has been decreased [*BIO/TECHNOLOGY,* 9, 266 (1991)].

In the production of a human CDR-grafted antibody, how to efficiently identify the amino acid residues relating to the antigen binding activity in FR is most important, so that the three-dimensional structure of an antibody is constructed and analyzed by X-ray crystallography [*J. Mol. Biol.,* 112, 535 (1977)], computer-modeling [*Protein Engineering,* 7, 1501 (1994)] or the like. Although the information of the three-dimensional structure of antibodies has been useful in the production of a human CDR-grafted antibody, method for producing a human CDR-grafted antibody which can be applied to all antibodies has not been established yet. Therefore, various attempts must be currently be necessary, for example, several modified antibodies of each antibody are produced and the relationship between each of the modified antibodies and its antibody binding activity is examined.

The modification of the selected amino acid residue of FRs in H chain and L chain V regions of a human antibody can be accomplished using various synthetic DNA for modification according to PCR as described in the item 3(5). With regard to the amplified product obtained by the PCR, the nucleotide sequence is determined according to the method as described in the item 3(2) to thereby confirm whether the objective modification has been carried out.

(7) Construction of human CDR-grafted antibody expression vector

A human CDR-grafted antibody expression vector can be constructed by cloning the cDNAs encoding the H chain and L chain V regions of the human CDR-grafted antibody constructed in the items 3(5) and (6) into upstream of the gene encoding the H chain and L chain C regions of a human antibody in the vector for expression of humanized antibody described in the item 3(1). For example, recognizing sequences of an appropriate restriction enzyme are introduced into the 5'-terminals of both terminals of a synthetic DNA fragment, among the synthetic DNA fragments which are used in the items 3(5) and (6) for constructing the H chain and L chain V regions of the human CDR-grafted antibody, so that they are cloned into upstream of the genes encoding the H chain and L chain C regions of a human antibody in the vector for expression of humanized antibody described in the item 3(1) in such a manner that they can be expressed in a suitable form, to thereby construct the human CDR-grafted antibody expression vector.

(8) Stable production of humanized antibody

A transformant capable of stably producing a human chimeric antibody and a human CDR-grafted antibody (both hereinafter referred to as "humanized antibody") can be obtained by introducing the vectors for expression of humanized antibody described in the items 3(4) and (7) into an appropriate animal cell.

The method for introducing a vector for expression of humanized antibody into an animal cell includes electroporation [Japanese Published Unexamined Patent Application No. 257891/90, *Cytotechnology,* 3, 133 (1990)] and the like.

As the animal cell into which a vector for expression of humanized antibody is introduced, any cell can be used so long as it is an animal cell which can produce the humanized antibody.

Examples include mouse myeloma cells such as NS0 cell and SP2/0 cell, Chinese hamster ovary cells such as CHO/dhfr$^-$ cell and CHO/DG44 cell, rat myeloma such as YB2/0 cell and IR983F cell, BHK cell derived from a Syrian hamster kidney, a human myeloma cell such as Namalwa cell, and the like, and a Chinese hamster ovary cell CHO/DG44 cell, a rat myeloma YB2/0 cell and the cells described in the item 1 and the like are preferred.

After introduction of the vector for expression of humanized antibody, a transformant capable of stably producing the humanized antibody can be selected by using a medium for animal cell culture comprising an agent such as G418 sulfate (hereinafter referred to as "G418"; manufactured by SIGMA) and the like in accordance with the method described in Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum (hereinafter referred to as "FBS") to these media, and the like. The humanized antibody can be formed and accumulated in the

culture supernatant by culturing the obtained transformant in a medium. The amount of production and antigen binding activity of the humanized antibody in the culture supernatant can be measured by a method such as enzyme-linked immunosorbent assay [hereinafter referred to as "ELISA"; *Antibodies: A Laboratory Manual,* Cold Spring Harbor Laboratory, Chapter 14 (1998), *Monoclonal Antibodies: Principles and Practice,* Academic Press Limited (1996)] or the like. Also, the amount of the humanized antibody produced by the transformant can be increased by using a DHFR gene amplification system in accordance with the method described in Japanese Published Unexamined Patent Application No. 257891/90.

The humanized antibody can be purified from a culture supernatant of the transformant using a protein A column [*Antibodies, A Laboratory Manual,* Cold Spring Harbor Laboratory, Chapter 8 (1988), *Monoclonal Antibodies: Principles and Practice,* Academic Press Limited (1996)]. In addition, purification methods generally used for the purification of proteins can also be used. For example, the purification can be carried out through the combination of gel filtration, ion exchange chromatography, ultrafiltration and the like. The molecular weight of the H chain, L chain or antibody molecule as a whole of the purified humanized antibody can be measured, e.g., by polyacrylamide gel electrophoresis [hereinafter referred to as "SDS-PAGE"; *Nature,* 227, 680 (1970)], Western blotting [*Antibodies, A Laboratory Manual,* Cold Spring Harbor Laboratory, Chapter 12, (1988), *Monoclonal Antibodies: Principles and Practice,* Academic Press Limited (1996)] or the like.

B. Preparation of Fc fusion protein

(1) Construction of Fc fusion protein expression vector

An Fc fusion protein expression vector is an expression vector for animal cell into which genes encoding the Fc region of a human antibody and a protein to be fused are inserted, which can be constructed by cloning each of genes encoding the Fc region of a human antibody and the protein to be fused into an expression vector for animal cell.

The Fc region of a human antibody includes those comprising a part of a hinge region and/or CH1 in addition to regions comprising CH2 and CH3 regions. Also, it can be any Fc region so long as at least one amino acid of CH2 or CH3 may be deleted, substituted, added or inserted, and substantially has the binding activity to the Fcγ receptor.

As the genes encoding the Fc region of a human antibody and the protein to be fused, a chromosomal DNA comprising an exon and an intron can be used, and a cDNA can also be used. The method for linking the genes and the Fc region includes PCR using each of the gene sequences as the template *(Molecular Cloning,* Second Edition; *Current Protocols in Molecular Biology,* Supplement 1-34).

As the expression vector for animal cell, any vector can be used, so long as a gene encoding the C region of a human antibody can be inserted thereinto and expressed therein. Examples include pAGE107 [*Cytotechnology,* 3, 133 (1990)], pAGE103 [*J. Biochem.,* 101, 1307 (1987)], pHSG274 [*Gene,* 27, 223 (1984)], pKCR [*Proc. Natl. Acad. Sci. USA,* 78, 1527 (1981), pSG1 β d2-4 [*Cytotechnology,* 4, 173 (1990)] and the like. The promoter and enhancer in the expression vector for animal cell include SV40 early promoter and enhancer [*J. Biochem.,* 101, 1307 (1987)], Moloney mouse leukemia virus LTR promoter [*Biochem. Biophys. Res. Commun.,* 149, 960 (1987)], immunoglobulin H chain promoter [*Cell,* 41, 479 (1985)] and enhancer [*Cell,* 33, 717 (1983)], and the like.

(2) Obtaining of DNA encoding Fc region of human antibody and protein to be fused

A DNA encoding the Fc region of a human antibody and the protein to be fused can be obtained in the following manner.

A cDNA is synthesized from mRNA extracted from a cell or tissue which expresses the protein of interest to be fused with Fc. The synthesized cDNA is cloned into a vector such as a phage or a plasmid to obtain a cDNA library. A recombinant phage or recombinant plasmid comprising cDNA encoding the protein of interest is isolated from the library by using the gene sequence part of the protein of interest as the probe. A full nucleotide sequence of the protein of interest on the recombinant phage or recombinant plasmid is determined, and a full length amino acid sequence is deduced from the nucleotide sequence.

As the non-human animal, any animal such as mouse, rat, hamster or rabbit can be used so long as a cell or tissue can be removed therefrom.

The method for preparing a total RNA from a cell or tissue includes the guanidine thiocyanate-cesium trifluoroacetate method [*Methods in Enzymology,* 154, 3 (1987)] and the like, and the method for preparing mRNA from total RNA includes an oligo (dT)-immobilized cellulose column method *(Molecular Cloning,* Second Edition) and the like. In addition, a kit for preparing mRNA from a cell or tissue includes Fast Track mRNA Isolation Kit (manufactured by Invitrogen), Quick Prep mRNA Purification Kit (manufactured by Pharmacia) and the like.

The method for synthesizing a cDNA and preparing a cDNA library includes the usual methods (*Molecular Cloning,* Second Edition; *Current Protocols in Molecular Biology,* Supplement 1-34); methods using a commercially

available kit such as SuperScript™, Plasmid System for cDNA Synthesis and Plasmid Cloning (manufactured by GIBCO BRL) or ZAP-cDNA Synthesis Kit (manufactured by Stratagene); and the like.

In preparing the cDNA library, the vector into which a cDNA synthesized by using mRNA extracted from a cell or tissue as the template is inserted may be any vector so long as the cDNA can be inserted. Examples include ZAP Express [*Strategies,* 5, 58 (1992)], pBluescript II SK(+) [*Nucleic Acids Research,* 17, 9494 (1989)], λzapII (manufactured by Stratagene), λgt10 and λgt11 [*DNA Cloning, A Practical Approach,* I, 49 (1985)], Lambda BlueMid (manufactured by Clontech), λExCell, pT7T3 18U (manufactured by Pharmacia), pcD2 [*Mol. Cell. Biol.,* 3, 280 (1983)], pUC18 [*Gene,* 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [*Strategies,* 5, 81 (1992)], C600 [*Genetics,* 39, 440 (1954)], Y1088 and Y1090 [*Science,* 222, 778 (1983)], NM522 [*J. Mol. Biol.,* 166, 1 (1983)], K802 [*J. Mol. Biol.,* 16, 118 (1966)], JM105 [*Gene,* 38, 275 (1985)] and the like.

As the method for selecting a cDNA clone encoding the protein of interest from the cDNA library, a colony hybridization or a plaque hybridization using an isotope- or fluorescence-labeled probe can be used (*Molecular Cloning,* Second Edition). The cDNA encoding the protein of interest can also be prepared by preparing primers and using a cDNA synthesized from mRNA or a cDNA library as the template according to PCR.

The method for fusing the protein of interest with the Fc region of a human antibody includes PCR. For example, synthesized oligo DNAs (primers) are designed at the 5'-terminal and 3'-terminal of the gene sequence encoding the protein of interest, and PCR is carried out using the primers to prepare a PCR product. In the same manner, primers are designed for the gene sequence encoding the Fc region of a human antibody to be fused to prepare a PCR product. At this time, the primers are designed in such a manner that the same restriction enzyme site or the same gene sequence is present between the 3'-terminal of the PCR product of the protein to be fused and the 5'-terminal of the PCR product of the Fc region. When it is necessary to modify the amino acids around the linked site, mutation is introduced by using the primer into which the mutation is introduced. PCR is further carried out by using the two kinds of the obtained PCR fragments to link the genes. Also, they can be linked by carrying out ligation after treatment with the same restriction enzyme.

The nucleotide sequence of the DNA can be determined by digesting the gene sequence linked by the above method with appropriate restriction enzymes, cloning the DNA fragments into a plasmid such as pBluescript SK(-) (manufactured by Stratagene), carrying out analysis by using a generally used nucleotide sequence analyzing method such as the dideoxy method of Sanger *et al.* [*Proc. Natl. Acad Sci. USA,* 74, 5463 (1977)] or a nucleotide sequence analyzer such as ABI PRISM 377 DNA Sequencer (manufactured by PE Biosystems).

Whether or not the obtained cDNA encodes the full length amino acid sequences of the Fc fusion protein comprising a secretory signal sequence can be confirmed by deducing the full length amino acid sequence of the Fc fusion protein from the determined nucleotide sequence and comparing it with the amino acid sequence of interest.

(3) Stable production of Fc fusion protein

A transformant capable of stably producing an Fc fusion protein can be obtained by introducing the Fc fusion protein expression vector described in the item (1) into an appropriate animal cell.

The method for introducing the Fc fusion protein expression vector into an animal cell include electroporation [Japanese Published Unexamined Patent Application No. 257891/90, *Cytotechnology,* 3, 133 (1990)] and the like.

As the animal cell into which the Fc fusion protein expression vector is introduced, any cell can be used, so long as it is an animal cell which can produce the Fc fusion protein.

Examples include mouse myeloma cells such as NSO cell and SP2/0 cell, Chinese hamster ovary cells such as CHO/dhfr$^-$ cell and CHO/DG44 cell, rat myeloma such as YB2/0 cell and IR983F cell, BHK cell derived from a Syrian hamster kidney, a human myeloma cell such as Namalwa cell, and the like, and preferred are a Chinese hamster ovary cell CHO/DG44 cell, a rat myeloma YB2/0 cell, the host cells used in the method of the present invention described in the item 1 and the like.

After introduction of the Fc fusion protein expression vector, a transformant capable of stably producing the Fc fusion protein can be selected by using a medium for animal cell culture comprising an agent such as G418 in accordance with the method described in Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum to these media, and the like. The Fc fusion protein can be formed and accumulated in the culture supernatant by culturing the obtained transformant in a medium. The amount of production and antigen binding activity of the Fc fusion protein in the culture supernatant can be measured by a method such as ELISA. Also, the amount of the Fc fusion

protein produced by the transformant can be increased by using a *dhfr* gene amplification system and the like in accordance with the method described in Japanese Published Unexamined Patent Application No. 257891/90.

The Fc fusion protein can be purified from a culture supernatant culturing the transformant by using a protein A column or a protein G column *(Antibodies,* Chapter 8; *Monoclonal Antibodies).* In addition, purification methods generally used for the purification of proteins can also be used. For example, the purification can be carried out through the combination of a gel filtration, an ion exchange chromatography and an ultrafiltration. The molecular weight as a whole of the purified Fc fusion protein molecule can be measured by SDS-PAGE [*Nature,* 227, 680 (1970)], Western blotting (*Antibodies,* Chapter 12, *Monoclonal Antibodies*) or the like.

Thus, the processes for producing an antibody composition using an animal cell as the host have been described, but, as described above, the antibody can also be produced by a microorganism, a yeast, an insect cell, a plant cell, an animal individual or a plant individual.

When a cell has the ability to express a glycoprotein such as an antibody molecule innately, the antibody composition or glycoprotein of the present invention can be produced by preparing a producing cell using the method described in the item 1, culturing the cell and then purifying the antibody or glycoprotein of interest from the resulting culture.

4. Activity evaluation of glycoprotein composition

Methods for measuring a protein amount of the purified glycoprotein composition, affinity between the glycoprotein composition to its receptor, half-life of the glycoprotein composition in blood, distribution in tissue after the glycoprotein is administered into the living body and change of interaction between a protein necessary for expression of pharmacological activity and the glycoprotein composition are measured by known methods described in *Current Protocols In Protein Science,* John Wiley & Sons Inc., (1995); *New Biochemical Experimentation Series 19-Animal Experimental Test,* Tokyo Kagaku Dojin, edited by Japanese Biochemical Society (1991); *New Biochemical Experimentation Series 8-Intracellular Information and Cell Response,* Tokyo Kagaku Dojin, edited by Japanese Biochemical Society (1990); *New Biochemical Experimentation Series 9-Hormone I, Peptide hormone,* Tokyo Kagaku Dojin, edited by Japanese Biochemical Society (1991); *Experimental Biologicay Course 3-Isotope Experimental Test,* Maruzen (1982); *Monoclonal Antibodies: Principles and Applications,* Wiley-Liss, Inc., (1995); *Enzyme-Linked Immuno Adsorbent Assay,* 3rd Ed., Igaku Shoin (1987); *Revised Enzyme Immunoassay,* Gakusai Kikaku (1985); and the like.

Specific examples include a method in which a purified glycoprotein composition is labeled with a compound such as a radioisotope and binding activity to a receptor of the labeled glycoprotein composition or an interacted protein is quantitatively determined. Furthermore, interaction between the proteins can be measured by using various apparatus such as BIAcore Series manufactured by Biacore *(J. Immnunol. Methods,* 145, 229 (1991); *Experimental Medicine Supplement, Biomanual UP Series, Experimental Test of Intermolecular Interaction Experimental Test,* Yodo-sha (1996)).

By administration of the labeled glycoprotein composition into the living body, the half-life in blood and the distribution of the glycoprotein in tissue after administered into the living body can be observed. Detection of the labeled material is preferably carried out by a detection method in which a method for detecting a labeled substance is combined with an antigen-antibody reaction using an antibody specific to the glycoprotein composition which is to be detected.

5. Activity evaluation of antibody composition

As the method for measuring the amount of the purified antibody composition, the binding activity of the antibody composition to an antigen and the effector function of the antibody composition, the known method described in *Monoclonal Antibodies, Antibody Engineering* and the like can be used.

For example, when the antibody composition is a humanized antibody, the binding activity of the antibody composition to an antigen and the binding activity of the antibody composition to an antigen-positive cultured clone can be measured by methods such as ELISA and an immunofluorescent method [*Cancer Immunol. Immunother.,* 36, 373 (1993)]. The cytotoxic activity of the antibody composition against an antigen-positive cultured clone can be evaluated by measuring CDC activity, ADCC activity [*Cancer Immunol. Immunother.,* 36, 373 (1993)] and the like.

Also, safety and therapeutic effect of the antibody composition in human can be evaluated by using an appropriate model of animal species relatively close to human, such as *Macaca fascicularis.*

6. Analysis of sugar chains in glycoprotein composition

The sugar chain structure in the glycoprotein expressed in various cells can be analyzed in accordance with the general analysis of the sugar chain structure of a glycoprotein. For example, the sugar chain which is bound to IgG molecule in the antibody composition comprises a neutral sugar such as galactose, mannose or fucose, an amino sugar

such as *N*-acetylglucosamine and an acidic sugar such as sialic acid, and can be analyzed by a method, such as a sugar chain structure analysis, using sugar composition analysis, two dimensional sugar chain mapping or the like.

(1) Analysis of neutral sugar and amino sugar compositions

The sugar chain composition in the glycoprotein composition can be analyzed by carrying out acid hydrolysis of sugar chains with trifluoroacetic acid or the like to release a neutral sugar or an amino sugar and measuring the composition ratio.

Examples include a method using a sugar composition analyzer (BioLC) manufactured by Dionex. The BioLC is an apparatus which analyzes a sugar composition by HPAEC-PAD (high performance anion-exchange chromatography-pulsed amperometric detection) [*J. Liq. Chromatogr.,* 6, 1577 (1983)].

The composition ratio can also be analyzed by a fluorescence labeling method using 2-aminopyridine. Specifically, the composition ratio can be calculated in accordance with a known method [*Agric. Biol. Chem.,* 55(1), 283-284 (1991)], by labeling an acid-hydrolyzed sample with a fluorescence with 2-aminopyridylation and then analyzing the composition by HPLC.

(2) Analysis of sugar chain structure

The sugar chain structure of the glycoprotein composition can be analyzed by the two dimensional sugar chain mapping method [*Anal. Biochem.,* 171, 73 (1988), *Biochemical Experimentation Methods 23 - Methods for Studying Glycoprotein Sugar Chains* (Japan Scientific Societies Press) edited by Reiko Takahashi (1989)]. The two dimensional sugar chain mapping method is a method for deducing a sugar chain structure by, e.g., plotting the retention time or elution position of a sugar chain by reverse phase chromatography as the X axis and the retention time or elution position of the sugar chain by normal phase chromatography as the Y axis, respectively, and comparing them with those of known sugar chains.

Specifically, sugar chains are released from a glycoprotein by hydrazinolysis, and the released sugar chain is subjected to fluorescence labeling with 2-aminopyridine (hereinafter referred to as "PA") [*J. Biochem.,* 95, 197 (1984)], and then the sugar chains are separated from an excess PA-treating reagent and the like by gel filtration, and subjected to reverse phase chromatography. Thereafter, each peak of the separated sugar chains are subjected to normal phase chromatography. The sugar chain structure can be deduced by plotting the results on a two dimensional sugar chain map and comparing them with the spots of a sugar chain standard (manufactured by Takara Shuzo) or a literature [*Anal. Biochem.,* 171, 73 (1988)].

The structure deduced by the two dimensional sugar chain mapping method can be confirmed by further carrying out mass spectrometry such as MALDI-TOF-MS of each sugar chain.

7. Application of the glycoprotein composition of the present invention

Since the glycoprotein composition of the present invention has a sugar chain structure to which fucose is not bound, for example, effects, such as improvement of affinity with a receptor for the glycoprotein composition, improvement of serum half-life of the glycoprotein composition, improvement of tissue distribution after administration of the glycoprotein composition into blood and improvement of its interaction with a protein necessary for pharmacological activity, can be expected. Particularly, the antibody composition of the present invention has a high effector function, namely antibody-dependent cellular cytotoxicity.

The above glycoprotein composition having high physiological activity is useful for preventing and treating various diseases including cancers, inflammatory diseases, immune diseases such as autoimmune diseases, allergies and the like, cardiovascular diseases and various diseases which are caused by viral and bacterial infections.

In the case of cancers, namely malignant tumors, cancer cells grow. General anti-tumor agents inhibit the growth of cancer cells. In contrast, an antibody having high antibody-dependent cell-mediated cytotoxic activity can treat cancers by injuring cancer cells through its cell killing effect, and therefore, it is more effective as a therapeutic agent than the general anti-tumor agents. At present, in the therapeutic agent for cancers, an anti-tumor effect of an antibody medicament alone is not sufficient, so that combination therapy with chemotherapy has been carried out [*Science,* 280, 1197 (1998)]. If higher anti-tumor effect is found by the antibody composition produced in the present invention alone, the dependency on chemotherapy will be decreased and side effects will be reduced.

In immune diseases such as inflammatory diseases, autoimmune diseases and allergies, *in vivo* reactions of the diseases are induced by the release of a mediator molecule by immunocytes. For example, the allergy reaction can be suppressed by eliminating immunocytes using an antibody having high antibody-dependent cell-mediated cytotoxic activity.

The cardiovascular diseases include arteriosclerosis and the like. The arteriosclerosis is treated using balloon

catheter at present, but cardiovascular diseases can be prevented and treated by inhibiting growth of arterial cells in restricture after balloon catheter treatment using an antibody having high antibody-dependent cell-mediated cytotoxic activity.

Various diseases including viral and bacterial infections can be prevented and treated by inhibiting proliferation of cells infected with a virus or bacterium using an antibody having high antibody-dependent cell-mediated cytotoxic activity.

An antibody which recognizes a tumor-related antigen, an antibody which recognizes an allergy- or inflammation-related antigen, an antibody which recognizes cardiovascular disease-related antigen, an antibody which recognizes autoimmune disease-related antigen and an antibody which recognizes a viral or bacterial infection-related antigen are exemplified below.

The antibody which recognizes a tumor-related antigen includes anti-GD2 antibody [*Anticancer Res.,* 13, 331 (1993)], anti-GD3 antibody [*Cancer Immunol. Immunother.,* 36, 260 (1993)], anti-GM2 antibody [*Cancer Res.,* 54, 1511 (1994)], anti-HER2 antibody [*Proc. Natl. Acad Sci. USA,* 89, 4285 (1992)], anti-CD52 antibody [*Nature,* 332, 323 (1998)], anti-MAGE antibody [*British J. Cancer,* 83, 493 (2000)], anti-HM1.24 antibody [*Molecular Immunol.,* 36, 387 (1999)], anti-parathyroid hormone-related protein (PTHrP) antibody *[Cancer,* 88, 2909 (2000)], anti-FGF8 antibody [*Proc. Natl. Acad. Sci. USA,* 86, 9911 (1989)], anti-basic fibroblast growth factor antibody, anti-FGF8 receptor antibody [*J. Biol. Chem.,* 265, 16455 (1990)], anti-basic fibroblast growth factor receptor antibody, anti-insulin-like growth factor antibody [*J. Neurosci. Res.,* 40, 647 (1995)], anti-insulin-like growth factor receptor antibody [*J. Neurosci. Res.,* 40, 647 (1995)], anti-PMSA antibody [*J. Urology,* 160, 2396 (1998)], anti-vascular endothelial cell growth factor antibody [*Cancer Res.,* 57, 4593 (1997)], anti-vascular endothelial cell growth factor receptor antibody [*Oncogene,* 19, 2138 (2000)], anti-CA125 antibody, anti-17-1A antibody, anti-integrin α5β3 antibody, anti-CD33 antibody, anti-CD22 antibody, anti-HLA antibody, anti-HLA-DR antibody, anti-CD20 antibody, anti-CD 19 antibody, anti-EGF receptor antibody [*Immunology Today,* 21, 403 (2000)], anti-CD10 antibody [*American Journal of Clinical Pathology,* 113, 374 (2000)] and the like.

The antibody which recognizes an allergy- or inflammation-related antigen includes anti-interleukin 6 antibody [*Immunol. Rev.,* 127, 5 (1992)], anti-interleukin 6 receptor antibody [*Molecular Immunol.,* 31, 371 (1994)], anti-interleukin 5 antibody [*Immunol. Rev.,* 127, 5 (1992)], anti-interleukin 5 receptor antibody and anti-interleukin 4 antibody [*Cytokine,* 3, 562 (1991)], anti-interleukin 4 receptor antibody [*J. Immunol. Meth.,* 217, 41 (1991)], anti-tumor necrosis factor antibody [*Hybridoma,* 13, 183 (1994)], anti-tumor necrosis factor receptor antibody [*Molecular Pharmacol.,* 58, 237 (2000)], anti-CCR4 antibody [*Nature,* 400, 776 (1999)], anti-chemokine antibody [*J. Immuno. Meth.,* 174, 249 (1994)], anti-chemokine receptor antibody [*J. Exp. Med.,* 186, 1373 (1997)], anti-IgE antibody, anti-CD23 antibody, anti-CD11a antibody [*Immunology Today,* 21, 403 (2000)], anti-CRTH2 antibody [*J Immunol.,* 162, 1278 (1999)], anti-CCR8 antibody (WO99/25734 anti-CCR3 antibody (US6207155) and the like.

The antibody which recognizes a cardiovascular disease-related antigen includes anti-GpIIb/IIIa antibody [*J. Immunol.,* 152, 2968 (1994)], anti-platelet-derived growth factor antibody [*Science,* 253, 1129 (1991)], anti-platelet-derived growth factor receptor antibody [*J. BioL Chem.,* 272, 17400 (1997)], anti-blood coagulation factor antibody [*Circulation,* 101*,* 1158 (2000)] and the like.

The antibody which recognizes an antigen relating to autoimmune diseases (for example, psoriasis, rheumarthritis, Crohn's diseases, colitis ulcerosa, systemic erythematodes, disseminated sclerosis) includes an anti-auto-DNA antibody [*Immunol. Letters,* 72, 61 (2000)], anti-CD11a antibody, anti-ICAM3 antibody, anti-CD80 antibody, anti-CD2 antibody, anti-CD3 antibody, anti-CD4 antibody, anti-integrin α4β7 antibody, anti-CD40L antibody, anti-IL-2 receptor antibody [*Immunology Today,* 21, 403 (2000)], and the like.

The antibody which recognizes a viral or bacterial infection-related antigen includes anti-gp120 antibody [*Structure,* 8, 385 (2000)], anti-CD4 antibody [*J. Rheumatology,* 25, 2065 (1998)], anti-CCR4 antibody, anti-Vero toxin antibody [*J. Clin. Microbiol.,* 37, 396 (1999)], , and the like.

These antibodies can be obtained from public organizations such as ATCC (The American Type Culture Collection), RIKEN Gene Bank at The Institute of Physical and Chemical Research, and National Institute of Bioscience and Human Technology, Agency of Industrial Science and Technology, or private reagent sales companies such as Dainippon Pharmaceutical, R & D SYSTEMS, PharMingen, Cosmo Bio and Funakoshi.

The medicament comprising the glycoprotein composition obtained in the present invention can be administered as a therapeutic agent alone, but generally, it is preferred to provide it as a pharmaceutical formulation prepared by an arbitrary method well known in the technical field of pharmaceuticals, by mixing it with one or more pharmaceutically acceptable carriers.

It is desirable to select a route of administration which is most effective for treatment. Examples include oral administration and parenteral administration, such as buccal, tracheal, rectal, subcutaneous, intramuscular or intravenous. In the case of an antibody preparation, intravenous administration is preferred.

The dosage form includes sprays, capsules, tablets, granules, syrups, emulsions, suppositories, injections, ointments, tapes and the like.

The pharmaceutical preparation suitable for oral administration includes emulsions, syrups, capsules, tablets,

powders, granules and the like.

Liquid preparations, such as emulsions and syrups, can be produced using, as additives, water; sugars such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like.

Capsules, tablets, powders, granules and the like can be produced using, as additive, excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid ester; plasticizers such as glycerine; and the like.

The pharmaceutical preparation suitable for parenteral administration includes injections, suppositories, sprays and the like.

Injections can be prepared using a carrier, such as a salt solution, a glucose solution, a mixture of both thereof or the like. Also, powdered injections can be prepared by freeze-drying the humanized antibody in the usual way and adding sodium chloride thereto.

Suppositories can be prepared using a carrier such as cacao butter, hydrogenated fat, carboxylic acid or the like.

Sprays can be prepared using the compound as such or using the antibody composition together with a carrier which does not stimulate the buccal or airway mucous membrane of the patient and can facilitate absorption of the compound by dispersing it as fine particles.

The carrier includes lactose, glycerol and the like. Depending on the properties of the compound and the carrier used, it is possible to produce pharmaceutical preparations such as aerosols, dry powders and the like. In addition, the components exemplified as additives for oral preparations can also be added to the parenteral preparations.

Although the clinical dose or the frequency of administration varies depending on the objective therapeutic effect, administration method, treating period, age, body weight and the like, it is usually 10 μg/kg to 20 mg/kg per day and per adult.

Also, as the method for examining antitumor effect of the antibody composition against various tumor cells, *in vitro* tests include CDC activity measuring method, ADCC activity measuring method and the like, and *in vivo* tests include antitumor experiments using a tumor system in an experimental animal such as a mouse, and the like.

CDC activity and ADCC activity measurements and antitumor experiments can be carried out in accordance with the methods described in *Cancer Immunology Immunotherapy,* 36, 373 (1993); *Cancer Research,* 54, 1511 (1994) and the like.

The present invention will be described below in detail based on Examples; however, Examples are only simple illustrations, and the scope of the present invention is not limited thereto.

BRIEF DESCRIPTION OF THE INVENTION

Fig. 1 is a graph showing ADCC activity of two purified anti-CCR4 human chimeric antibodies. The ordinate shows the cytotoxic activity, and the abscissa shows the antibody concentration. □ corresponds to the activity of an SM3G1/CCR4 antibody produced by a transformant SM3G1/CCR4 obtained from a GDP-mannose 4,6-dehydratase gene knockout clone CHO SM, and ■ corresponds to the activity of CHO/CCR4 antibody produced by transformant CHO/CCR4 obtained from CHO/DG44 cell of the parent cell line.

Fig. 2 is a graph showing changes in (A) density of viable cells, (B) survival ratio of cells and (C) antibody concentration, when a serum-free fed-batch culturing was carried out using transformants SM3G1/CCR4-AF and CHO/CCR4-AF naturalized to a serum-free medium. The abscissa in each graph shows cultured days after the commencement of the culturing. □ corresponds to a result of the transformant SM3G1/CCR4-AF, and ■ corresponds to a result of the transformant CHO/CCR4-AF.

Fig. 3 is a graph showing the binding activity of 11 anti-CCR4 chimeric antibodies having a different ratio of sugar chains to which fucose is not bound, to a soluble human FcγRIIIa. The ordinate shows the binding activity, and the abscissa shows the ratio of sugar chains to which fucose is not bound. o corresponds to the binding activity of each anti-CCR4 chimeric antibody, and the solid line in the drawing is a calibration prepared based on the binding activity of each anti-CCR4 chimeric antibody.

Fig. 4 is a graph showing the binding activity of anti-CCR4 chimeric antibodies contained in the samples collected from the serum-free fed-batch culturing of transformants SM3G1/CCR4-AF and CHO/CCR4-AF, to a soluble human FcγRIIIa. The ordinate shows the binding activity, and the abscissa shows the sample-collected days after commencement of the culturing. □ corresponds to the activity of samples derived from the transformant SM3G1/CCR4-AF, and ■ corresponds to the activity of samples derived from the transformant CHO/CCR4-AF.

Fig. 5 is a graph showing the construction of plasmid pBS-ATIII.

Fig. 6 is a graph showing the construction of plasmid pKAN-ATIII.
Fig. 7 is a graph showing the construction of plasmids pBS-ATIIIN135Q and pKAN-ATIIIN135Q.

Example 1

Obtaining of clone in which a gene encoding an enzyme capable of catalyzing a dehydration reaction to convert GDP-mannose into GDP-4-keto,6-deoxy-GDP-mannose is not expressed:

1. Obtaining of lectin-resistant CHO/DG44 clone

The CHO/DG44 cell (*Proc. Natl. Acad. Sci. USA,* 77, 4216 (1980)) was cultured in an IMDM-FBS(10)-HT(1) medium [IMDM medium (manufactured by Invitrogen) containing 10% fetal bovine serum (FBS) (manufactured by Invitrogen) and 1 × concentration of HT supplement (manufactured by Invitrogen)] using a 75 $cm^2$ flask for adhesion culture (manufactured by Greiner), and allowed to proliferate until reaching just before the confluent stage. After washing the cells with 5 ml of Dulbecco PBS (hereinafter referred to as PBS) (manufactured by Invitrogen), 1.5 ml of 0.05% trypsin (manufactured by Invitrogen) diluted with PBS was added thereto and the cells were allowed to stand at 37°C for 5 minutes to peal off them from the culture container bottom. The pealed cells were recovered by a centrifugation operation generally carried out in cell culturing and suspended to give a density of $1\times10^5$ cells/ml by adding the IMDM-FBS(10)-HT (1) medium, and then 0.1 μg/ml of an alkylation agent, MNNG (manufactured by SIGMA), was added or not added thereto. After allowing the cells to stand at 37°C for 3 days in a $CO_2$ incubator (manufactured by TABAI), the culture supernatant was discarded, and the cells were washed, peeled off, recovered and suspended in the IMDM-FBS(10)-HT (1) medium, in the similar manner as described above, and then seeded into a 96-well plate for adherent culture (manufactured by Asahi Techno Glass) at a density of 1,000 cells/well. To each well, 1 mg/ml of *Lens culinaris* agglutinin (hereinafter referred to as LCA, manufactured by Vector) was added as a final concentration in the medium. After culturing at 37°C for 2 weeks in a $CO_2$ incubator, the thus formed colonies were isolated as lectin-resistant CHO/DG44 clones.

2. Determination of GDP-mannose 4,6-dehydratase mRNA of the obtained lectin-resistant CHO/DG44 clones

The expressed amount of GDP-mannose 4,6-dehydratase as an enzyme capable of catalyzing a dehydration reaction to convert GDP-mannose into GDP-4-keto,6-deoxy-GDP-mannose in each of the lectin-resistant CHO/DG44 clones obtained in the item 1 of this Example was analyzed in the following manner using RT-PCR method.

(1) Preparation of RNA from lectin-resistant CHO/DG44 clone and preparation of single-stranded cDNA

RNA samples were prepared respectively from $1\times10^7$ cells of the parent cell line CHO/DG44 cell and each of the lectin-resistant CHO/DG44 clones obtained in item 1 of this Example, using RNeasy Protect Mini Kit (manufactured by QIAGEN) in accordance with the instructions attached thereto. Subsequently, single-stranded cDNA was synthesized from 5 μg of each RNA in 20 μl of a reaction mixture using SUPER SCRIPT First-Strand synthesis system for RT-PCR (manufactured by Invitrogen) in accordance with the instructions attached thereto.

(2) Expression amount analysis of β-actin gene using RT-PCR

In order to confirm quality of each of the respective clone-derived single-stranded cDNA samples prepared in the above item (1), amplification of β-actin cDNA by PCR was examined in the following manner.
After 20 μl of a reaction mixture [1 × EX Taq Buffer (manufactured by Takara Shuzo), 0.2 mM of dNTPs, 0.5 unit of EX Taq polymerase (manufactured by Takara Shuzo) and 0.5 μM of the synthetic oligo DNA primers of SEQ ID NOs:11 and 12] comprising, as the template, 0.5 μl of each of the respective clone-derived single-stranded cDNA samples prepared in the above (1) was prepared, the reaction mixture was heated at 94°C for 5 minutes and then 14 cycles of the reaction, one cycle consisting of reaction at 94°C for one minute and reaction at 68°C for 2 minutes, were carried out using DNA Thermal Cycler 480 (manufactured by Perkin Elmer). After 10 μl of the resulting PCR reaction mixture was subjected to agarose electrophoresis, the DNA fragments were stained using Cyber Green (manufactured by BMA), and then the amount of the expected DNA fragment of approximately 800 bp was measured using Fluor Imager SI (manufactured by Molecular Dynamics). As a result, it was able to detect the expression of β-actin at a similar level by using every clone-derived single-stranded cDNA.

(3) Analysis of expression amount of GDP-mannose 4,6-dehydratase gene using RT-PCR method

Next, the expression amount of a gene encoding GDP-mannose 4,6-dehydratase in the respective lectin-resistant CHO/DG44 clones obtained in the above item (1) was analyzed. In order to amplify cDNA of the gene encoding GDP-mannose 4,6-dehydratase by PCR, a synthetic DNA primer of 26 mer having the nucleotide sequence represented by SEQ ID NO:13 and a synthetic DNA primer of 28 mer having the nucleotide sequence represented by SEQ ID NO: 14 were prepared based on the cDNA sequence of CHO cell-derived GDP-mannose 4,6-dehydratase represented by SEQ ID NO:1. Subsequently, 20 μl of a reaction solution [1 × EX Taq Buffer (manufactured by Takara Shuzo), 0.2 mM of dNTPs, 0.5 unit of EX Taq polymerase (manufactured by Takara Shuzo) and 0.5 μM of the synthetic DNA primers of SEQ ID NOs:13 and 14] comprising, as the template, 0.5 μl of each of the respective clone-derived single-stranded cDNA samples prepared in the above item (1) was prepared, and after heating at 94°C for 5 minutes, the reaction was carried out by 30 cycles, one cycle consisting of reaction at 94°C for 1 minute and reaction at 68°C for 2 minutes using DNA Thermal Cycler 480 (manufactured by Perkin Elmer). After 10 μl of the resulting PCR reaction solution was subjected to agarose electrophoresis, the DNA fragments were stained using Cyber Green (manufactured by BMA), and then amount of the expected DNA fragment of about 430 bp was measured using Fluor Imager SI (manufactured by Molecular Dynamics). As a result, it was confirmed that a clone in which expression of GDP-mannose 4,6-dehydratase gene is not observed is present in the obtained lectin-resistant CHO/DG44 clone. This clone in which expression of GDP-mannose 4,6-dehydratase gene was not observed was named clone CHO SM.

In this connection, when resistance of the thus obtained clone CHO SM to various species of lectin was examined, the clone CHO SM showed a resistance also to a lectin which recognizes the same sugar chain structure as the sugar chain structure which is recognized by LCA, namely other lectin which recognizes a sugar chain structure in which 1-position of fucose is bound to 6-position ofN-acetylglucosamine in the reducing end through α-bond in N-glycoside-linked sugar chains. Specifically, it showed resistance to a medium comprising *Pisum sativum* agglutinin (hereinafter referred to as PSA, manufactured by Vector) at a final concentration of 1 mg/ml or to a medium comprising *Aleuria aurantia* lectin (hereinafter referred to as AAL, manufactured by Vector) at a final concentration of 1 mg/ml.

Example 2

Genomic analysis of clone in which a gene of an enzyme capable of catalyzing a dehydration reaction to convert GDP-mannose into GDP-4-keto,6-deoxy-GDP-mannose is not expressed:

Using a T75 flask for adhesion culture (manufactured by Greiner), each of CHO/DG44 cell and the clone CHO SM obtained in Example 1 was cultured in IMDM-FBS(10)-HT(1) medium until it reached just before the confluent stage, and then genomic DNA was prepared in accordance with a conventionally known method [*Nucleic Acid Research,* 3, 2303 (1976)], and the thus obtained genomic DNA was dissolved overnight in 300 μl of TE-RNase buffer solution (pH 8.0) [10 mmol/l Tris-HCl, 1 mmol/l EDTA, 200 μg/l RNase A].

After 12 μg of the genomic DNA prepared in the above was digested with three different restriction enzymes *Eco*RI (manufactured by Takara Shuzo), *Hind*III (manufactured by Takara Shuzo) and *Bgl*II (manufactured by Takara Shuzo), respectively, the DNA fragments were recovered using the ethanol precipitation method. Then, the mixture was dissolved in 20 μl of TE buffer (pH 8.0) [10 mmol/l Tris-HCl, 1 mmol/l EDTA] and subjected to 0.8% (w/v) agarose gel electrophoresis. After the electrophoresis, the genomic DNA fragments were transferred onto a nylon membrane in accordance with a conventionally known method [*Proc. Natl. Acad Sci. USA,* 76, 3683 (1979)]. After completion of the transfer, the nylon membrane was heated at 80°C for 2 hours.

Next, in order to confirm the quality of the genomic DNA transferred onto the nylon membrane, Southern hybridization was carried out using, as the probe, α1,6-fucosyltransferase (FUT8) gene which is considered to be present in the genome of every cell line. The probe for detecting the FUT8 gene was prepared in the following manner. Firstly, 10 μg of a plasmid mfFUT8-pCR2.1 comprising mouse FUT8 cDNA, described in Example 11 of WO02/31140, was dissolved in 50 μl of M buffer (manufactured by Takara Shuzo), digested overnight with a restriction enzyme *Hind*III (manufactured by Takara Shuzo), and then the reaction solution was replaced by H buffer (manufactured by Takara Shuzo) and digestion reaction with a restriction enzyme *Eco*RI (manufactured by Takara Shuzo) was further carried out overnight, After completion of the reaction, the reaction solution was subjected to 2% agarose electrophoresis, and an *Eco*RI-*Hind*III fragment of 156 bp containing exon 2 of the FUT8 gene was purified. Next, 25 ng of the thus obtained DNA fragment was radiation-labeled using 1.75 MBq of [α-$^{32}$P]dCTP and Megaprime DNA labeling system, dCTP (manufactured by Amersham Bioscience).

Hybridization was carried out as follows. Firstly, the above-described nylon membrane was sealed in a roller bottle, and pre-hybridization was carried out at 65°C for 3 hours by adding 15 ml of a hybridization solution [4 × SSPE, 5 × Denhaldt's solution, 0.5% (w/v) SDS, 0.1 mg/ml sermon sperm DNA]. Next, the $^{32}$p-labeled probe DNA was denatured with heat, charged into the bottle and heated overnight at 65°C.

After the hybridization, the nylon membrane was soaked in 50 ml of 2 × SSC-0.1% (w/v) SDS and heated at 65°C for 15 minutes. After repeating the above washing step twice, the nylon membrane was soaked in 50 ml of 0.2 × SSC-0.1% (w/v) SDS and heated at 65°C for 15 minutes. After washing, the nylon membrane was exposed to an X-ray film at -80°C for two nights for development. After the development, the nylon membrane was boiled in a stripping solution

[1% SDS, 0.1 × SSC] to release the probe and again subjected to hybridization with a different probe.

By this method, a fragment specific to exon 2 of the FUT8 gene was detected in the genomic DNA of each of the clone CHO/DG44 and clone CHO SM. Based on the above results, degradation and the like were not found in the genomic DNA samples transferred on the nylon membrane, derived from the clone CHO SM and clone CHO/DG44, and they showed the identical quality.

On the other hand, a probe specific to GMD gene exon 5 was prepared as follows. Firstly, primers (SEQ ID NOs:15 and 16) which specifically bind to the exon 5 were designed based on a conventionally known human GMD genomic DNA sequence (NCBI accession No. NT_034880). The region corresponds to a region from the base number 346 to the base number 538 of the human GMD cDNA sequence represented by SEQ ID NO:2. Next, polymerase chain reaction (PCR) was carried out by preparing 100 μl of a reaction solution [ExTaq buffer (manufactured by Takara Shuzo), 0.2 mmol/l of dNTPs and 2.5 μmol/l of the above-described gene-specific primers (SEQ ID NOs:15 and 16)] containing 10 ng of the plasmid pAGE249GMD described in Example 15 of WO02/31140. After heating at 94°C for 5 minutes, the PCR was carried out by 30 cycles, one cycle consisting of reaction at 94°C for 1 minute, reaction at 58°C for 2 minutes and reaction at 72°C for 3 minutes. After the PCR, the reaction solution was subjected to 2% agarose electrophoresis, and a DNA fragment of about 200 bp was purified. Then, 25 ng of the thus obtained DNA fragment was radiation-labeled using 1.75 MBq of [$\alpha$-$^{32}$P]dCTP and Megaprime DNA labeling system, dCTP (manufactured by Amersham Bioscience). As a result of hybridization on the above-described nylon membrane using the probe, a fragment specific to exon 5 of the GMD gene was found in the genomic DNA derived from the CHO/DG44 cell, while the fragment specific to exon 5 of the GMD gene was not detected in the genomic DNA derived from the clone CHO SM. Based on the above results, it was shown that the clone CHO SM is a GMD knockout cell in which at least an exon 5-containing region among the GMD-encoding genomic region was deleted.

Example 3

Production of antibody using a clone in which a genomic gene encoding an enzyme capable of catalyzing a dehydration reaction to convert GDP-mannose into GDP-4-keto,6-deoxy-GDP-mannose is knocked out:

1. Preparation of anti-CCR4 human chimeric antibody producing cells

Cells stably producing an anti-CCR4 human chimeric antibody were prepared as follows, by introducing the anti-CCR4 human chimeric antibody expression plasmid pKANTEX2160 described in WO01/64754 into the GDP-mannose 4,6-dehydratase gene-knocked out clone CHO SM prepared in Example 1 and its parent cell line CHO/DG44.

After 4 μg of the anti-CCR4 human chimeric antibody expression vector pKANTEX2160 was introduced into $1.6 \times 10^6$ of cells by electroporation *[Cytotechnology,* 3, 133 (1990)], the cells were suspended in 10 ml of IMDM-dFBS (10)-HT(1) [IMDM medium comprising 10% dFBS (manufactured by Invitrogen) and 1 × concentration HT supplement] and dispensed at 100 μl/well into a 96 well culture plate (manufactured by Iwaki Glass). After culturing at 37°C for 24 hours in a 5% $CO_2$ incubator, the medium was changed to IMDM-dFBS(10) (IMDM medium comprising 10% dialyzed FBS), followed by culturing for 1 to 2 weeks. Since colonies of transformants showing HT-independent growth were formed, culture supernatants were recovered from the wells in which the growth was observed, and the expressed amount of .the anti-CCR4 human chimeric antibody in each well was measured by the ELISA described in item 2 of this Example.

Regarding the transformants in the wells in which production of the anti-CCR4 human chimeric antibody was found in the culture supernatant, in order to increase the antibody production making use of a DHFR gene amplification system, they were suspended in the IMDM-dFBS(10) medium comprising 50 nM of MTX, to give a density of 1 to $2 \times 10^5$ cells/ml, and dispensed at 0.5 ml into a 24 well plate (manufactured by Iwaki Glass). By culturing at 37°C for 1 to 2 weeks in a 5% $CO_2$ incubator, transformants showing resistance to 50 nM MTX were induced. Regarding the transformants in the wells in which their growth was observed, the MTX concentration was increased to 200 nM and then to 500 nM, and a transformant which can grow in the IMDM-dFBS(10) medium comprising 500 nM of MTX and produces the anti-CCR4 human chimeric antibody in a large amount was finally obtained. The transformant obtained from the CHO/DG44 cells of the parent cell line was named transformant CHO/CCR4, and the transformant obtained from the GDP-mannose 4,6-dehydratase gene-knocked out clone CHO SM was named transformant SM3G1/CCR4. Also, the thus obtained transformant SM3G1/CCR4 has been deposited, under the name of SM3G1/CCR4, with International Patent Organism Depositary, National Institute of Advanced Industrial Science and Technology (Central 6, 1-1-1 Higashi, Tsukuba, Ibaraki, Japan) on September 9, 2003 with accession number FERM BP-08473.

2. Measurement of human IgG antibody concentration in culture supernatant (ELISA)

A goat anti-human IgG (H & L) antibody (manufactured by American Qualex) was diluted with PBS to give a

concentration of 1 μg/ml, dispensed at 50 μl/well into a 96 well ELISA plate (manufactured by Greiner) and then allowed to stand at 4°C overnight for adsorption. After washing with PBS, PBS containing BSA at a concentration of 1% (hereinafter referred to as 1% BSA-PBS) (manufactured by Wako Pure Chemical Industries) was added at 100 μl/well and allowed to react at room temperature for 1 hour to thereby block the remaining active groups. The 1% BSA-PBS was discarded, and culture supernatant of a transformant or variously diluted solution of antibody purified from the culture supernatant was added at 50 μl/well and allowed to react at room temperature for 1 hour. After the reaction, each well was washed with PBS containing Tween 20 at a concentration of 0.05% (hereinafter referred to as Tween-PBS) (manufactured by Wako Pure Chemical Industries), and then a peroxidase-labeled goat anti-human IgG (H & L) antibody solution (manufactured by American Qualex) was added as a secondary antibody solution at 50 μl/well and allowed to react at room temperature for 1 hour. After the reaction and subsequent washing with Tween-PBS, an ABTS substrate solution [a solution prepared by dissolving 0.55 g of 2,2'-azino-bis(3-ethylbenzothiazoline-6-sulfonic acid)ammonium (manufactured by Wako Pure Chemical Industries) in 1 liter of 0.1 M citrate buffer (pH 4.2), and adding hydrogen peroxide (manufactured by Wako Pure Chemical Industries) to give a concentration of 1 μl/ml just before use] was added at 50 μl/well to develop color, and the absorbance at 415 nm (hereinafter referred to as OD415) was measured.

3. Purification of anti-CCR4 human chimeric antibodies

Using the transformants SM3G1/CCR4 and CHO/CCR4 obtained in the item 1 of this Example, the anti-CCR4 human chimeric antibodies respectively produced were purified as follows.

Each of the transformants was cultured at 37°C in a 5% $CO_2$ incubator using the IMDM-dFBS(10) medium contained in a 182 $cm^2$ flask (manufactured by Greiner). When the cell density reached confluent several days thereafter, the culture supernatant was discarded, the cells were washed with 25 ml of PBS, and then 35 ml of EXCELL 301 medium (manufactured by JRH) was injected. After culturing at 37°C for 7 days in a 5% $CO_2$ incubator, the cell suspension was recovered and centrifuged at 3,000 rpm and at 4°C for 5 minutes to recover the supernatant which was then sterilized by filtration through a 0.22 μm Millex GV filter (manufactured by Millipore). Each of the anti-CCR4 human chimeric antibodies was purified from the corresponding culture supernatant obtained by the above method, using a Mab Select (manufactured by Amersham Biosciences) column and in accordance with the instructions attached thereto. Regarding the purified anti-CCR4 human chimeric antibodies, the antibody obtained from the transformant CHO/CCR4 was named CHO/DG44 antibody, and the antibody obtained from the transformant SM3G1/CCR4 was named SM3G1/CCR4 antibody.

4. Measurement of binding activity of anti-CCR4-human chimeric antibody to human CCR4 antigen (ELISA)

Binding activity of the CHO/CCR4 antibody and SM3G1/CCR4 antibody purified in the item 3 in this Example to a human CCR4 antigen was measured by ELISA as below.

Compound 1 (SEQ ID NO: 17) was selected as a human CCR4 extracellular region peptide capable of reacting with the anti-CCR4 chimeric antibody. In order to use it in the activity measurement by ELISA, a conjugate with BSA (bovine serum albumin) (manufactured by Nacalai Tesque) was prepared by the following method and used as the antigen. That is, 100 ml of a DMSO solution comprising 25 mg/ml SMCC [4-(N-maleimidomethyl)-cyclohexane-1-carboxylic acid *N*-hydroxysuccinimide ester] (manufactured by Sigma) was added dropwise to 900 ml of a 10 mg BSA-containing PBS solution under stirring by using a vortex, followed by gently stirring for 30 minutes. To a gel filtration column such as NAP-10 column equilibrated with 25 ml of PBS, 1 ml of the reaction solution was applied and then eluted with 1.5 ml of PBS and the resulting eluate was used as a BSA-SMCC solution (BSA concentration was calculated by the absorbance at 280 nm). Next, 250 ml of PBS was added to 0.5 mg of Compound 1 and then completely dissolved by adding 250 ml of DMF, and the BSA-SMCC solution was added thereto under vortex, followed by gently stirring for 3 hours. The reaction solution was dialyzed against PBS at 4°C overnight, sodium azide was added thereto to give a final concentration of 0.05%, and the mixture was filtered through a 0.22 mm filter to be used as a BSA-Compound 1 solution.

The prepared conjugate as mentioned above was dispensed at 0.05 μg/ml and 50 μl/well into a 96 well EIA plate (manufactured by Greiner) and incubated for adhesion at 4°C overnight. After washing each well with PBS, 1% BSA-PBS was added thereto in 100 μl/well and allowed to react at room temperature for 1 hour to block the remaining active groups. After washing each well with Tween-PBS, a culture supernatant of a transformant was added at 50 μl/well and allowed to react at room temperature for 1 hour. After the reaction, each well was washed with Tween-PBS, and then a peroxidase-labeled goat anti-human IgG(γ) antibody solution (manufactured by American Qualex) diluted 6000 times with 1% BSA-PBS as the secondary antibody solution was added at 50 μl/well and allowed to react at room temperature for 1 hour. After the reaction and subsequent washing with Tween-PBS, the ABTS substrate solution was added at 50 μl/well for color development, and OD415 was measured. It was confirmed that the CHO/CCR4 antibody and SM3G1/CCR4 antibody obtained in the item 3 in this Example have similar binding activity to the human CCR4

extracellular region peptide.

5. Measurement of ADCC activity of anti-CCR4 human chimeric antibody

The ADCC activity of two kinds of the anti-CCR4 human chimeric antibody purified products obtained in the item 3 of this Example was measured by targeting at CCR/EL4 cell which is a mouse thymoma cell EL4 cell line in which human CCR4 is highly expressed, described in WO01/64754.

(1) Preparation of a target cell suspension

CCR/EL4 cells were washed with RPMI 1640-FCS(5) medium (RPMI 1640 medium (manufactured by GIBCO BRL) containing 5% FCS) by centrifugation and suspension and then adjusted to give a density of $2 \times 10^5$ cells/ml by using RPMI 1640-FCS(5) medium and used as the target cell suspension.

(2) Preparation of a human effector cell suspension

Peripheral blood (50 ml) was collected from a healthy person and gently mixed with 0.5 ml of heparin sodium (manufactured by Shimizu Pharmaceutical Co., Ltd.). The monocyte layer was separated from this mixture by centrifugation (800 g, 20 minutes) using Lymphoprep (manufactured by AXIS SHIELD) according to the attached instructions. After washing three times with RPMI1640-FCS(5) medium through centrifugation, the cells were suspended in the same medium at a density of $5 \times 10^6$ cells/ml to give an effector cell suspension.

(3) Measurement of ADCC activity

The target cell suspension prepared in the above (1) (50 μl) was put into each well of a 96-well U-shaped bottom plate (manufactured by Falcon) ($1 \times 10^4$ cells/well). Then, 50 μl of the effector cell suspension prepared in (2) was added to each well ($2.5 \times 10^5$ cells/well; the ratio of effector cells to target cells becomes 25:1). Subsequently, each of the anti-CCR4 human chimeric antibodies was added to give a final concentration of 0.1 to 1000 ng/ml and to make a total volume of 150 μl, followed by reaction at 37°C for 4 hours. After the reaction, the plate was subjected to centrifugation, and the lactate dehydrogenase (LDH) activity of the supernatant was measured by obtaining absorbance data using CytoTox96 Non-Radioactive Cytotoxicity Assay (manufactured by Promega) according to the attached instructions. The absorbance data for target cell spontaneous release were obtained by the same procedure as above using only the medium instead of the effector cell suspension and the antibody solution, and those for effector cell spontaneous release were obtained by the same procedure using only the medium instead of the target cell suspension and the antibody solution. The absorbance data for target cell total release were obtained by the same procedure as above using the medium instead of the antibody solution and the effector cell suspension, adding 15 μl of 9% Triton X-100 solution 45 minutes before the completion of the reaction, and measuring the LDH activity of the supernatant. The ADCC activity was calculated according to the following equation.

$$\text{Cytotoxic activity (\%)} = \frac{\left(\text{Absorbance of sample}\right) - \left(\text{Absorbance for effector cell spontaneous release}\right) - \left(\text{Absorbance for target cell spontaneous release}\right)}{\left(\text{Absorbance for target cell total release}\right) - \left(\text{Absorbance for target cell spontaneous release}\right)} \times 100$$

Fig. 1 shows the ADCC activity of the CHO/CCR4 antibody and the SM3G1/CCR4 antibody against the CCR4/EL4 cells. The SM3G1/CCR4 antibody produced by the transformant SM3G1/CCR4 obtained from GDP-mannose 4,6-dehydratase gene-knocked out clone CHO SM showed about 100-fold increase of the ADCC activity. Also, the similar results were obtained even if the donors of the effector cells were different.

6. Analysis of monosaccharide composition of anti-CCR4-human chimeric antibody

Analysis of the neutral sugar and amino sugar composition of the two kinds of the anti-CCR4 human chimeric antibody purified products obtained in the item 3 in this Example was carried out as follows.

After the antibody was dried under reduced pressure using a centrifugal concentrator, a 2.0 to 4.0 mol/l trifluoroacetic acid solution was added thereto and acid hydrolysis was carried out at 100°C for 2 to 4 hours to release neutral sugars and amino sugars from the protein. The trifluoroacetic acid solution was removed with a centrifugal concentrator, and the sugars were redissolved in deionized water and subjected to analysis using a carbohydrate analysis system (DX-500 manufactured by Dionex). The analysis was carried out according to the elution program shown in Table 1 using CarboPac PA-1 column and CarboPac PA-1 guard column (manufactured by Dionex), a 10 to 20 mM solution of sodium hydroxide in deionized water as an eluting solution and a 500 mM solution of sodium hydroxide in deionized water as a washing solution.

Table 1 Elution program for neutral sugar and amino sugar composition analysis

| Time (min.) | 0 | 35 | 35.1 | 45 | 45.1 | 58 |
|---|---|---|---|---|---|---|
| Eluting solution (%) | 100 | 100 | 0 | 0 | 100 | 100 |
| Washing solution (%) | 0 | 0 | 100 | 100 | 0 | 0 |

From the obtained peak areas of neutral and amino sugar components, the composition ratio of components (fucose, galactose and mannose) was calculated, regarding the value of N-acetylglucosamine as 4.

Table 2 shows the results. The ratio of sugar chains having a structure in which 1-position of fucose is not bound to 6-position N-acetylglucosamine in the reducing end in the antibody produced by the transformant CHO/CCR4 obtained by the CHO/DG44 cell which was the parent cell line was estimated to be about 15%. On the other hand, the ratio of sugar chains having a structure in which 1-position of fucose is not bound to 6-position N-acetylglucosamine in the reducing end in the antibody produced by the transformant SM3G1/CCR4 obtained by the GDP-mannose 4,6-dehydratease gene-knocked out clone CHO SM was significantly high and the sugar chain structure modified with fucose was lower than the detection limit, so that the ratio was estimated to be close to 100%.

Table 2

| Antibody | Ratio of complex type biantennary sugar chains in which fucose is not bound |
|---|---|
| CHO/CCR4 | 15% |
| SM3G1/CCR4 | 100% |

Example 4

Serum-free fed-batch culturing using GDP-mannose 4,6-dehydratase gene-knocked out transformant SM3G1/CCR4:

1. Naturalization of GDP-mannose 4,6-dehydratase gene-knocked out transformant SM3G1/CCR4 to serum-free medium

Naturalization of the transformants SM3G1/CCR4 and CHO/CCR4 obtained in the item 1 of Example 3 to a serum-free medium was carried out as follows.

Each of the transformants was suspended in IMDM-dFBS(10) medium comprising MTX at a concentration of 500 nM to give a cell density of 2 to $4\times10^5$ cells/ml, inoculated into a 75 $cm^2$ flask for adhesion culture (manufactured by Greiner) and statically cultured at 37°C in a 5% $CO_2$ incubator. The cells which became confluent were peeled off using a 0.05% trypsin (manufactured by Invitrogen) solution and suspended in IMDM-dFBS(10) medium comprising MTX at a concentration of 500 nM and then the supernatant was removed by centrifugation. The thus obtained cells were suspended in EX-CELL 302 medium (manufactured by JRH) containing 500 nM of MTX and 6 mM of L-glutamine (manufactured by Invitrogen) (hereinafter referred to as serum-free medium) to give a density of $5\times10^5$ cells/ml, and 15 ml of the cell suspension was inoculated into a 125 ml conical flask (manufactured by Coming). The atmosphere in the flask was substituted by feeding 5% $CO_2$ of 4-fold or more volume of the culture vessel and then the vessel was sealed to carry out suspension rotation culturing at 35°C and at 90 to 100 rpm. By repeating the sub-culturing at intervals of 3 to 4 days, transformants capable of growing in the serum-free medium were finally obtained. Hereinafter, the naturalized transformant SM3G1/CCR4 with the serum-free medium is referred to as SM3G1/CCR4-AF, and the naturalized transformant CHO/CCR4 with the serum-free medium as CHO/CCR4-AF.

2. Serum-free fed-batch culturing using GDP-mannose 4,6-dehydratase gene-knocked out transformant SM3G1/CCR4-AF naturalized with serum-free medium

Serum-free fed-batch culturing was carried out as follows using the transformants SM3G1/CCR4-AF and CHO/CCR4-AF naturalized with the serum-free medium in the item 1 of this Example.

The serum-free medium described in the above item was modified and further supplemented with a 20% (w/v) glucose solution to give a final concentration of 5,000 mg/l, and the mixture was used as the basal medium of the fed-batch culturing (hereinafter referred to as serum-free fed-batch culture medium). The feed medium used was a medium prepared in higher concentration than usual adding concentration, comprising amino acids (L-alanine 0.177 g/l, L-arginine monohydrochloride 0.593 g/l, L-asparagine monohydrate 0.177 g/l, L-aspartic acid 0.212 g/l, L-cystine dihydrochloride 0.646 g/l, L-glutamic acid 0.530 g/l, L-glutamine 5.84 g/l, glycine 0.212 g/l, L-histidine monohydrochloride dihydrate 0.297 g/l, L-isoleucine 0.742 g/l, L-leucine 0.742 g/l, L-lysine monohydrochloride 1.031 g/l, L-methionine 0.212 g/l, L-phenylalanine 0.466 g/l, L-proline 0.283 g/l, L-serine 0.297 g/l, L-threonine 0.671 g/l, L-tryptophan 0.113 g/l, L-tyrosine disodium dihydrate 0.735 g/l and L-valine 0.664 g/l), vitamins (d-biotin 0.0918 mg/l, calcium D-pantothenate 0.0283 g/l, choline chloride 0.0283 g/l, folic acid 0.0283 g/l, myo-inositol 0.0509 g/l, niacin amide 0.0283 g/l, pyridoxal hydrochloride 0.0283 g/l, riboflavin 0.00283 g/l, thiamine hydrochloride 0.0283 g/l and cyanocobalamin 0.0918 g/l), and insulin 0.314 g/l.

Each of the transformants SM3G1/CCR4-AF and CHO/CCR4-AF was suspended in the serum-free fed-batch culture medium to give a density of $3\times10^5$ cells/ml, and 40 ml of the cell suspension was inoculated into a 250 ml capacity conical flask (manufactured by Coming). The atmosphere in the flask was substituted by feeding 5% $CO_2$ of 4-fold or more volume of the culture vessel and then the vessel was sealed to carry out suspension rotation culturing at 35°C and at 90 to 100 rpm for 13 days. On the 3rd day, 6th day, 9th day and 11th day after commencement of the culturing, 3.3 ml of the feed medium was added for the purpose of supplementing consumed amounts of amino acids and the like, and a 20% (w/v) glucose solution was added to give a final concentration of 5,000 mg/l for the purpose of controlling the glucose concentration. On the day 0, 3rd day, 6th day, 9th day, 11th day and 13th day after commencement of the culturing, about 2 ml of the culture broth was collected, the density of viable cells and survival ratio of cells were measured by trypan blue staining, and the concentration of anti-CCR4 chimeric antibody contained in each culture supernatant was measured by the ELISA described in the item 2 of Example 3.

The results are shown in Fig. 2A to C. The density of viable cells (A), survival ratio of cells (B) and concentration of anti-CCR4 chimeric antibody in culture supernatant (C) of the transformant SM3G1/CCR4-AF were almost the same as those of the transformant CHO/CCR4-AF, and no influence of the knockout of GDP-mannose 4,6-dehydratase gene upon cell growth and antibody production was found.

3. Determination of anti-CCR4 chimeric antibody having a sugar chain in which 1-position of fucose is not bound to 6-position of N-acetylglucosamine in the reducing end through α-bond, based on the binding activity to soluble human FcγRIIIa

The ratio of the sugar chain in which 1-position of fucose is not bound to 6-position of N-acetylglucosamine in the reducing end through α-bond in the anti-CCR4 chimeric antibody contained in the serum-free fed-batch culture samples of the transformants SM3G1/CCR4-AF and CHO/CCR4-AF, collected in the item 2 of this Example, was measured based on the binding activity to soluble human FcγRIIIa (hereinafter referred to as shFcγRIIIa) described in the item 3 of Example 11 of WO03/085119.

(1) Preparation of anti-CCR4 chimeric antibodies having different ratios of sugar chains in which 1-position of fucose is not bound to 6-position of N-acetylglucosamine in the reducing end through α-bond

Standard samples having different ratios of sugar chains in which 1-position of fucose is not bound to 6-position of N-acetylglucosamine in the reducing end through α-bond were prepared using the YB2/0 cell-derived anti-CCR4 chimeric antibody KM2760-1 and CHO/DG44 cell-derived KM3060 described in the item 5 of Example 4 of WO03/085119. When the ratio of sugar chains in which 1-position of fucose is not bound to 6-position of N-acetylglucosamine in the reducing end through α-bond was measured by the monosaccharide composition analysis described in the item 6 of Example 3 on 11 standard samples including KM2761-1, KM3060 and 9 samples prepared by mixing both of them, KM2761-1 was 90%, KM3060 was 10%, and the prepared 9 standard samples were 82%, 74%, 66%, 58%, 50%, 42%, 34%, 26% and 18%, respectively.

(2) Evaluation of the binding activity of anti-CCR4 chimeric antibody to shFcγRIIIa

The evaluation was carried out in accordance with the method described in item 3 of Example 11 of WO03/085119. Firstly, a human CCR4 extracellular region peptide conjugate at a concentration of 1.0 μg/ml was dis-

pensed at 50 μl/well into a 96 well ELISA plate (manufactured by Greiner) and then allowed to stand at 4°C overnight for adsorption. After washing with PBS, 1% BSA-PBS was added at 100 μl/well and allowed to react at room temperature for 1 hour to thereby block the remaining active groups. After washing each well with Tween-PBS, each anti-CCR4 chimeric antibody sample diluted with 1% BSA-PBS to 5.0 μg/ml was added at 50 μl/well and allowed to react at room temperature for 1 hour. After washing each well with Tween-PBS, an shFcγRIIIa solution diluted with 1% BSA-PBS to 5.0 μg/ml was added at 50 μl/well and allowed to react at room temperature for 1 hour. After washing with Tween-PBS, an HRP-labeled anti-His-tag mouse antibody Penta-His HRP Conjugate (manufactured by QIAGEN) prepared into a solution of 0.1 μg/ml using 1% BSA-PBS was added at 50 μl/well and allowed to react at room temperature for 1 hour. After washing with Tween-PBS, the ABTS substrate solution was added at 50 μl/well for color development and OD415 was measured.

Fig. 3 shows the binding activity of each of the anti-CCR4 chimeric antibodies prepared in the above item (1), a having known ratio of sugar chains in which 1-position of fucose is not bound to 6-position N-acetylglucosamine in the reducing end through α-bond, to shFcγRIIIa. The binding activity to shFcγRIIIa increased in proportion to the ratio of sugar chains in which 1-position of fucose is not bound to 6-position of N-acetylglucosamine in the reducing end through α-bond.

Fig.4 shows the binding activity of anti-CCR4 chimeric antibodies, contained in the serum-free fed-batch samples collected in the item 2 of this Example, to shFcγRIIIa, as measured values of OD415. The shFcγRIIIa-binding activity was hardly found in all of the samples derived from the transformant CHO/CCR4-AF. On the other hand, the samples derived from the transformant SM3GI/CCR4-AF showed strong binding activity to shFcγRIIIa throughout the culturing period.

Table 3 shows the ratio of sugar chains in which 1-position of fucose is not bound to 6-position of N-acetylglucosamine in the reducing end through α-bond in the anti-CCR4 chimeric antibody contained in each cultured sample, calculated based on the calibration curve of Fig. 3. Also, samples showing a value exceeding the absorbance of KM2760-1 (90%) were shown as 90% or more (>90%). The ratio of sugar chains in which 1-position of fucose is not bound to 6-position of N-acetylglucosamine in the reducing end through α-bond was as small as 10 to 11% in the samples derived from the transformant CHO/CCR4-AF, while the samples derived from the transformant SM3G1/CCR4-AF showed a large value of 90% or more throughout the culturing period.

Table 3

| | Culturing days | | | | | |
|---|---|---|---|---|---|---|
| Antibody name | 0 | 3 | 6 | 9 | 11 | 13 |
| Strain DG44/CCR4-AF | 11.1% | 10.9% | 10.7% | 10.9% | 10.8% | 11.2% |
| Strain SM3G1/CCR4-AF | >90% | >90% | >90% | >90% | >90% | >90% |

(3) Monosaccharide composition analysis of anti-CCR4 chimeric antibody

On the 13th day of the serum-free fed-batch culturing, about 40 ml of cell suspension was recovered from each of the antibody transformants SM3G1/CCR4-AF and CHO/CCR4-AF, respective anti-CCR4 chimeric antibodies was purified in accordance with the method described in the item 3 of Example 3, and then monosaccharide composition analysis of the anti-CCR4 chimeric antibodies was carried out in accordance with the method described in the item 6 of Example 3.

Table 4 shows the ratio of sugar chains in which 1-position of fucose is not bound to 6-position of N-acetylglucosamine in the reducing end through α-bond, occupying the total complex type N-glycoside-linked sugar chains, which is calculated based on the monosaccharide composition ratio of each antibody. The ratio of sugar chains in which fucose is not bound was 15% in the anti-CCR4 chimeric form derived from the transformant CHO/CCR4-AF, but since the peak of fucose in the anti-CCR4 chimeric antibody derived from the transformant SM3G1/CCR4-AF was equal to or lower that the detection limit, its ratio of sugar chains in which fucose is not bonded was estimated to be 100%.

Based on the above results, it was confirmed that the GDP-mannose 4,6-dehydratase gene knockout transformants can also stably produce antibodies having sugar chains in which 1-position of fucose is not bound to 6-position of N-acetylglucosamine in the reducing end through α-bond under the serum-free fed-batch culturing.

Table 4

| Antibody | Ratio of sugar chains in which fucose is not bound |
|---|---|
| CHO/CCR4-AF | 15% |
| SM3G1/CCR4-AF | ~100% |

Example 5

Production of human antithrombin III and mutation type human antithrombin III, having sugar chains in which fucose is not bound, and biological activities thereof:

Using the clone CHO SM obtained in Example 1 as a GDP-mannose 4,6-dehydratase gene knockout clone, cells capable of stably producing human antithrombin III (hereinafter referred to as ATIII) and a mutation type human antithrombin III in which Asn at position 135 from the N-linked type sugar chain addition site in the mature type is substituted with Gln (hereinafter referred to as ATIIIN135Q) were prepared by the methods shown below.

1. Preparation of plasmid pBS-ATIII

The following PCR was carried out by preparing two ATIII gene-specific primers (SEQ ID NOs:19 and 20) from a human ATIII gene sequence (UniGene: Hs.75599, SEQ ID NO:18), to which restriction enzyme sites (for *Eco*RI and *Bam*HI) and the Kozak's sequence were added. That is, by preparing 20 μl of a reaction solution [Pyrobes® DNA Polymerase (manufactured by Takara Bio), 10 × Pyrobest buffer, 0.2 mmol/l of dNTP mixture and 0.5 μmol/l of the above-described primers (SEQ ID NOs:19 and 20)] comprising human liver-derived cDNA as the template, after heating at 94°C for 1 minute, the PCR was carried out by reaction of 30 cycles, one cycle consisting of reaction at 98°C for 30 seconds, reaction at 50°C for 1 minute and reaction at 72°C for 2 minutes. After the PCR, the reaction solution was subjected to 1.5% (w/v) agarose gel electrophoresis, and an ATIII gene DNA fragment of about 1,400 bp was confirmed and purified using QIAquick Gel Extraction Kit (manufactured by QIAGEN).

The thus obtained purified ATIII DNA fragment was dissolved in 17 μl of water, 20 μl of a reaction solution was prepared by adding 10 units of *Eco*RI (manufactured by Takara Bio), 10 units of *Bam*HI (manufactured by Takara Bio) and 2 μl of 10 × H buffer to the solution, and then the digestion reaction was carried out at 37°C for 16 hours Next, 3 μg of a plasmid pBluescript II KS(+) (manufactured by Stratagene) was dissolved in 17.5 μl of sterile water, 20 μl of a reaction solution was prepared by adding 10 units of *Eco*RI and 2 μl of 10 × H buffer to the solution, and then the digestion reaction was carried out at 37°C for 16 hours. After the reaction, phenol/chloroform extraction treatment and ethanol precipitation were carried out, and the thus recovered plasmid was dissolved in 17.5 μl of water. After 20 μl of a reaction solution was further prepared by adding 10 units of *Bam*HI and 2 μl of 10 × K buffer to the solution, the digestion reaction was carried out at 37°C for 16 hours.

The ATIII DNA fragment (*Eco*RI-*Bam*HI) and the pBluescript II KS(+) fragment (*Eco*RI-*Bam*HI), both obtained in the above, were subjected to 1.5% (w/v) agarose gel electrophoresis, and respective DNA fragments of about 1,400 bp and 3 kbp were purified using QIAquick Gel Extraction Kit (manufactured by QIAGEN). Next, 20 μl of a reaction solution comprising 20 ng of the ATIII DNA fragment (*Eco*RI-*Bam*HI), 80 ng of the pBluescript II KS(+) fragment (*Eco*RI-*Bam*BI) and Ligation High (manufactured by TOYOBO) was prepared, and the ligation reaction was carried out at 16°C for 16 hours. Using the thus obtained plasmid DNA, *Escherichia coli* DH5α (manufactured by TOYOBO) was transformed by the heat shock method. Plasmid DNA was prepared from each transformant using QIAprep® Spin Miniprep Kit (manufactured by QIAGEN), and its nucleotide sequence was analyzed using BigDye Terminator Cycle Sequencing Ready Reaction Kit v2.0 (manufactured by QIAGEN) and a DNA sequencer ABI PRISM 377 (manufactured by Applied Biosystems). As a result, a plasmid pBS-ATIII containing ATIII gene sequence shown in Fig. 5 was obtained.

2. Preparation of expression vector pKAN-ATIII

After 20 μl of a reaction solution was prepared by dissolving 3 μg of the pBS-ATIII prepared in the item of this Example in 17 μl of water and adding thereto 10 units of *Eco*RI (manufactured by Takara Bio), 10 units of *Bam*HI (manufactured by Takara Bio) and 2 μl of 10 × H buffer, the digestion reaction was carried out at 37°C for 16 hours.

Next, 3 μg of the plasmid pKANTEX93 described in the item 1(3) of Example 6 was dissolved in 17.5 μl of water. After 20 μl of a reaction solution was prepared by adding 10 units of *Eco*RI and 2 μl of 10 × H buffer to the solution, the digestion reaction was carried out at 37°C for 16 hours. After the reaction, phenol/chloroform extraction treatment and ethanol precipitation were carried out, and the thus recovered plasmid was dissolved in 17.5 μl of water. After 20 μl of a reaction solution was further prepared by adding 10 units of *Bam*HI and 2 μl of 10 × K buffer to the solution, the digestion reaction was carried out at 37°C for 16 hours.

The pBS-ATIII fragment (*Eco*RI-*Bam*HI) and pKANTEX93 fragment (*Eco*RI-*Bam*HI), both obtained in the above, were subjected to 1.5% (w/v) agarose gel electrophoresis, and respective DNA fragments of about 1.4 kb and 9 kb were purified using QIAquick Gel Extraction Kit (manufactured by QIAGEN). Next, 20 μl of a reaction solution containing 50 ng of the purified pBS-ATIII fragment (*Eco*RI-*Bam*HI), 30 ng of the purified pKANTEX93 fragment (*Eco*RI-*Bam*HI) and Ligation High (manufactured by TOYOBO) was prepared, and the ligation reaction was carried out at 16°C for 16 hours. Using the thus obtained plasmid DNA, *Escherichia coli* DH5α (manufactured by TOYOBO) was transformed by the heat shock method. By preparing a plasmid DNA from the transformant using QIAprep® Spin Miniprep Kit (manufactured by

QIAGEN), pKAN-ATIII shown in Fig. 6 was obtained.

3. Preparation of plasmid pBS-ATIIIN135Q

Firstly, two primers (SEQ ID NOs:21 and 22) in which the amino acid Asn at position 167 (position 135 in the mature ATIII) was substituted with Gln were prepared from the ATIII gene sequence (UniGene: Hs.75599) represented by SEQ ID NO:18. Using the pBS-ATIII prepared in the item 1 of this Example, these primers and Quick Change® Site-Directed Mutagenesis Kit (manufactured by STRATAGENE), amino acid substitution of the ATIII gene sequence was carried out. The method was carried out in accordance with the manual attached to the kit. Plasmid DNA samples were prepared from the thus obtained transformants using QIAprep® Spin Miniprep Kit (manufactured by QIAGEN), and their nucleotide sequences were analyzed using BigDye Terminator Cycle Sequencing Ready Reaction Kit v2.0 (manufactured by QIAGEN) and a DNA sequencer ABI PRISM 377 (manufactured by Applied Biosystems). As a result, a plasmid pBS-ATIIIN135Q comprising a mutation type ATIII gene sequence, shown in Fig. 7, was obtained.

4. Preparation of expression vector pKAN-ATIIIN135Q

After 3 μg of the pB5-ATIIIN135Q prepared in the item 3 of this Example was dissolved in 17 μl of water, 20 μl of a reaction solution was prepared by adding thereto 10 units of *Eco*RI (manufactured by Takara Bio), 10 units of *Bam*HI (manufactured by Takara Bio) and 2 μl of 10 × H buffer and the digestion reaction was carried out at 37°C for 16 hours.

Next, 3 μg of the plasmid pKANTEX93 described in the item 1(3) of Example 6 was dissolved in 17.5 μl of water. By adding 10 units of *Eco*RI and 2 μl of 10 × H buffer to the solution, 20 μl of a reaction solution was prepared to carry out the digestion reaction at 37°C for 16 hours. After the reaction, phenol/chloroform extraction treatment and ethanol precipitation were carried out, and the thus recovered plasmid was dissolved in 17.5 μl of water. By further adding 10 units of *Bam*HI and 2 μl of 10 × K buffer to the solution, 20 μl of a reaction solution was prepared to carry out the digestion reaction at 37°C for 16 hours.

The pBS-ATIIIN135Q fragment (*Eco*RI-*Bam*HI) and pKANTEX93 fragment (*Eco*RI-*Bam*HI) obtained in the above were subjected to 1.5% (w/v) agarose gel electrophoresis, and about 1.4 kb and 9 kb of DNA fragments were respectively purified using QIAquick Gel Extraction Kit (manufactured by QIAGEN). Next, 20μl of a reaction solution containing 50 ng of the purified pBS-ATIIIN135Q fragment (*Eco*RI-*Bam*HI), 30 ng of the purified pKANTEX93 fragment (*Eco*RI-*Bam*HI) and Ligation High (manufactured by TOYOBO) was prepared, and the ligation reaction was carried out at 16°C for 16 hours. Using the thus obtained plasmid DNA, *Escherichia coli* DH5α (manufactured by TOYOBO) was transformed by heat shock method. By preparing a plasmid DNA from the resulting transformant using QIAprep® Spin Miniprep Kit (manufactured by QIAGEN), pKAN-ATIIIN135Q shown in Fig. 7 was obtained.

5. Introduction of ATIII and ATIIIN135Q expression plasmids into clone CHO SM

The plasmids pKAN-ATIII and pKAN-ATIIIN135Q prepared in the items 2 and 4 of this Example were respectively introduced into the clone CHO SM prepared in Example 1. The gene introduction was carried out by the following procedure based on the conventionally known electroporation method [*Cytotechnology,* 3, 133 (1990)]. Firstly, 30 μg of the plasmid pKAN-ATIII or pKAN-ATIIIN135Q was linearlized by preparing 200 μl of a reaction solution containing 20 μl of NEBuffer 3 (manufactured by New England Biolabs) and 100 unites of a restriction enzyme *Mlu*I (manufactured by New England Biolabs) and carrying out the digestion reaction at 37°C for 16 hours. After the reaction, the reaction solution was purified by phenol/chloroform extraction treatment and ethanol precipitation to thereby recover the linear plasmid.

Next, the clone CHO SM obtained in Example 1 was suspended in a K-PB S buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to prepare a suspension of $8\times10^7$ cells/ml. After 200 μl of the cell suspension ($1.6\times10^6$ cells) was mixed with 9 μg of the above-described linear plasmid, a total volume of the cell-DNA mixture was transferred into Gene Pulser Cuvette (2 mm in inter-electrode distance, manufactured by BIO-RAD), and gene introduction was carried out using a cell fusion device Gene Pulser (manufactured by BIO-RAD) under conditions of 350 V in pulse voltage and 250 μF in electric capacity. After the gene introduction, the cell suspension was suspended in 30 ml of IMDM medium (manufactured by Life Technologies) supplemented with 10% fetal bovine serum (manufactured by Life Technologies) and 50 μg/ml of gentamicin (manufactured by Nacalai Tesque) and inoculated at 100 μl/well into 96-well plates for adhesion culture (manufactured by Greiner). The culturing was carried out under conditions of 5% $CO_2$ and 37°C.

6. Obtaining of 500 nM MTX-resistant strain

Each of the pKAN-ATIII-introduced cell and pKAN-ATIIIN135Q-introduced cell obtained in the above item was cultured for 6 days, and then the culture supernatants were discarded and the IMDM medium supplemented with 10% dialyzed fetal bovine serum, 50 μg/ml gentamicin and 50 nM methotrexate (MTX) (manufactured by SIGMA) was dispensed at 100 μl/well. The culturing was continued for 9 days while repeating this medium exchanging work at an interval of 3 to 4 days. Next, the culturing was continued for 18 days while repeating the medium exchanging work using the IMDM medium supplemented with 10% dialyzed fetal bovine serum, 50 μg/ml gentamicin and 200 nM MTX at an interval of 3 to 4 days, and the finally formed colonies were inoculated into a 24 well plate (manufactured by Greiner). Subsequently, the culturing was continued for 19 days while repeating the medium exchanging work using the IMDM medium supplemented with 10% dialyzed fetal bovine serum, 50 μg/ml gentamicin and 500 nM MTX at an interval of 3 to 4 days, optionally expanding the process to thereby obtaining clones resistant to 500 nM MTX.

7. Selection of clones highly producing ATIII and ATIIIN135Q

From each of the several 500 nm MTX-resistant clones obtained in the above item, $1.0\times0^6$ cells were collected, suspended in 5 ml of the IMDM medium supplemented with 10% dialyzed fetal bovine serum, 50 μg/ml gentamicin and 500 nM MTX, and then cultured by inoculating into a T25 flask. The culture supernatants were recovered after 3 days of the culturing, and the amounts of ATIII and ATIIIN135Q contained in the supernatants were measured using ELISA for antithrombin(ATIII) kit (manufactured by Affinity Biological). The method was effected in accordance with the manual attached hereto, and a pharmaceutical preparation Neuart® (manufactured by Mitsubishi Pharma Corporation) was used in preparing the calibration curve.. As a result, it was confirmed that ATIII and ATIIIN135Q are expressed in the culture supernatants of the thus obtained ATIII expressing clone pKAN-ATIII1 GMDKO and ATIIIN135Q -expressing clone pKAN-ATIIIN135Q6 GMDKO at a concentration of 513 ng/ml and of 45.4 ng/ml, respectively. Also, the clones pKAN-ATIII1 GMDKO and pKAN-ATIIIN135Q6 GMDKO were deposited, under the names of pKAN-ATID1 GMDKO and pKAN-AT11IN135Q6 GMDKO, with International Patent Organism Depositary, National Institute of Advanced Industrial Science and Technology (Central 6, 1-1-1 Higashi, Tsukuba, Ibaraki, Japan), on August 10, 2004 as accession numbers FERM ABP-10083 and FERM ABP-10084, respectively. In addition, it was confirmed that the ATIII and ATIIIN135Q produced by the GDP-mannose 4,6-dehydratase gene knockout cell have no fucose modification in their sugar chain structures, show improved stability in blood in comparison with the ATIII and ATIIIN135Q prepared by the usual CHO cell, and also show significant activity differences in pharmacological activity such as heparin binding activity.

## INDUSTRIAL APPLICABILITY

The present invention provides a cell capable of producing a glycoprotein having high physiological activity; a process for producing a glycoprotein using the cell; a glycoprotein produced by the process; and use thereof
Free Text of Sequence Listing:

SEQ ID NO:11 - Explanation for synthetic sequence : Synthetic DNA
SEQ ID NO: 12 - Explanation for synthetic sequence : Synthetic DNA
SEQ ID NO:13 - Explanation for synthetic sequence : Synthetic DNA
SEQ ID NO: 14 - Explanation for synthetic sequence : Synthetic DNA
SEQ ID NO: 15 - Explanation for synthetic sequence : Synthetic DNA
SEQ ID NO:16 - Explanation for synthetic sequence : Synthetic DNA
SEQ ID NO: 19 - Explanation for synthetic sequence : Synthetic DNA
SEQ ID NO: 20 - Explanation for synthetic sequence : Synthetic DNA
SEQ ID NO: 21 - Explanation for synthetic sequence : Synthetic DNA
SEQ ID NO:22 - Explanation for synthetic sequence : Synthetic DNA

**Claims**

1. A cell in which a genomic gene encoding an enzyme capable of catalyzing a dehydration reaction to convert GDP-mannose into GDP-4-keto,6-deoxy-GDP-mannose is knocked out.

2. The cell according to claim 1, wherein all of alleles on a genome encoding an enzyme capable of catalyzing a dehydration reaction to convert GDP-mannose into GDP-4-keto,6-deoxy-GDP-mannose are knocked out.

3. The cell according to claim 1 or 2, wherein at least exon 5 of the genomic gene encoding an enzyme capable of catalyzing a dehydration reaction to convert GDP-mannose into GDP-4-keto,6-deoxy-GDP-mannose is deleted.

4. The cell according to any one of claims 1 to 3, wherein the enzyme capable of catalyzing a dehydration reaction to convert GDP-mannose into GDP-4-keto,6-deoxy-GDP-mannose is GDP-mannose 4,6-dehydratase.

5. The cell according to any one of claims 1 to 4, which is resistant to a lectin which recognizes a sugar chain structure in which 1-position of fucose is bound to 6-position of N-acetylglucosamine in the reducing end through α-bond in a complex type N-glycoside-linked sugar chain.

6. The cell according to claim 5, wherein the cell which is resistant to a lectin is a cell selected based on the fact that the cell shows a higher survival ratio than a cell before the genomic gene is not knocked out, when the cells are cultured in a medium comprising lectin which recognizes a sugar chain structure in which 1-position of fucose is bound to 6-position of N-acetylglucosamine in the reducing end through α-bond in a complex type N-glycoside-linked sugar chain.

**7.** The cell according to any one of claims 1 to 6, which comprises a gene encoding a glycoprotein.

**8.** The cell according to claim 7, wherein the glycoprotein is a glycoprotein which does not have a sugar chain structure in which 1-position of fucose is bound to 6-position of N-acetylglucosamine in the reducing end through α-bond in a complex type N-glycoside-linked sugar chain.

**9.** The cell according to claim 7 or 8, wherein the glycoprotein is an antibody.

**10.** The cell according to claim 9, wherein the antibody belongs to an IgG class.

**11.** A process for producing a glycoprotein composition, which comprises using the cell according to any one of claims 1 to 10.

**12.** A process for producing a glycoprotein composition, which comprises culturing the cell according to any one of claims 1 to 10 in a medium to form and accumulate the glycoprotein composition in the culture, and recovering and purifying the glycoprotein composition from the culture.

**13.** The process according to claim 11 or 12, wherein the glycoprotein is an antibody.

**14.** A transgenic non-human animal or plant or the progenies thereof, which is produced by using the cell according to any one of claims 1 to 6.

**15.** The transgenic non-human animal or plant or the progenies thereof according to claim 14, wherein the transgenic non-human animal is an animal selected from the group consisting of cattle, sheep, goat, pig, horse, mouse, rat, fowl, monkey and rabbit.

**16.** The transgenic non-human animal or plant or the progenies thereof according to claim 14 or 15, which is introduced with a gene encoding a glycoprotein.

**17.** The transgenic non-human animal or plant or the progenies thereof according to claim 16, wherein the glycoprotein is an antibody.

**18.** The transgenic non-human animal or plant or the progenies thereof according to claim 17, wherein the antibody belongs to an IgG class.

**19.** A process for producing a glycoprotein, which comprises rearing the transgenic non-human animal or plant according to any one of claims 14 to 18; isolating a tissue or body fluid comprising a glycoprotein composition introduced from the reared animal or plant; and recovering and purifying the glycoprotein composition of interest from the isolated tissue or body fluid.

**20.** A process for producing a glycoprotein composition, which comprises isolating a glycoprotein-producing cell from the transgenic non-human animal or plant or the progenies thereof according to any one of claims 14 to 18; culturing the isolated glycoprotein-producing cell in a medium to form and accumulate the glycoprotein composition in the culture; and recovering and purifying the glycoprotein composition from the culture.

**21.** The process according to claim 19 or 20, wherein the glycoprotein is an antibody.

**22.** A glycoprotein composition produced by the process according to any one of claims 11 to 13 and 19 to 21.

**23.** An antibody composition produced by the process according to claim 13 or 21.

**24.** A medicament comprising the composition according to claim 22 or 23 as an active ingredient.

FIG. 1

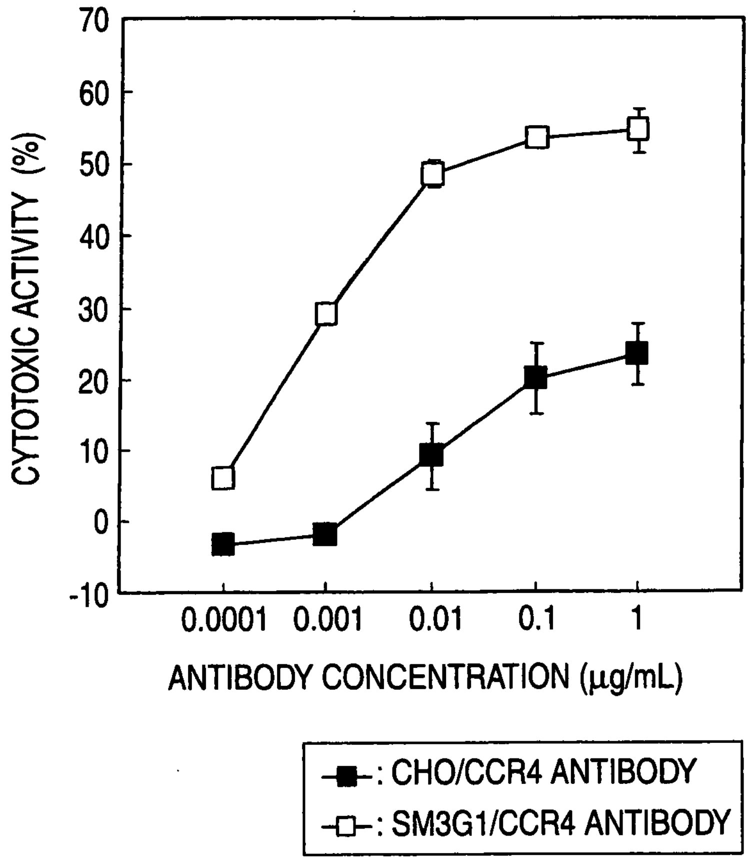
70
60
50
40
30
20
10
0
-10
CYTOTOXIC ACTIVITY (%)
0.0001
0.001
0.01
0.1
1
ANTIBODY CONCENTRATION (μg/mL)
: CHO/CCR4 ANTIBODY
: SM3G1/CCR4 ANTIBODY

FIG. 2 (A)

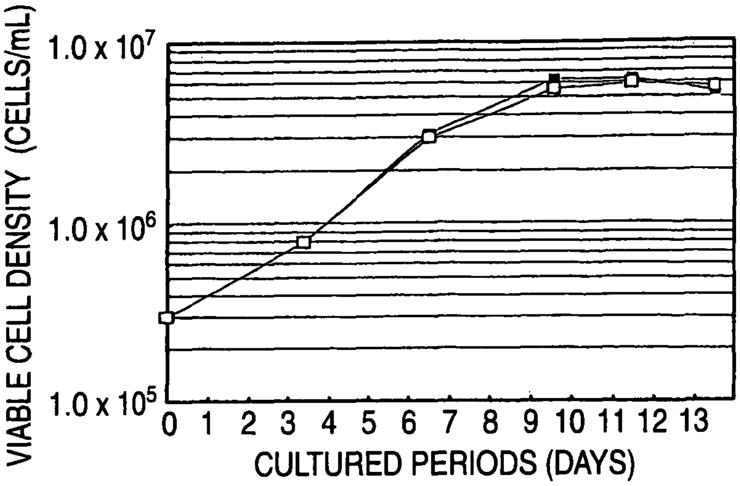


FIG. 2 (B)

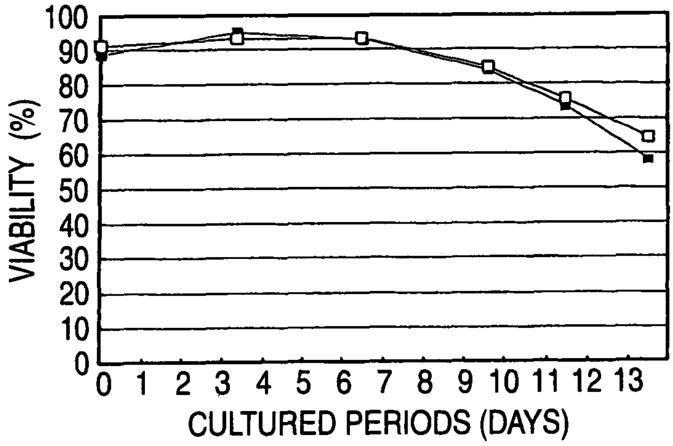


FIG. 2 (C)

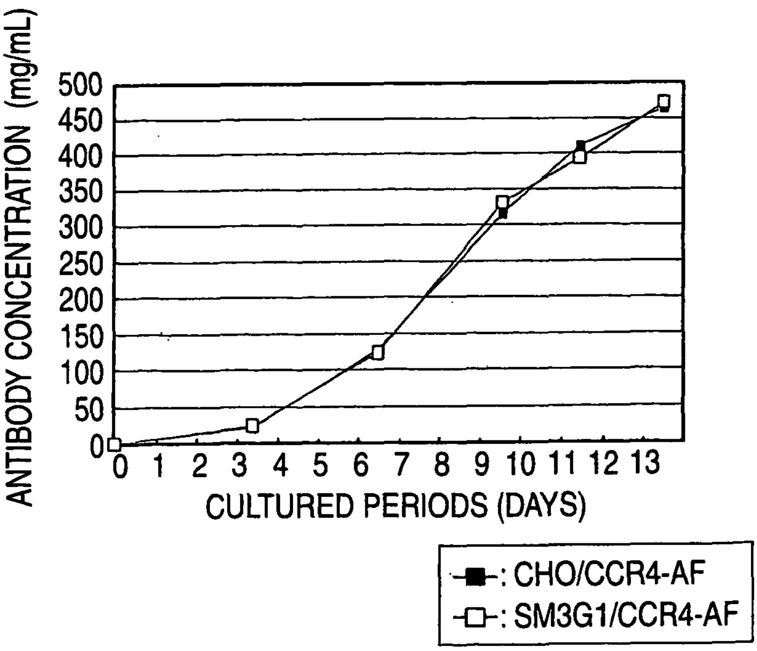

FIG. 3

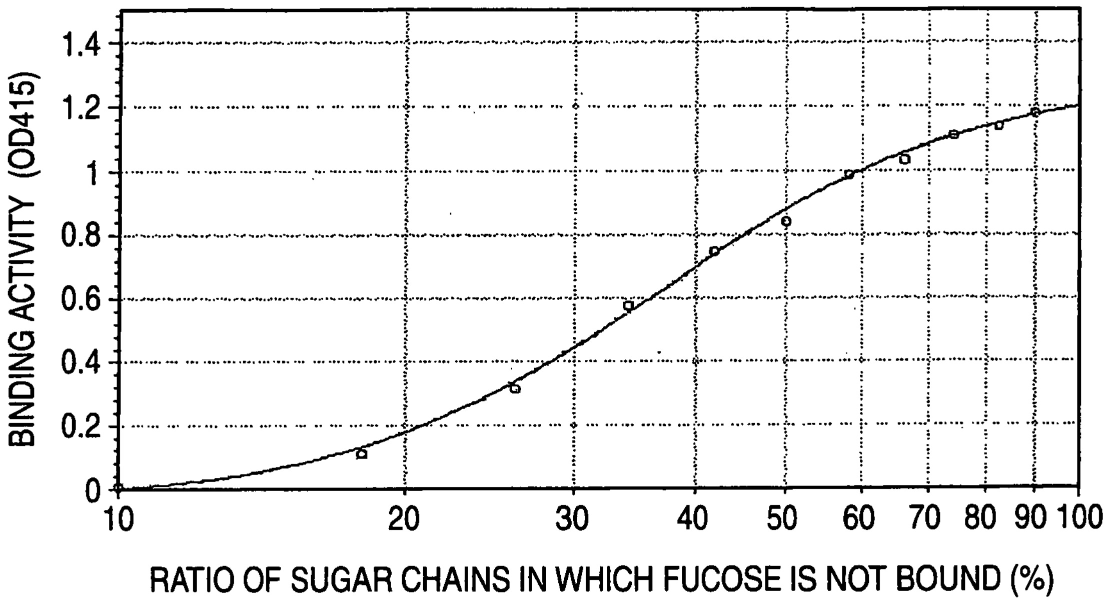
BINDING ACTIVITY (OD415)
1.4
1.2
1
0.8
0.6
0.4
0.2
0
10
20
30
40
50
60
70
80
90
100
RATIO OF SUGAR CHAINS IN WHICH FUCOSE IS NOT BOUND (%)

*FIG. 4*

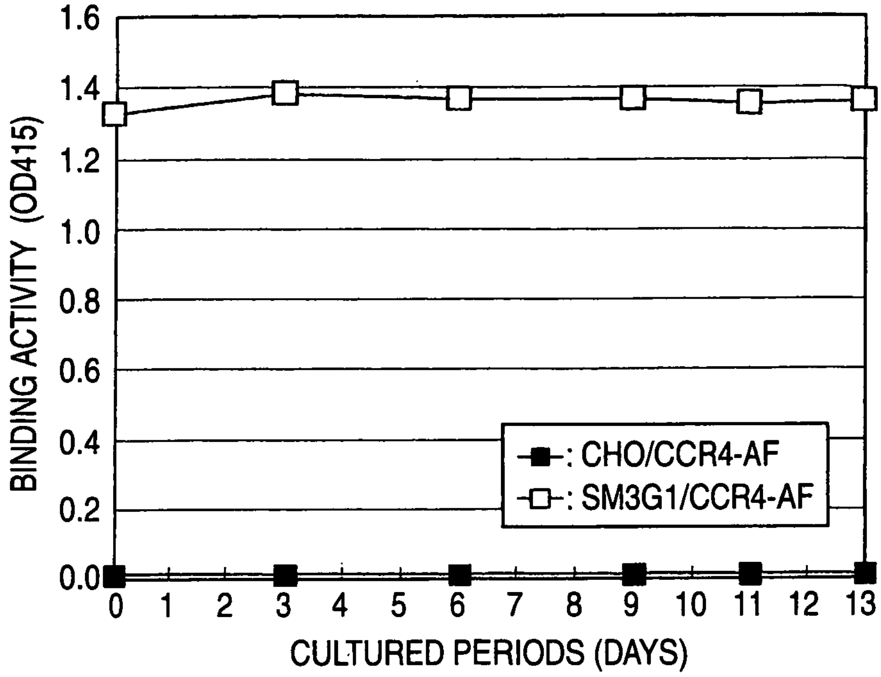
1.6
1.4
1.2
1.0
0.8
0.6
0.4
0.2
0.0
BINDING ACTIVITY (OD415)
0 1 2 3 4 5 6 7 8 9 10 11 12 13
CULTURED PERIODS (DAYS)
-■-: CHO/CCR4-AF
-□-: SM3G1/CCR4-AF

*FIG. 5*

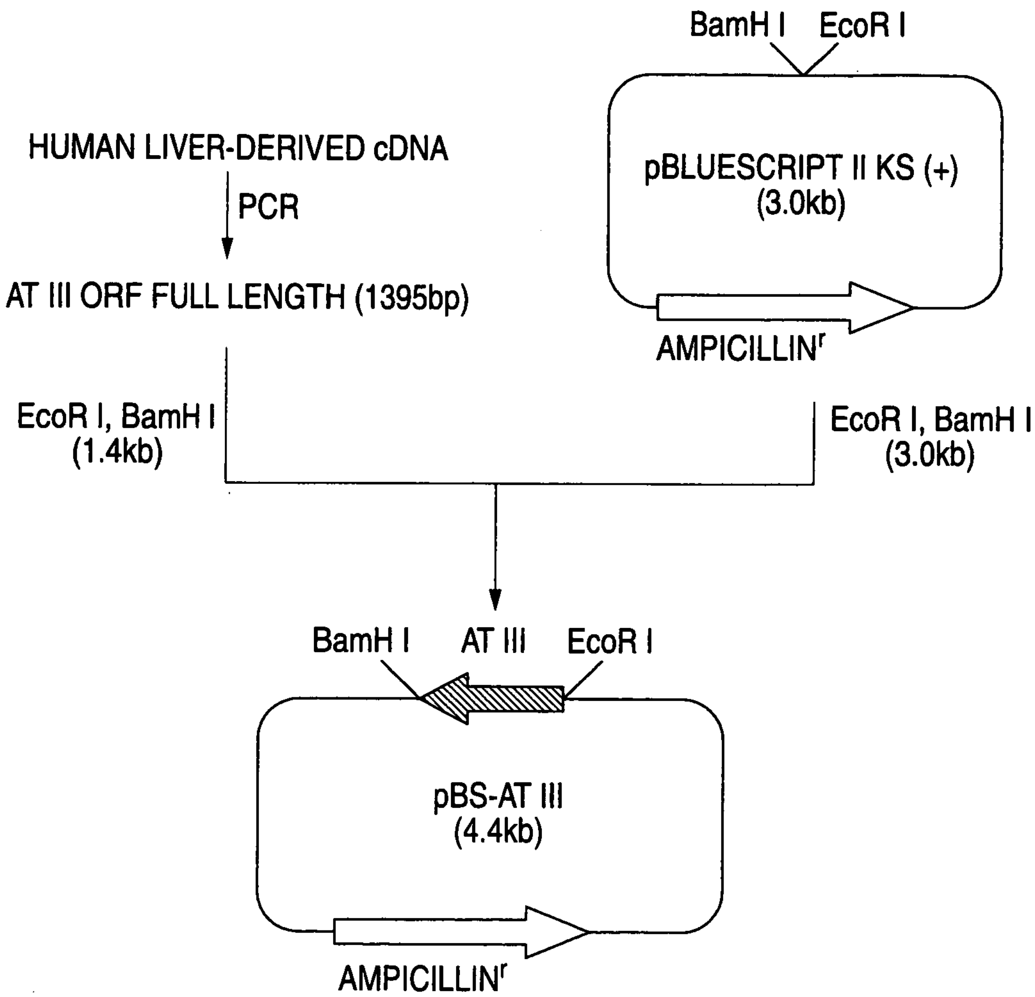
BamH I
EcoR I
HUMAN LIVER-DERIVED cDNA
PCR
pBLUESCRIPT II KS (+)
(3.0kb)
AT III ORF FULL LENGTH (1395bp)
AMPICILLIN^r
EcoR I, BamH I
(1.4kb)
EcoR I, BamH I
(3.0kb)
BamH I
AT III
EcoR I
pBS-AT III
(4.4kb)
AMPICILLIN^r

FIG. 6

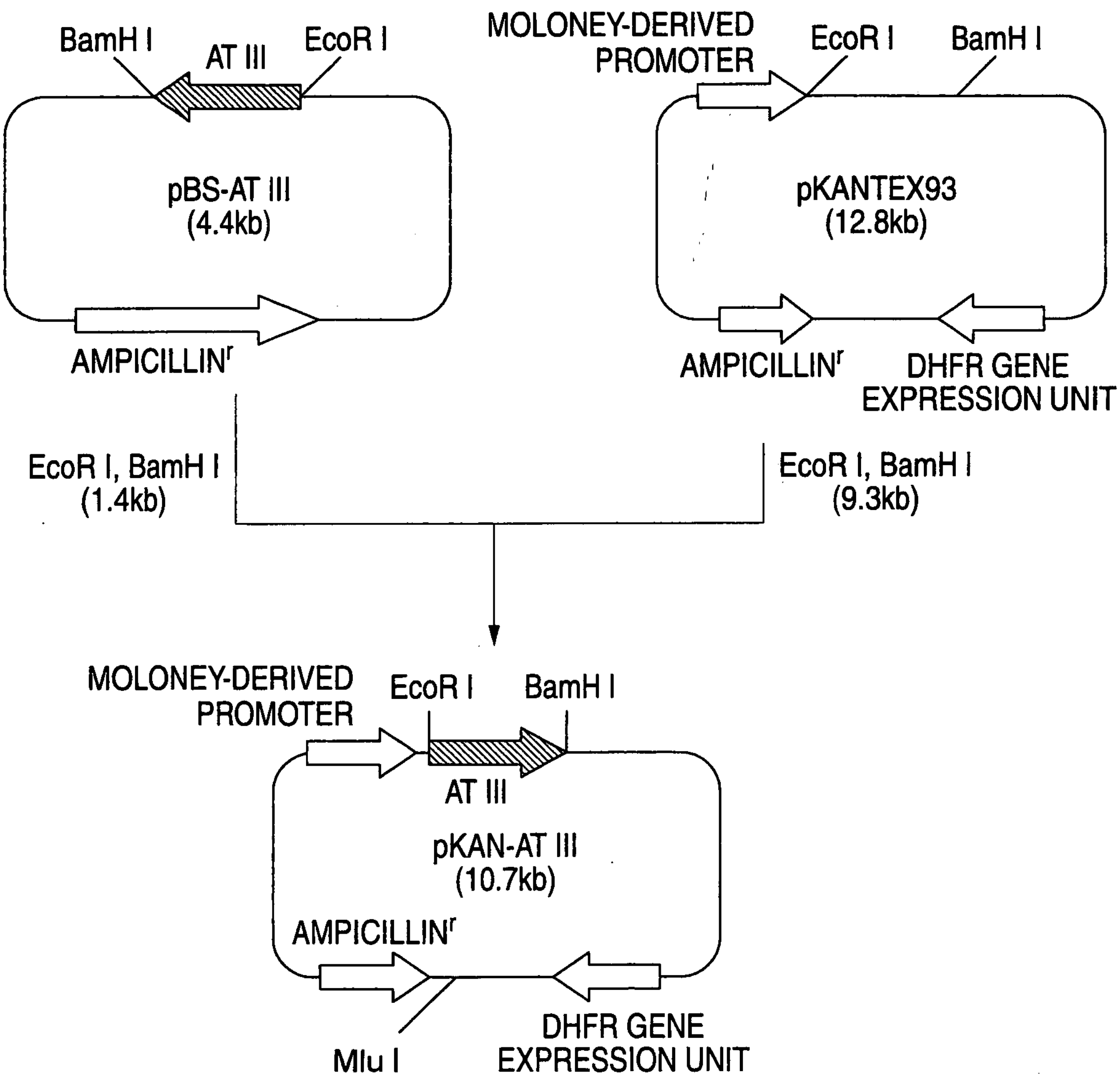
BamH I
AT III
EcoR I
pBS-AT III
(4.4kb)
AMPICILLIN$^r$
MOLONEY-DERIVED PROMOTER
EcoR I
BamH I
pKANTEX93
(12.8kb)
AMPICILLIN$^r$
DHFR GENE EXPRESSION UNIT
EcoR I, BamH I
(1.4kb)
EcoR I, BamH I
(9.3kb)
MOLONEY-DERIVED PROMOTER
EcoR I
BamH I
AT III
pKAN-AT III
(10.7kb)
AMPICILLIN$^r$
Mlu I
DHFR GENE EXPRESSION UNIT

FIG. 7

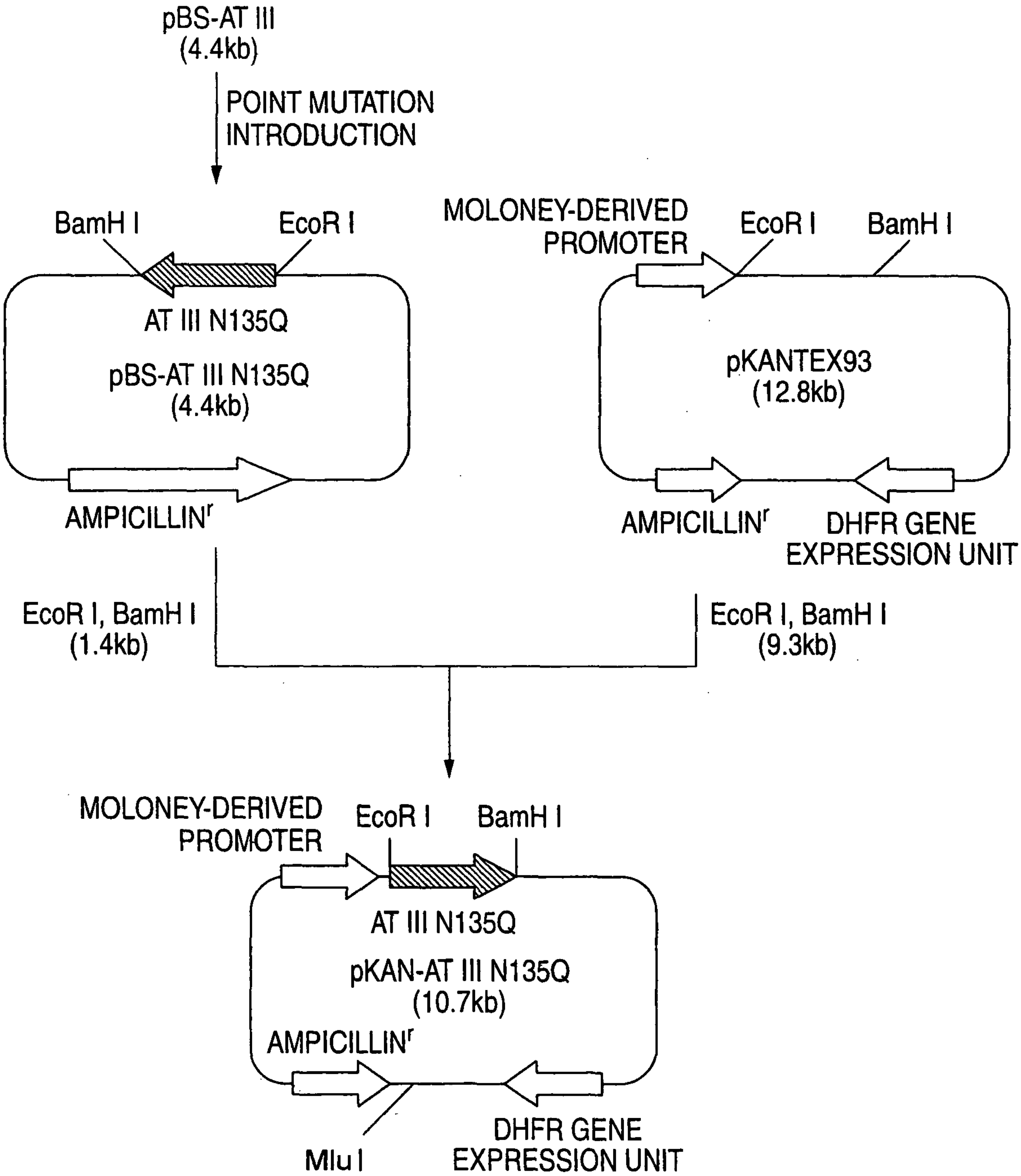
pBS-AT III
(4.4kb)
POINT MUTATION
INTRODUCTION
BamH I
EcoR I
AT III N135Q
pBS-AT III N135Q
(4.4kb)
AMPICILLINr
MOLONEY-DERIVED
PROMOTER
EcoR I
BamH I
pKANTEX93
(12.8kb)
AMPICILLINr
DHFR GENE
EXPRESSION UNIT
EcoR I, BamH I
(1.4kb)
EcoR I, BamH I
(9.3kb)
MOLONEY-DERIVED
PROMOTER
EcoR I
BamH I
AT III N135Q
pKAN-AT III N135Q
(10.7kb)
AMPICILLINr
Mlu I
DHFR GENE
EXPRESSION UNIT

**INTERNATIONAL SEARCH REPORT**

International application No.
PCT/JP2004/015318

A. CLASSIFICATION OF SUBJECT MATTER
Int.Cl[7] C12N5/10, C12N15/13, C12P21/08, A01K67/027, A01H5/00, A61K39/395

According to International Patent Classification (IPC) or to both national classification and IPC

B. FIELDS SEARCHED

Minimum documentation searched (classification system followed by classification symbols)
Int.Cl[7] C12N5/10, C12N15/13, C12P21/08, A01K67/027, A01H5/00, A61K39/395

Documentation searched other than minimum documentation to the extent that such documents are included in the fields searched

Electronic data base consulted during the international search (name of data base and, where practicable, search terms used)
REGISTRY(STN), CA(STN), WPI(DIALOG), BIOSIS(DIALOG), JSTPlus(JOIS), GenBank/EMBL/DDBJ/GeneSeq, SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| X | WO 02/31140 A1 (Kyowa Hakko Kogyo Co., Ltd.), 18 April, 2002 (18.04.02), & AU 200194198 A & US 2003/0115614 A1 & EP 1331266 A1 & BR 200114475 A & KR 2003081312 A & MX 2003002974 A1 | 1-24 |
| P,X | YAMANE-OHNUKI, N. et al., Establishment of FUT8 knockout Chinese hamster ovary cells: an ideal host cell line for producing completely defucosy lated antibodies with enhanced antibody-dependent cellular cytotoxicity.Biotechnol. Bioeng., 05 September, 2004 (05.09.04), Vol.87, No.5, pages 614 to 622 | 1-24 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 January, 2005 (13.01.05) | 01 February, 2005 (01.02.05) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/015318

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 03/85107 A1 (Kyowa Hakko Kogyo Co., Ltd.), 16 October, 2003 (16.10.03), & AU 2003236022 A1 & US 2004/0110704 A1 | 1-24 |
| P,X | WO 03/85118 A1 (Kyowa Hakko Kogyo Co., Ltd.), 16 October, 2003 (16.10.03), & AU 2003236015 A1 & US 2004/0132140 A1 | 1-24 |

Form PCT/ISA/210 (continuation of second sheet) (January 2004)